# EUROPEAN PATENT APPLICATION

(11) **EP 4 374 869 A1**
(43) Date of publication of application: **29.05.2024**
(21) Application number: 22209887.3
(22) Date of filing: 28.11.2022
(51) Int. Cl.: A61K 38/14, A61K 31/7048, A61P 25/28

(54) **CYCLIC LIPOPEPTIDE ANTIBIOTIC COMPOUNDS FOR USE IN THE TREATMENT OF PROTEIN AGGREGATION DISEASE**

(71) Applicant: Deutsches Zentrum für Neurodegenerative Erkrankungen e.V. (DZNE), 53127 Bonn (DE)
(72) Inventor: ZWECKSTETTER, Markus, 37085 Göttingen (DE); RAMIREZ, Lisa-Marie, 37075 Göttingen (DE); IBÁÑEZ DE OPAKUA LÓPEZ DE ABETXUKO, Alain, 37073 Göttingen (DE); VORBERG, Ina, 53227 Bonn (DE); HEBESTREIT, Alina, 53121 Bonn (DE); MADHUBABU, Gajula Balija, 500046 Hyderabad (IN)
(74) Representative: Hertin und Partner Rechts- und Patentanwälte PartG mbB

(57) **Abstract**

The invention relates to a cyclic lipopeptide antibiotic compound, or derivative and/or salt thereof, for use in the treatment and/or prevention of a medical condition associated with protein aggregation, preferably amyloidosis. The invention further relates to the compound comprising 5 to 20 amino acids, 3 or more non-proteinogenic amino acids, 5 or more amino acids with a side chain comprising an aromatic ring, one to four cyclic structures, and at least one amino acid is substituted with a hydrophobic moiety. In another preferred embodiment the treatment comprises binding of the compound to a protein and inhibiting protein aggregate formation and/or fibril elongation and/or inhibition of association between monomers of aggregating proteins, inhibition of protein oligomerization, inhibition of protein condensation, inhibition of secondary nucleation processes in fibril formation, such as disrupting replication of aggregate formation on the surface of an existing fibril, and/or inhibition of fibril elongation by capping fibril ends.

## Description

The invention is in the fields of biochemistry and medicine and relates to cyclic peptide compounds for use in the prevention and/or treatment of medical conditions associated with protein aggregation, such as amyloidosis.

The invention relates to a cyclic lipopeptide antibiotic compound, or derivative and/or salt thereof, for use in the treatment and/or prevention of a medical condition associated with protein aggregation, such as amyloidosis. The invention therefore relates to said compounds for use in inhibiting protein aggregation in the treatment and/or prevention of a medical condition associated with protein aggregation, and methods for inhibiting protein aggregation, preferably amyloidosis, comprising administering said compounds to a subject in need thereof.

The invention further relates to a compound comprising 5 to 20 amino acids, 3 or more non-proteinogenic amino acids, 5 or more amino acids with a side chain comprising an aromatic ring, one to four cyclic structures, and at least one amino acid is substituted with a hydrophobic moiety, for use in the treatment of a medical condition associated with protein aggregation, such as amyloidosis.

Preferred compounds of the invention relate to a ramoplanin, or derivative and/or salt thereof, such as ramoplanose, ramoplanin aglycon, ramoplanin A1-A3, enduracidin A, NAI-603 and/or enduracidin B, preferably ramoplanin A2 and/or NAI-603, or to a teicoplanin, or derivative and/or salt thereof, such as teicoplanin A2-1, teicoplanin A2-2, teicoplanin A2-3, teicoplanin A2-4, teicoplanin A2-5, ristocetin A, ristocetin B and/or vancomycin.

In one embodiment the treatment comprises non-covalent binding of the compound to a protein and inhibiting protein aggregate formation, fibril elongation, inhibition of association between monomers of aggregating proteins, inhibition of protein condensation and/or inhibition of protein oligomerization. In embodiments, the method comprises inhibition of secondary nucleation processes in fibril formation, such as disrupting replication of aggregate formation on the surface of an existing fibril, and/or inhibition of fibril elongation by capping fibril ends.

The invention further relates to a pharmaceutical composition comprising a compound of the invention for use in the treatment of a medical condition associated with protein aggregation, such as amyloidosis.

### BACKGROUND OF THE INVENTION

The aggregation of proteins characterizes the pathology of various diseases, in which proteins misfold and/or form large assemblies. Such aggregation may be caused by mutations, protein modifications, or environmental stress conditions (Valastyan et al., 2014). Notable examples of protein aggregation diseases are neurodegenerative disorders, such as Alzheimer's disease (AD), Lewy body dementia, Down syndrome, progressive supranuclear palsy, hereditary cerebral hemorrhage with amyloidosis (Dutch type), Guam Parkinson Dementia, Parkinson's disease (PD), HIV-associated dementia, amyotrophic lateral sclerosis (ALS), and multiple sclerosis (MS). Amyloidogenic proteins can further accumulate in the myocardium to cause cardiac amyloidosis (Kittleson et al., 2020). Also, the metabolic disorder type 2 diabetes (T2D) is characterized by amyloid deposits, specifically derived from the islet amyloid polypeptide (Kahn et al., 1999). Several studies support the correlation between islet amyloid deposition with the development and progression of type 2 diabetes (Westermark et al., 1994; Clark et al., 1988). Notably, islet amyloid has been detected postmortem in type 2 diabetes patients.

Further, mounting evidence suggests that neurodegenerative diseases can also have associated ocular manifestation (Burre et al., 2018). Amyloidosis in the pathogenesis of ocular diseases is aggravated by inflammation as well as oxidative stress, both of which are mechanisms linked to neurodegeneration (Colligris et al., 2018). Notably, amyloid β plaques have been found in the retina and lens of AD patients (Perez-Garmendia et al., 2020; Koronyo-Hamaoui et al., 2011; Koronyo et al., 2012; Jiang et al., 2016; La Morgia et al., 2016; Koronyo et al., 2017). Hyperphosphorylated tau was likewise observed in the postmortem retina of AD patients (Goldstein et al., 2003). In addition, amyloid β deposits are implicated in the pathogenesis of the ocular diseases age-related macular degeneration (Schön et al., 2013) and glaucoma (Johnson et al., 2022).

Recent evidence also shed light on the role of mutant p53 aggregation in tumor growth (Kanapathipillai at al., 2018). Mutants such as R248Q, R248W, and R175H have been reported to form aggregates in tumor samples (Sorangi et al., 2016; De smet et al., 2017) and the aggregation behavior may follow a prion-like mechanism (Ano Bom et al., 2012).

Although these diseases have diverse pathways of pathogenesis, they share a common molecular pathology in the form of protein aggregation, such as amyloid deposits. Historically, such deposits were found to mimic properties of starch (amylum in Latin) in iodine staining of histological samples, hence they were attributed the name amyloid (Sipe et al., 2000). Insoluble deposits inside and outside of cells, which are stained by amyloid-specific dyes, are formed from protein that can be mutated, misfolded, partially fragmented, or modified in a way that renders them prone to aggregate formation. About 36 proteins are considered to be aggregation-prone proteins, including, without limitation, tau, amyloid β peptide, alpha-synuclein, TDP-43, prion protein, immunoglobulin light chain, immunoglobulin heavy chain, apo serum amyloid A, wild-type and mutant transthyretin, gelsolin and its variants, keratoepithelin, lactoferrin, lactadherin, islet amyloid peptide, tumor protein p53, tumor protein PTEN, RAD51 and nucleoporin Nup98 (Benson et al., 2020). Fibrils formed by these proteins (including their fragments and modified counterparts) share common traits such as elongated morphology, ability to bind strongly to dyes thioflavin T and Congo red, and a β-sheet fold.

Of the above-mentioned diseases and medical conditions, dementia associated with Alzheimer's disease (AD) is one of the biggest challenges facing society today and in the future. AD is characterized by pathological hallmarks such as the accumulation of senile plaques, mainly composed of amyloid β (Aβ) fibrils and neurofibrillary tangles (NFTs) made up of tau fibrils. The formation and spread of these protein aggregates in the brain are implicated in the impairment of neurotransmitter systems and the loss of neurons. In the early stages of the disease, neural connections involved in memory, including the entorhinal cortex and hippocampus, are affected. As the disease progresses the pathology spreads to other areas of the brain, such as the cerebral cortex important for language, logic, and social interactions. By 2030, it is estimated that the cost of caring for people with dementia will have risen to 250 billon EUR in the EU and to 1.7-2.8 trillion USD worldwide, a total that could undermine social and economic development and overwhelm health and social services.

There is currently no disease-modifying therapy for dementia associated with AD. For the last 20 years only two approved treatment options have been available: (i) inhibitors to cholinesterase enzyme and (ii) antagonists to N-methyl d aspartate. Both are partially effective in treating AD symptoms, but do not prevent or cure AD.

As accumulation of the protein tau in the brain is a pathological hallmark of AD, and the decline of cognition in patients with AD closely correlates with the degree and site of tau pathology, many pharmaceutical companies therefore target pathological tau for disease intervention, with a strong focus and the development of tau antibodies. A major limitation of the currently investigated tau antibodies is, however, that they cannot enter neurons and thus are unable to act where tau accumulation occurs. There is thus a clear need to develop drugs that inhibit tau aggregation inside neurons.

The development of peptides that inhibit or reverse the aggregation of amyloidogenic proteins is a potentially useful approach to target various types of amyloidosis. Peptides called "β-sheet breakers", so named due to their ability to disrupt molecular interactions that build a cross-β sheets structure, have been designed and synthesized previously as potential amyloid fibril elongation inhibitors (Poduslo et al., 1999; Estrada et al., 2006; Bett et al., 2010). A subclass of peptide-based β-sheet breakers is comprised of cyclic peptides, which have been shown to have an activity in reducing the cytotoxicity of protein aggregates (Griffin et al., 2012; Kapurniotu et al., 2003; Zheng et al., 2011; Luo et al., 2014).

Despite some advances in addressing unwanted protein aggregation, no disease modifying treatments or means for the effective prevention of amyloid associated medical conditions have been developed. Thus, in view of the large number, high prevalence and severity of amyloidassociated diseases, the provision of effective means for prevention and or treatment of these medical conditions is urgently needed.

### SUMMARY OF THE INVENTION

In light of the prior art the technical problem underlying the present invention is the provision of improved or alternative means for treating or preventing medical conditions associated with protein aggregation, such as amyloidosis.

Another problem underlying the invention was the provision of a disease modifying treatment of medical conditions associated with amyloidosis, targeting amyloid formation and aggregation in a subject.

Another problem underlying the invention is the provision of compounds that directly prevent and/or inhibit amyloidosis comprising inhibiting protein aggregate formation and/or fibril elongation, inhibiting an association between monomers of aggregating proteins, inhibiting protein oligomerization, inhibiting protein condensation, inhibiting secondary nucleation processes in fibril formation, disrupting replication of aggregate formation on the surface of an existing fibril, and/or inhibiting fibril elongation by capping fibril ends.

These problems are solved by the features of the independent claims. Preferred embodiments of the present invention are provided by the dependent claims.

The invention therefore relates to a cyclic lipopeptide antibiotic compound, or derivative and/or salt thereof, for use in the treatment and/or prevention of a medical condition associated with protein aggregation.

In one embodiment, the invention relates to a cyclic lipopeptide antibiotic compound, or derivative and/or salt thereof, for use in inhibiting protein aggregation in the treatment and/or prevention of a medical condition associated with protein aggregation. In embodiments, the invention relates to methods for inhibiting protein aggregation, preferably amyloidosis, comprising administering said compounds to a subject in need thereof.

In one embodiment the treatment comprises the reduction and/or inhibition of amyloid protein aggregation in a subject.

The compounds of the present invention are particularly well suited for use in the treatment and/or prevention of a medical condition associated with amyloidosis. In the primary nucleation stage of fibrillization and amyloid formation, monomeric proteins assemble into dimers, trimers, or other higher order structures in the process of building a β-sheet template. Such a template then recruits additional protein molecules and forms the aggregate nucleus.

Due to their structural composition and associated properties, the compounds of the present invention interact with aggregation-prone protein monomers, thereby hindering and blocking association between monomers or assisting the formation of off-pathway oligomers. The compounds further disrupt secondary nucleation processes in amyloid fibril formation, including the replication of the formation of new aggregates (including fibrils or oligomers) on the surface of an existing amyloid fibril. Moreover, the compounds of the present invention can block fibril elongation by capping the fibril ends.

The unique combination of pi-stacking interactions afforded by the aromatic side chains and the presence of hydrogen-bond donors and acceptors on the compound core renders an interaction surface that is compatible to binding with a general cross-section of an amyloid fibril.

Crucial to the stability of amyloid fibrils are favorable hydrophobic contacts and hydrogen bonding patterns. Typically, the core of protein aggregates is enriched with hydrophobic side chains stacked together. The compounds of the present invention favorably associate with the exposed hydrophobic surface of the growing amyloid aggregate through pi-stacking and hydrophobic interactions mediated by the aromatic side chains.

The cyclic compounds of the present invention are furthermore resistant toward chemical degradation, and are thus metabolized at a slower rate than linear peptides. The cyclic structure also increases the interacting surface of the compounds, which is useful in recognizing the binding surface of protein aggregates. Moreover, the rigidity imparted by cyclization makes the compound more conformationally stable, which improves binding affinity by reducing nonspecific binding of alternative conformations of the compound.

A further advantage of cyclization is the enhanced membrane permeability of the compounds, as the constrained structure results in a lower energy barrier for passing through a membrane and binding to transporters, thereby increasing both passive diffusion and active transport across a membrane.

The present invention thus relates to a novel medical use and novel methods of treatment using a class of compounds not previously suggested to exhibit anti-protein aggregation properties. The mechanism underlying the invention thus credibly enables the medically relevant effects described herein. The mode of action of the compounds of the invention, with respect to inhibiting protein aggregation as described at length herein, represents a novel property for this class of compounds enabling novel medical treatments. The identification of the unexpected inhibitory properties of this compound class in disrupting protein aggregation enables the treatment of novel patient collectives, addressing unmet needs in the treatment of protein aggregation diseases. The mode of action enables novel dosing schemes, novel modes of administration and/or the treatment of novel patient groups, based on the unexpected discovery of the properties of these compounds regarding inhibiting protein aggregation.

As shown in more detail below, various compounds of the relevant compound class are effective in inhibiting protein aggregation. Due to the structural variation evident and the maintenance of the relevant biological activity, the relevant compound class is termed as cyclic lipopeptide antibiotic compounds, or derivatives and/or salts thereof.

In other embodiments, the compound is a cyclic lipopeptide antibiotic compound.

In other embodiments, the compound is a cyclic lipopeptide depsipeptide antibiotic compound, or derivative and/or salt thereof.

In other embodiments, the compound is a cyclic glycolipopeptide antibiotic compound, or derivative and/or salt thereof.

In other embodiments, the compound is a cyclic non-ribosomal antibiotic compound.

In other embodiments, the compound is a cyclic non-ribosomal lipopeptide depsipeptide antibiotic compound, or derivative and/or salt thereof.

In other embodiments, the compound is a cyclic non-ribosomal glycolipopeptide antibiotic compound, or derivative and/or salt thereof.

In embodiments, the compounds described herein may be termed protein aggregation inhibitors, preferably inhibitors of amyloid protein aggregation.

In embodiments, the compounds described herein may be termed protein condensation inhibitors, preferably inhibitors of amyloid protein condensation.

In preferred embodiments, the derivatives are those according to the chemical formulae presented herein. In other embodiments, alternative structural derivatives are encompassed in the present invention. In preferred embodiments, the derivative exhibits the desired property of inhibiting protein aggregation. A skilled person is capable of determining whether any given compound is a derivative of the compound class disclosed herein and whether said compound exhibits the desired properties of the invention.

In embodiments, the compound class is defined by the presence of a functional property, namely the reduction and/or inhibition of protein aggregation. Said protein aggregation may be evident when using one or more of the in vitro (or other) assays described in detail in the examples below. A skilled person is capable, without undue effort, in ascertaining whether any given compound within the compound class of the present invention exhibits the desired properties using the guidance provided herein and their common general knowledge.

The compounds of the invention may, in embodiments, be defined by their chemical structure, preferably according to the chemical formulae or written description enclosed herein.

In one embodiment, the compound comprises
a. 5 to 20 amino acids, preferably 7 to 17 amino acids,
b. 3 or more non-proteinogenic amino acids,
c. 5 or more amino acids with a side chain comprising an aromatic ring, preferably selected from the group consisting of tyrosine, phenylalanine, phenylglycine and derivatives thereof,
d. one to four cyclic structures, and/or
e. at least one amino acid is substituted with a hydrophobic moiety.

Each of these structural features a. to e. may be used in isolation, or in any sub-combination, to define the compound of the invention. In one embodiment, the compound comprises all structural features a., b., c., d. and e.

In one embodiment, the compound comprises
a. 5 to 20 amino acids, preferably 5, 6, 7, 8, 9, 10, 11, 12, 13, 14, 15, 16, 17, 18, 19 or 20 amino acids, more preferably 7 to 17 amino acids, wherein the compound comprises 3 or more non-proteinogenic amino acids, such as 3, 4, 5, 6, 7, 8, 9, or 10 such amino acids,
b. 5 or more amino acids with a side chain comprising an aromatic ring, preferably 5, 6, 7, 8, 9, or 10 such amino acids, wherein the amino acids with a side chain comprising an aromatic ring are preferably selected from the group consisting of tyrosine, phenylalanine, phenylglycine or any structural derivative thereof comprising an aromatic ring,
c. one to four cyclic structures, such as 1, 2, 3 or 4 such cyclic structures, and/or
d. at least one amino acid is substituted with a hydrophobic moiety, such as 1, 2, 3, 4, 5, 6 or more such amino acids.

In one embodiment, the hydrophobic moiety is a carbonyl group C=OR, wherein R is selected from the group consisting of alkyl, alkenyl, aryl, heteroaryl, alkyl aryl, and alkyl heteroaryl (preferably C5 to C20 alkyl, C5 to C20 alkenyl, C3 to C6 cycloalkyl, or benzyl), wherein R is optionally substituted with C1-C5 alkyl, C1-C5 alkenyl or C3 to C6 cycloalkyl.

In embodiments, the presence of hydrophobic moieties within the compounds enhances permeability through lipophilic membranes. As amyloid fibril formation usually occurs within cells such as neurons, good membrane compatibility of the components is advantageous for their use in the treatment and/or prevention of medical condition associated with protein aggregation.

Further, as stated above, the core of protein aggregates is enriched with hydrophobic side chains stacked together. Thus, hydrophobic moieties advantageously enhance affinity of the compounds of the present invention to protein monomers and aggregates thereby hindering their formation, elongation and further aggregation.

In one embodiment, 1 to 5 amino acids, preferably 1 to 3 amino acids, are substituted with a sugar moiety, wherein the sugar moiety preferably comprises a mono-, di- or trisaccharide. Sugar moieties with greater numbers of sugars are also envisaged, such as tetra- or penta-saccharides.

Sugar moieties, such as mono-, di- or trisaccharides, advantageously enhance recognition and association of the compounds with cellular membranes and receptors. Similar to hydrophobic moieties, this enhances cellular uptake of the compounds which allows them to act effectively at the site of formation of the protein aggregates, thereby preventing their formation, elongation and further aggregation.

Additionally polar moieties (amino, hydroxy, and carbonyl) in the backbone and side chains of the compounds, such as are present in sugar moieties, can form hydrogen bonds with exposed donor or acceptor atoms on a growing amyloid fibril, thereby inhibiting recruitment of monomers or oligomers on the fibril surface, thus blocking fibril elongation. Thus, the structural components of the compound class have a direct effect in modulating and enhancing the relevant biological properties of the compounds. A skilled person is thus also capable of making adjustments to the specific example compounds described herein, within the structural constraints of the compound class, to maintain and/or optimize the relevant effects of the compounds with respect to inhibiting protein aggregation.

In embodiments, preferred compounds of the invention relate to a ramoplanin, or derivative and/or salt thereof, such as ramoplanose, ramoplanin aglycon, ramoplanin A1-A3, enduracidin A, NAI-603 and/or enduracidin B, preferably ramoplanin A2 and/or NAI-603. Variations in the structure of the ramoplanins are possible and may be generated without undue effort by a skilled person and assessed for the relevant biological properties.

For example, in one embodiment,
a. the compound comprises 17 amino acids (numbered 1 to 17), preferably comprising 6 to 8 amino acids with an aromatic side chain, 14 to 15 non-proteinogenic amino acids,
b. the compound forms a single ring structure and is cyclized via a lactone bond formed between a carboxyl end of a terminal amino acid residue 17 with a hydroxy group of amino acid residue 2,
c. amino acid 11 of the compound is substituted with a mono-, di- or trisaccharide sugar moiety, and/or
d. amino acid 1 is substituted with a hydrophobic moiety, wherein the hydrophobic moiety is a carbonyl group C=OR, wherein R is selected from the group consisting of alkyl, alkenyl, aryl, heteroaryl, alkyl aryl, and alkyl heteroaryl (preferably C5 to C20 alkyl, C5 to C20 alkenyl, C3 to C6 cycloalkyl, or benzyl), wherein R is optionally substituted with C1-C5 alkyl, C1-C5 alkenyl or C3 to C6 cycloalkyl.

Each of these structural features a. to d. may be used in isolation, or in any sub-combination, to define the compound of the invention. In one embodiment, the compound comprises all structural features a., b., c., and d.

In one embodiment the invention relates to a compound as described herein, according to 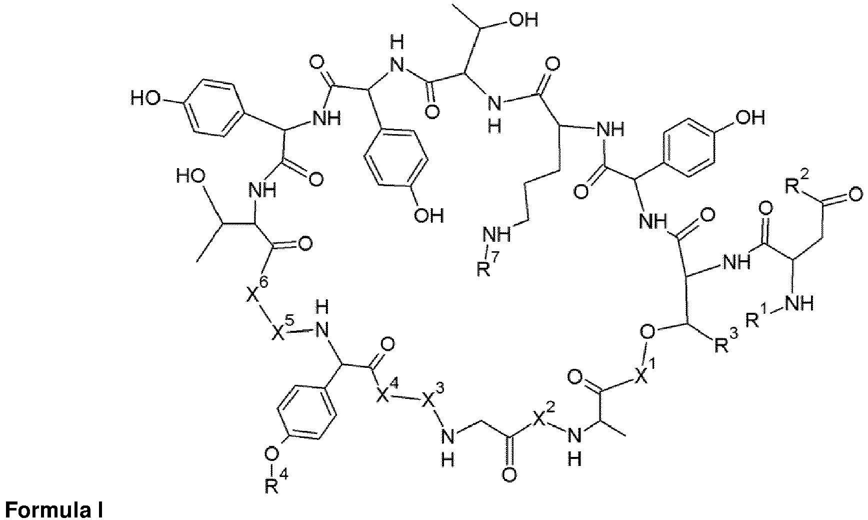 wherein:
**R¹** is a carbonyl group C=OR, wherein R is alkyl, alkenyl, cycloalkyl, aryl, heteroaryl, alkyl cycloalkyl, alkyl aryl, and alkyl heteroaryl (preferably C5 to C20 alkyl, C5 to C20 alkenyl, C3 to C6 cycloalkyl, or benzyl), wherein R is optionally substituted with C1-C5 alkyl, C1-C5 alkenyl or C3 to C6 cycloalkyl,
wherein **R¹** is preferably 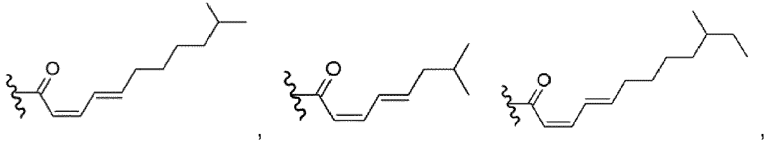 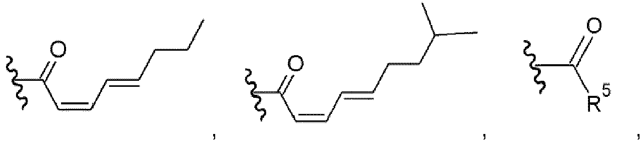 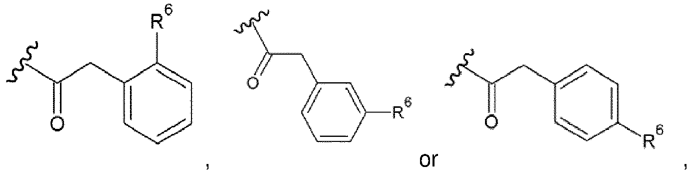
**R²** is H, -OH, an amine, an alkoxy group -OR, or a carbonyl group C=OR, wherein R is alkyl, alkenyl, cycloalkyl or aryl (preferably C1 to C12 alkyl, C3 to C6 cycloalkyl),
**R³** is H, alkyl, amide, carboxyl, carboxylate ester or an amine (preferably C1-C5 alkyl or an amide),
**R⁴** is H or a sugar moiety comprising 1 to 8 monosaccharide units (preferably comprising a mono-, di- or trisaccharide),
**R⁵** is alkyl, alkenyl, cycloalkyl, aryl, heteroaryl, alkyl cycloalkyl, alkyl aryl, and alkyl heteroaryl (preferably C5 to C20 alkyl, C5 to C20 alkenyl, C3 to C6 cycloalkyl, or benzyl), wherein R⁵ is optionally substituted with C1-C5 alkyl, C1-C5 alkenyl or C3 to C6 cycloalkyl,
**R⁶** is H, alkyl, alkenyl, aryl or cycloalkyl (preferably C1 to C12 alkyl, C3 to C6 cycloalkyl),
**R⁷** is H, alkyl, alkenyl, aryl or cycloalkyl (preferably C1 to C12 alkyl, C3 to C6 cycloalkyl),
**X¹** is an amino acid with a side chain comprising an aromatic ring, wherein the ortho-, meta- and/or para-positions of the aromatic ring are optionally substituted with halogen (preferably CI, Br, F), C1-C5 alkyl (preferably C1-C3 alkyl), C1-C5 haloalkyl or hydroxyl,
**X²** is an amino acid with a side chain comprising an alkyl group, amine, alkyl amine, amide, alkyl amide, carbamate, carboxamide or a heterocyclic ring structure (preferably comprising nitrogen),
**X³** is an amino acid with a side chain comprising an aromatic ring, wherein the ortho-, meta- and/or para-positions of the aromatic ring are optionally substituted with halogen (preferably CI, Br, F), C1-C5 alkyl (preferably C1-C3 alkyl), C1-C5 haloalkyl or hydroxyl,
**X⁴** is an amino acid with a side chain comprising an alkyl group or a hydroxyl group -R-OH, whereby R is C1 to C5 alkyl,
**X⁵** is an amino acid with a side chain comprising a comprising an alkyl group, amine, alkyl amine, amide, alkyl amide, carbamate, carboxamide or a heterocyclic ring structure (preferably comprising nitrogen),
**X⁶** is an amino acid with a side chain comprising an aromatic ring, wherein the ortho-, meta- and/or para-positions of the aromatic ring are optionally substituted with halogen (preferably CI, Br, F), C1-C5 alkyl (preferably C1-C3 alkyl), C1-C5 haloalkyl or hydroxyl, or with a side chain comprising an alkyl group, amine, alkyl amine, amide, alkyl amide, carbamate, carboxamide or a heterocyclic ring structure (preferably comprising nitrogen).

In one embodiment the invention relates to a compound as described herein, according to 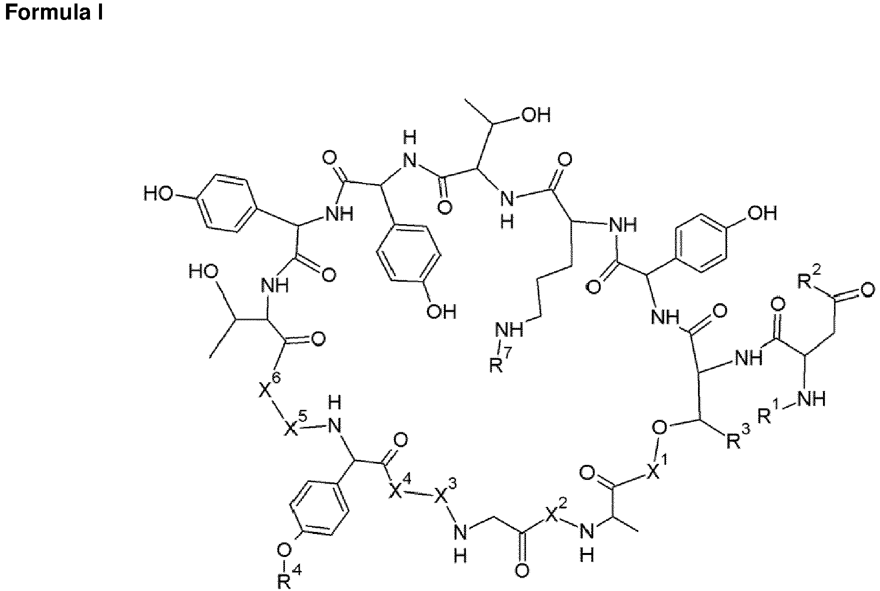 wherein:
**R¹** is 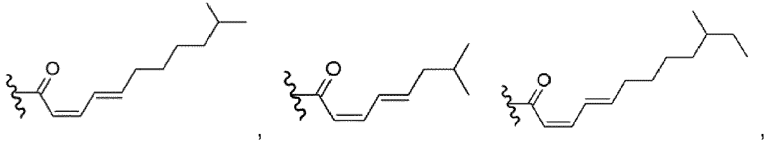 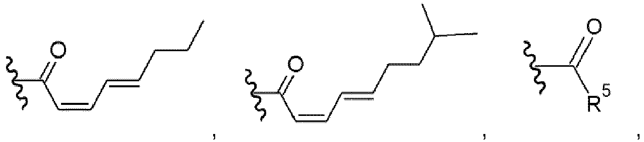 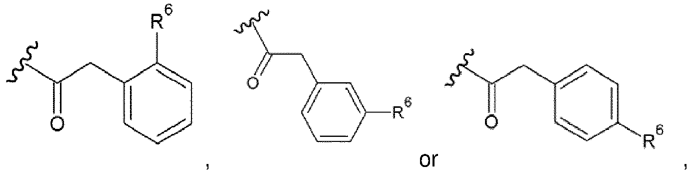
**R²** is -NH₂ or -OH,
**R³** is -CONH₂ or -CH₃,
**R⁴** is H or a mono-, di- or trisaccharide, preferably 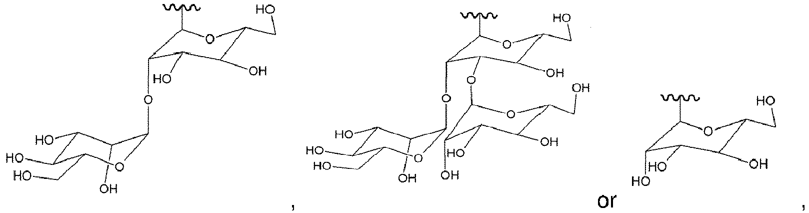
**R⁵** is C5 to C20 alkyl, C5 to C20 alkenyl, C3 to C6 cycloalkyl, or benzyl, wherein R⁵ is optionally substituted with C1-C5 alkyl, C1-C5 alkenyl or C3 to C6 cycloalkyl,
**R⁶** is H, alkyl, alkenyl, aryl or cycloalkyl (preferably C1 to C12 alkyl, C3 to C6 cycloalkyl),
**R⁷** is H, alkyl, alkenyl, aryl or cycloalkyl (preferably C1 to C12 alkyl, C3 to C6 cycloalkyl),
**X¹** is 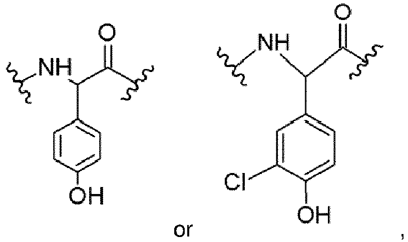
**X²** is 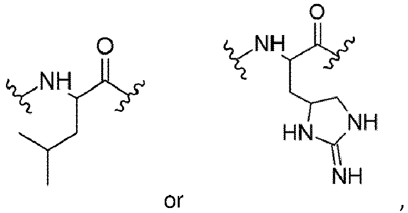
**X³** is 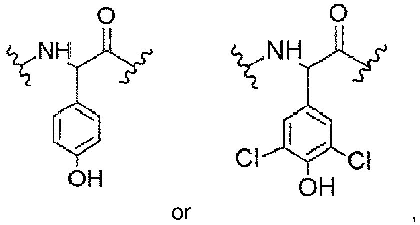
**X⁴** is 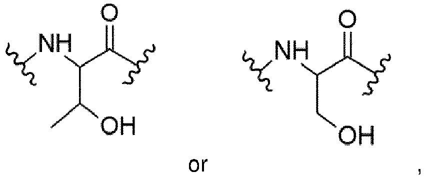
**X⁵** is 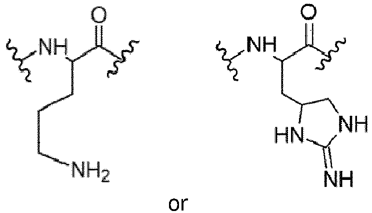
and
   **X⁶** is

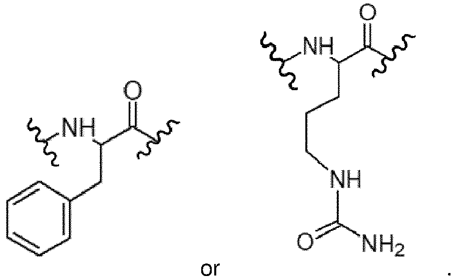

In one further embodiment the invention relates to a compound as described herein, according to 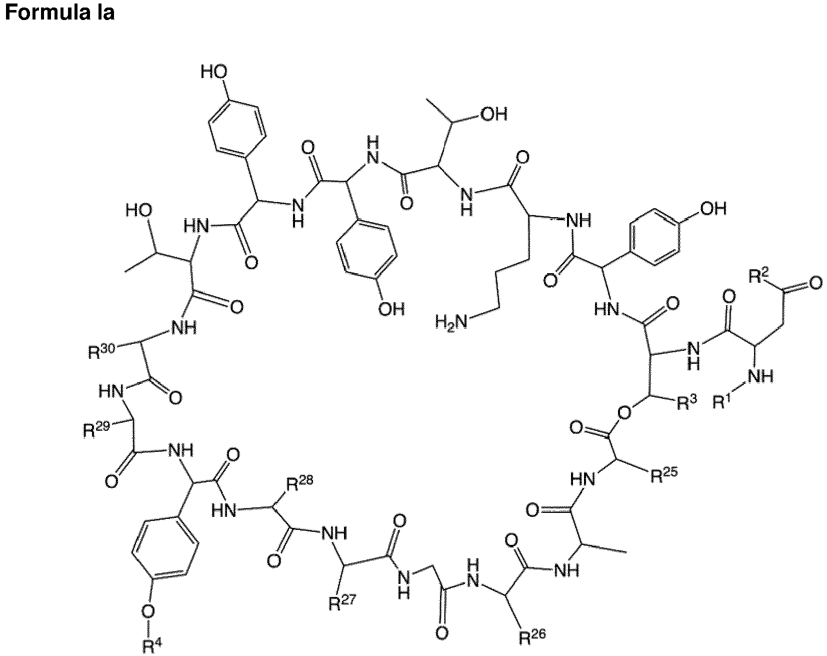 wherein:
**R¹** is a carbonyl group C=OR, wherein R is alkyl, alkenyl, cycloalkyl, aryl, heteroaryl, alkyl cycloalkyl, alkyl aryl, and alkyl heteroaryl (preferably C5 to C20 alkyl, C5 to C20 alkenyl, C3 to C6 cycloalkyl, or benzyl), wherein R is optionally substituted with C1-C5 alkyl, C1-C5 alkenyl or C3 to C6 cycloalkyl,
wherein **R¹** is preferably 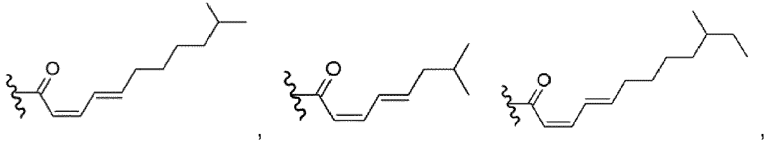 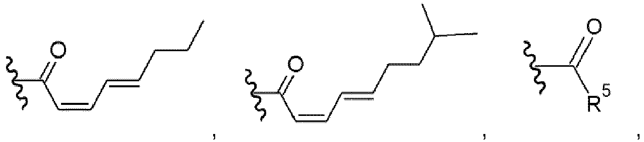 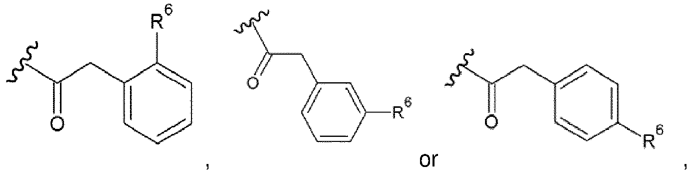
**R²** is H, -OH, an amine, an alkoxy group -OR, or a carbonyl group C=OR, wherein R is alkyl, alkenyl, cycloalkyl or aryl (preferably C1 to C12 alkyl, C3 to C6 cycloalkyl),
**R³** is H, alkyl, amide, carboxyl, carboxylate ester or an amine (preferably C1-C5 alkyl or an amide),
**R⁴** is H or a sugar moiety comprising 1 to 8 monosaccharide units (preferably comprising a mono-, di- or trisaccharide),
**R⁵** is alkyl, alkenyl, cycloalkyl, aryl, heteroaryl, alkyl cycloalkyl, alkyl aryl, and alkyl heteroaryl (preferably C5 to C20 alkyl, C5 to C20 alkenyl, C3 to C6 cycloalkyl, or benzyl), wherein R⁵ is optionally substituted with C1-C5 alkyl, C1-C5 alkenyl or C3 to C6 cycloalkyl,
**R⁶** is H, alkyl, alkenyl, aryl or cycloalkyl (preferably C1 to C12 alkyl, C3 to C6 cycloalkyl),
**R⁷** is H, alkyl, alkenyl, aryl or cycloalkyl (preferably C1 to C12 alkyl, C3 to C6 cycloalkyl),
**R²⁵** is an aromatic ring, wherein the ortho-, meta- and/or para-positions of the aromatic ring are optionally substituted with halogen (preferably CI, Br, F), C1-C5 alkyl (preferably C1-C3 alkyl), C1-C5 haloalkyl or hydroxyl,
**R²⁶** is an alkyl group, amine, alkyl amine, amide, alkyl amide, carbamate, carboxamide or a heterocyclic ring structure (preferably comprising nitrogen),
**R²⁷** is an aromatic ring, wherein the ortho-, meta- and/or para-positions of the aromatic ring are optionally substituted with halogen (preferably CI, Br, F), C1-C5 alkyl (preferably C1-C3 alkyl), C1-C5 haloalkyl or hydroxyl,
**R²⁸** is an alkyl group or a hydroxyl group -R-OH, whereby R is C1 to C5 alkyl,
**R²⁹** is an alkyl group, amine, alkyl amine, amide, alkyl amide, carbamate, carboxamide or a heterocyclic ring structure (preferably comprising nitrogen),
**R³⁰** is an aromatic ring, wherein the ortho-, meta- and/or para-positions of the aromatic ring are optionally substituted with halogen (preferably CI, Br, F), C1-C5 alkyl (preferably C1-C3 alkyl), C1-C5 haloalkyl or hydroxyl, or with a side chain comprising an alkyl group, amine, alkyl amine, amide, alkyl amide, carbamate, carboxamide or a heterocyclic ring structure (preferably comprising nitrogen).

In one embodiment the invention relates to a compound as described herein, according to 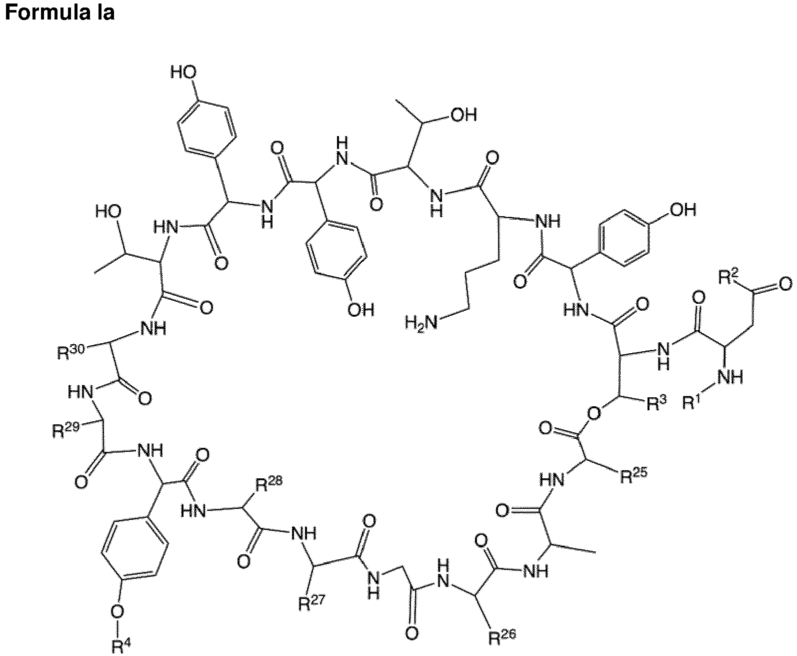 wherein:
**R¹** is 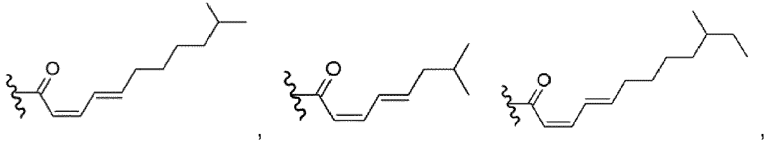 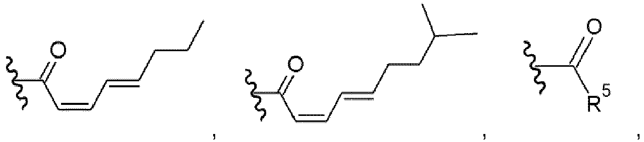 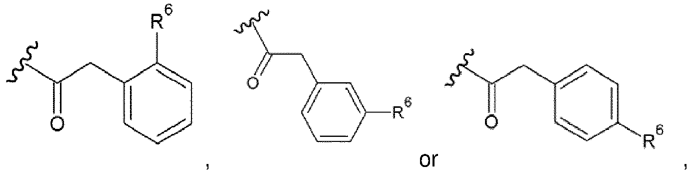
**R²** is -NH₂ or -OH,
**R³** is -CONH₂ or -CH₃,
**R⁴** is H or a mono-, di- or trisaccharide, preferably 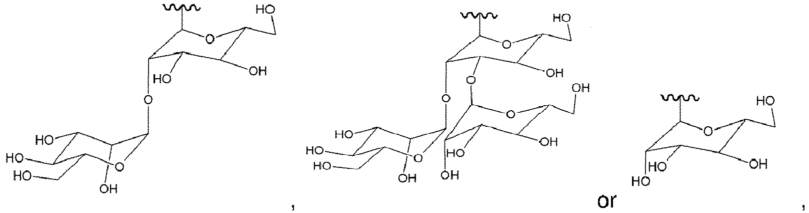
**R⁵** is C5 to C20 alkyl, C5 to C20 alkenyl, C3 to C6 cycloalkyl, or benzyl, wherein R⁵ is optionally substituted with C1-C5 alkyl, C1-C5 alkenyl or C3 to C6 cycloalkyl,
**R⁶** is H, alkyl, alkenyl, aryl or cycloalkyl (preferably C1 to C12 alkyl, C3 to C6 cycloalkyl),
**R⁷** is H, alkyl, alkenyl, aryl or cycloalkyl (preferably C1 to C12 alkyl, C3 to C6 cycloalkyl),
**R²⁵** is 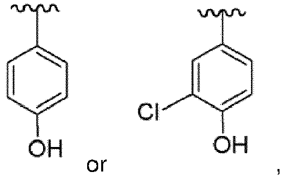
**R²⁶** is 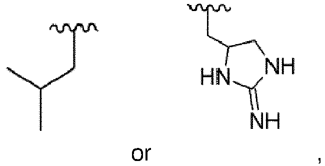
**R²⁷** is 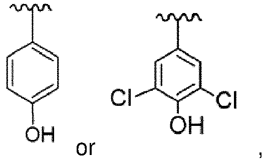
**R²⁸** is 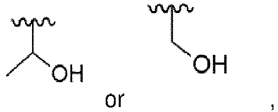
**R²⁹** is 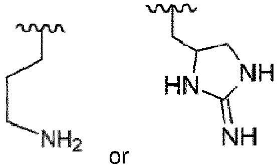 and
**R³⁰** is

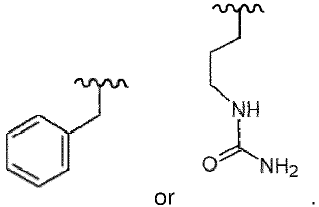

In one embodiment the compound is a Ramoplanin, or derivative and/or salt thereof, such as ramoplanose, ramoplanin aglycon, ramoplanin A1-A3, enduracidin A, NAI-603 and/or enduracidin B, preferably ramoplanin A2 and/or NAI-603.

In other embodiments, preferred compounds of the invention relate to a teicoplanin, or derivative and/or salt thereof, such as teicoplanin A2-1, teicoplanin A2-2, teicoplanin A2-3, teicoplanin A2-4, teicoplanin A2-5, ristocetin A, ristocetin B and/or vancomycin. Variations in the structure of the teicoplanins are possible and may be generated without undue effort by a skilled person and assessed for the relevant biological properties.

For example, in one embodiment
a. the compound comprises 7 amino acids (numbered 1 to 7), preferably comprising 5 to 7 amino acids with an aromatic side chain and 5 non-proteinogenic amino acids,
b. the compound comprises two to four ring structures, preferably three or four ring structures, and
c. amino acids 1, 2 and/or 4 of the compound are substituted with a sugar moiety, which may be the same or different,
wherein one or more said sugar moieties are optionally substituted with a hydrophobic moiety comprising -NHC=OR, wherein R is selected from alkyl, aryl, heteroaryl, alkyl aryl, and alkyl heteroaryl (preferably C5 to C20 alkyl, C5 to C20 alkenyl, C3 to C6 cycloalkyl, or benzyl), wherein R is optionally substituted with C1-C5 alkyl, C1-C5 alkenyl or C3 to C6 cycloalkyl.

Each of these structural features a. to c. may be used in isolation, or in any sub-combination, to define the compound of the invention. In one embodiment, the compound comprises all structural features a., b. and c.

In one embodiment the invention relates to a compound as described herein, according to 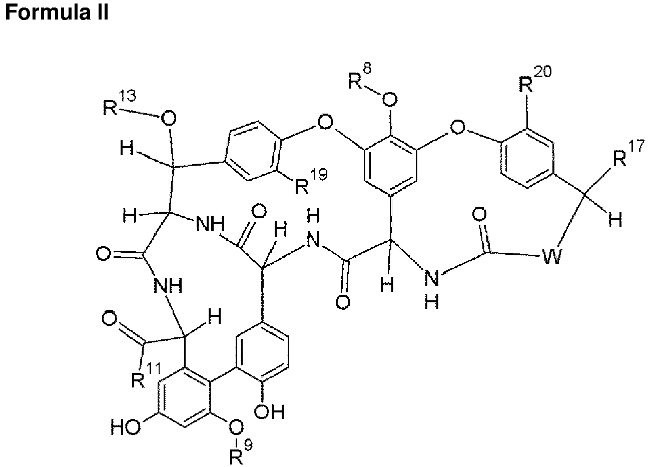 wherein
**R⁸**, **R⁹** and **R¹³** may be the same or different,
**R⁸**, **R⁹** and **R¹³** are H, or a sugar moiety comprising 1 to 8 monosaccharide units (preferably comprising a mono-, di-, tri- or tetra-saccharide), wherein at least **R⁸** is a sugar moiety, wherein **R⁸** is optionally substituted with -NH**R¹⁰**,
**R¹⁰** is a carbonyl group C=OR, wherein R is alkyl, alkenyl, cycloalkyl, aryl, heteroaryl, alkyl cycloalkyl, alkyl aryl, and alkyl heteroaryl (preferably C5 to C20 alkyl, C5 to C20 alkenyl, C3 to C6 cycloalkyl, or benzyl), wherein R is optionally substituted with C1-C5 alkyl, C1-C5 alkenyl or C3 to C6 cycloalkyl,
wherein **R¹³** is optionally substituted with an amine (-NH₂) or amide (preferably -NH-C=OCH₃),
**R¹¹** is -H, -OH, an amine, or -O**R¹²**,
**R¹²** is alkyl, alkenyl, aryl or cycloalkyl (preferably C1 to C12 alkyl, C3 to C6 cycloalkyl),
W is a linker selected from 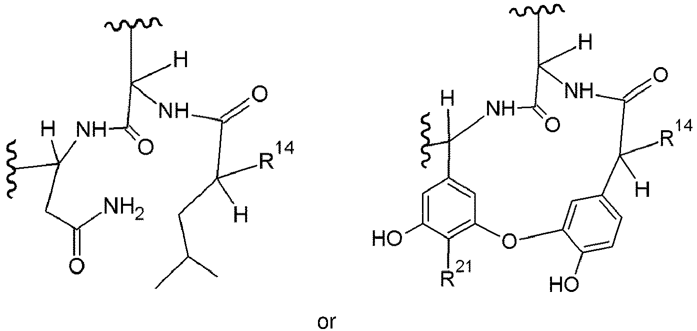
**R¹⁴** is alkyl, amine, alkyl amine, amide, alkyl amide, carbamate, carboxamide or a heterocyclic ring structure (preferably comprising nitrogen), optionally substituted with one or more halogens (preferably CI, Br, F), C1-C5 alkyl (preferably C1-C3 alkyl), C1-C5 haloalkyl, hydroxyl, alkoxy (preferably OCH₃, OCH₂CH₃), such as 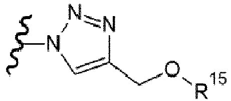
wherein **R¹⁴** is optionally substituted with **R¹⁵**,
**R¹⁵** is alkyl, aryl or cycloalkyl (preferably C1 to C12 alkyl, C3 to C6 cycloalkyl), optionally substituted with one or more halogens (preferably CI, Br, F), C1-C5 alkyl (preferably C1-C3 alkyl), C1-C5 haloalkyl, hydroxyl, alkoxy (preferably OCH₃, OCH₂CH₃), such as 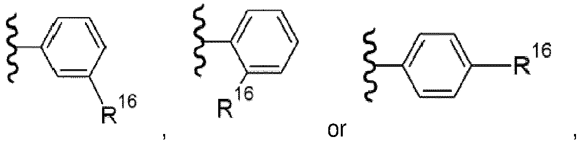 and
**R¹⁶** is alkyl, aryl or cycloalkyl (preferably C1 to C12 alkyl, C3 to C6 cycloalkyl)
**R¹⁷** is -H, -OH, or -O**R¹⁸**
**R¹⁸** is alkyl, alkenyl, aryl or cycloalkyl (preferably C1 to C12 alkyl, C3 to C6 cycloalkyl),
**R¹⁹** is -H or -a halogen (preferably CI, Br, F),
**R²⁰** is -H or -a halogen (preferably CI, Br, F),
**R²¹** is -H or alkyl, alkenyl, aryl or cycloalkyl (preferably C1 to C12 alkyl, C3 to C6 cycloalkyl).

In one embodiment the invention relates to a compound as described herein, according to 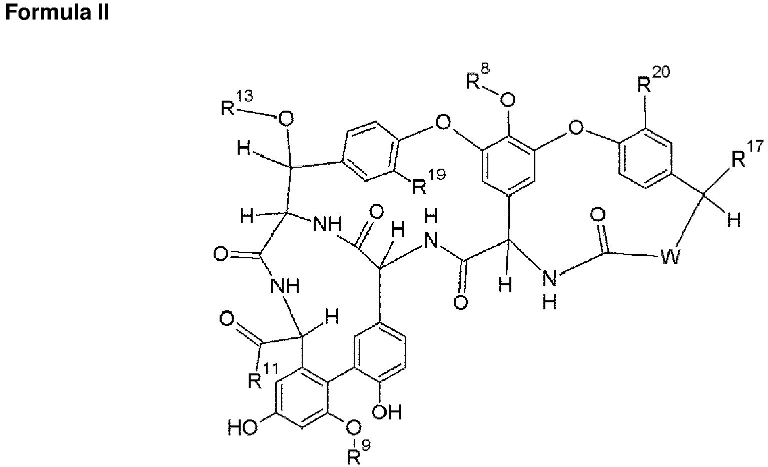 wherein
**R⁸** is 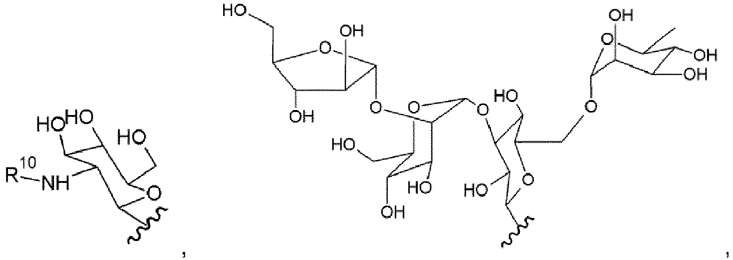 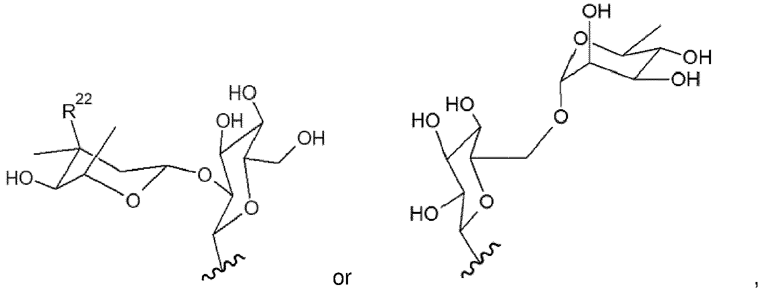
**R⁹** is 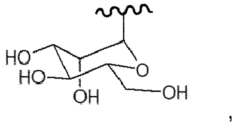
**R¹³** is 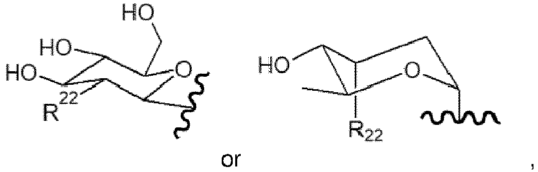
**R¹⁰** is a carbonyl group selected from: 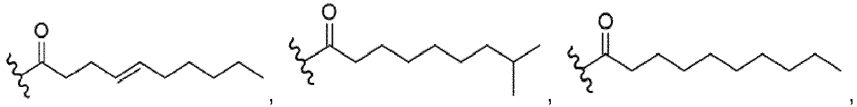 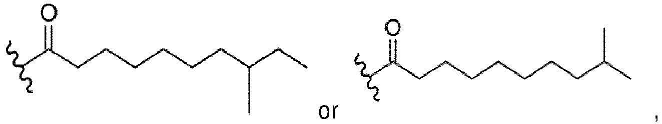
**R¹¹** is -H, -OH, or -O**R¹²**,
**R¹²** is alkyl, aryl or cycloalkyl (preferably C1 to C12 alkyl, C3 to C6 cycloalkyl),
**W** is a linker selected from 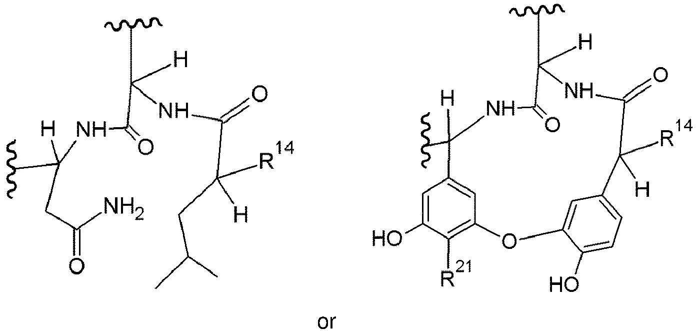
**R¹⁴** is amine (preferably NH₂), or 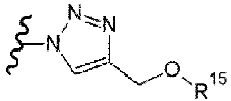
**R¹⁵** is H, alkyl, aryl or cycloalkyl (preferably C1 to C12 alkyl, C3 to C6 cycloalkyl), optionally substituted with **R¹⁶**, or is 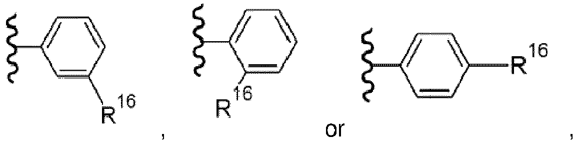 and
**R¹⁶** is alkyl, aryl or cycloalkyl (preferably C1 to C12 alkyl, C3 to C6 cycloalkyl),
**R¹⁷** is -H, -OH, or -O**R¹⁸**,
**R¹⁸** is alkyl, alkenyl, aryl or cycloalkyl (preferably C1 to C12 alkyl, C3 to C6 cycloalkyl),
**R¹⁹** is -H or -a halogen (preferably CI),
**R²⁰** is -H or -a halogen (preferably CI),
**R²¹** is -H or alkyl, alkenyl, aryl or cycloalkyl (preferably C1 to C12 alkyl, C3 to C6 cycloalkyl)
**R²²** is amine (preferably NH₂), amide (optionally -NHCOCH₃) or 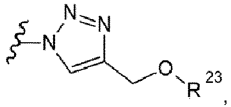
**R²³** is H, alkyl, aryl or cycloalkyl (preferably C1 to C12 alkyl, C3 to C6 cycloalkyl), or is 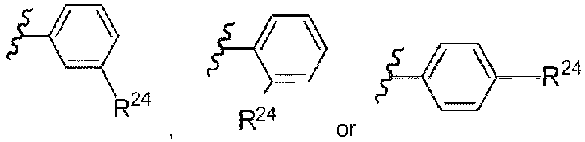 and
**R²⁴** is alkyl, aryl or cycloalkyl (preferably C1 to C12 alkyl, C3 to C6 cycloalkyl).

In one embodiment the compound is a teicoplanin, or derivative and/or salt thereof, such as teicoplanin A2-1, teicoplanin A2-2, teicoplanin A2-3, teicoplanin A2-4, teicoplanin A2-5, ristocetin A, ristocetin B and/or vancomycin.

As described herein, the compounds of the invention may be defined by their relevant biological properties. These properties may be in terms of the medical treatment of the invention and/or an in vitro property of the compound related to the desired biological effect. In embodiments, the primary biologically relevant property of the invention is the inhibition of protein aggregation.

In one embodiment, the medical treatment of the invention comprises binding of said compound to a protein and inhibiting protein aggregate formation and/or fibril elongation.

In one embodiment, the medical treatment of the invention comprises inhibition of association between monomers of aggregating proteins, inhibition of secondary nucleation processes in fibril formation, such as disrupting replication of aggregate formation on the surface of an existing fibril, and/or inhibition of fibril elongation by capping fibril ends.

As described in detail below, the medical condition to be treated is typically defined as a medical condition associated with protein aggregation. In embodiments, protein aggregation represents a pathological aspect (underlying cause) of the disease. In embodiments, the medical condition comprises protein aggregation and the treatment thus comprises the reduction and/or inhibition of protein aggregation in a subject.

In one embodiment the medical condition comprises a protein aggregate, preferably comprising one or more of medin, tau protein, α-synuclein (Asyn), transactive response DNA binding protein 43 kDa (TPD-43), superoxide dismutase (SOD1), islet amyloid polypeptide (IAPP), keratoepithelin, monoclonal immunoglobulin light chains (AL), transthyretin (TTR), tumor protein p53, serum amyloid A (AA), prion protein (PrP) and/or gelsolin (Gel).

In embodiments, the medical condition comprises a protein aggregate comprising one or more of tau protein, amyloid-β-peptide (Aβ), α-synuclein (Asyn), transactive response DNA binding protein 43 kDa (TPD-43), superoxide dismutase (SOD1), prion protein (PrP), islet amyloid polypeptide (IAPP), medin protein, an immunoglobulin light chain, immunoglobulin heavy chain, serum amyloid A (AA), β2-microglobulin (β2M), kerato-epithelin, monoclonal immunoglobulin light chains (AL), transthyretin (TTR), apo lipoprotein AI (ApoAl), apo lipoprotein AII (ApoAII), gesolin (Gel), lactoferrin (LactoF), fibrinogen Ag (Fib), lysozyme (Lys), leukocyte chemotactic factor 2 (LECT2), cystatin C (Cys), calcitonin (Cal), atrial natriuretic factor (ANF), prolactin (Pro), semenogelin (Sgl), p53, PTEN, RAD51, Nup98 and/or keratin (K).

In embodiments, the medical condition comprises amyloidosis, selected from the group of one or more of Alzheimer's disease (AD), frontotemporal dementia, primary age-related tauopathy (PART), familial British dementia (FBD), familial Danish dementia (FDD), chronic traumatic encephalopathy (CTE), progressive supranuclear palsy (PSP), corticobasal degeneration (CBD), Pick's disease (PiD), dementia with Lewy Bodies (DLB), progressive supranuclear palsy (PSP), globular glial tauopathy (GGT), tauopathy with hippocampal 4-repeat tau immunoreactive spherical inclusions, limbic-predominant neuronal inclusion body 4R tauopathy (LNT), frontotemporal dementia and parkinsonism linked to chromosome 17 (FTDP-17), argyrophilic grain disease (AGD), Huntington disease, glial globular tauopathy, neuro-astroglial tauopathy variants in the elderly, familial behavioural variant frontotemporal dementia associated with astrocyte-predominant tauopathy, amyotrophic lateral sclerosis (ALS), spinocerebellar ataxia type 11, spinal muscular atrophy (SMA), progressive ataxia and palatal tremor-related tauopathy, cerebral amyloid angiopathy (CAA), IgLON5 antibody-related tauopathy, Chromosome 21 trisomy-related Alzheimer's disease (Down's syndrome), vascular dementia, cerebral amyloid angiopathy, Gerstmann-Sträussler-Scheinker disease (GSS), Creutzfeldt-Jakob disease, fatal familial insomnia, Kuru, Niemann-Pick disease type C-related tauopathy, Nodding syndrome, Non-Guamanian motor neuron disease with neurofibrillary tangles, Parkinson's disease (PD), Parkinson's disease with dementia, Parkinsonism-dementia of Guam, Guadeloupean parkinsonism, Kosaka-Shibayama disease, post-encephalitic parkinsonism, SYNJ1 (PARK20) early-onset recessive form of parkinsonism with seizures and dystonia associated nigral tau pathology, multiple system atrophy, tau pathology associated with familial parkinsonism and progressive respiratory failure, limbic predominant neuronal-glial tau pathology in TARDBP gene mutations (I383; P112H), neuronal 4R tau pathology in fatal familial insomnia (PRNP D178N mutation), tau pathology associated with ADCY5 dyskinesia, tau pathology in chronic temporal lobe epilepsy, neurodegeneration with brain iron accumulation (NBIA), tau pathology in NBIA PANK2 and WDR45 gene mutations, tau pathology in NBIA PLA2G6 mutation, tau pathology in NBIA associated with autosomal-dominant mitochondrial membrane protein-associated neurodegeneration (MPAN), neuropil threads pretangles and neurofibrillary tangles in human immunodeficiency virus (HIV)-negative opiate abusers, tau pathology associated with acquired immunodeficiency syndrome (AIDS), diffuse neurofibrillary tangles with calcification, progressive ataxia and palatal tremor, SLC9A6-related parkinsonism, tau pathology associated with SPG7 gene mutation, striatal 4R tau pathology associated to X-linked parkinsonism with spasticity (ATP6AP2), tau pathology associated with SPAST gene-related hereditary spastic paraplegia, autism, autism spectrum disorders, retinal tauopathy, West Nile encephalomyelitis, TTBK2 gene-related spinocerebellar ataxia 11, herpes simplex encephalitis, tangle-only dementia (TOD), aging-related tau astrogliopathy (ARTAG), hippocampal tauopathy, subacute sclerosing panencephalitis (SSPE)-related tauopathy, FTLD-C9ORF72, Christianson syndrome, vacuolar tauopathy, lytico-bodig disease, ganglioglioma and gangliocytoma, meningioangiomatosis, lead encephalopathy, tuberous sclerosis complex, pantothenate kinase-associated neurodegeneration, neuronal ceroid lipofuscinosis, myotonic dystrophy, Fukuyama congenital muscular dystrophy, hemimegalencephaly, focal cortical dysplasias, Wolcott-Rallison syndrome, age-related vascular dysfunction, type 2 diabetes (T2D), ocular amyloidosis, AL-, AH-, AA-, β2M-, TTR-, ApoAl-, ApoAII-, Gel-, lactoF-, Fib-, Lys-, LECT2-, Cys-, Cal-, ANF-, Pro-, Sgl- and K-amyloidosis, preferably Alzheimer's disease (AD), Parkinson's disease (PD), amytrophic lateral sclerosis (ALS), age-related vascular dysfunction, type 2 diabetes (T2D), ocular amyloidosis, AL-amyloidosis, AA-amyloidosis, TTR amyloidosis and cancer.

In one embodiment, the medical condition is an age related vascular disfunction and/or vascular dementia, comprising amyloid protein aggregation, preferably comprising amyloid protein aggregation of a medin protein.

In one embodiment, the medical condition is Alzheimer's disease, comprising amyloid protein aggregation, preferably comprising amyloid protein aggregation of a tau protein, preferably tau self-assemblies in β-sheet-rich amyloid structures.

In one embodiment, the medical condition is Parkinson's disease, comprising amyloid protein aggregation, preferably comprising amyloid protein aggregation of a α-synuclein protein, more preferably the presence of Lewy bodies comprising aggregated α-synuclein (Asyn) protein accumulated in neurons of the subject.

In one embodiment, the medical condition is amytrophic lateral sclerosis (ALS), comprising amyloid protein aggregation, preferably comprising amyloid protein aggregation of TDP-43 and/or SOD1.

In one embodiment, the medical condition is an autoimmune disease, comprising amyloid protein aggregation, preferably diabetes mellitus, such as type 2 diabetes, preferably comprising amyloid protein aggregation of an islet amyloid polypeptide (IAPP) protein.

In one embodiment, the medical condition is an ocular disease, comprising amyloid protein aggregation, such as ocular amyloidosis, preferably comprising amyloid protein aggregation of kerato-epithelin.

In one embodiment, the medical condition is a cardiovascular, ocular and/or kidney disease, comprising amyloid protein aggregation, preferably comprising amyloid protein aggregation of monoclonal immunoglobulin light chains (AL) or transthyretin (TTR).

In one embodiment, the medical condition is an AA-amyloidosis, preferably comprising an aggregate of a serum amyloid A protein.

In one embodiment, the medical condition is an encephalopathy, comprising amyloid protein aggregation, preferably comprising amyloid protein aggregation of a prion protein (PrP), such as Creutzfeld-Jakob disease (CJD), bovine spongiform encephalopathy, chronic wasting disease or scrapie.

In one embodiment, the medical condition is a cancer, comprising amyloid protein aggregation, preferably comprising p53 amyloid aggregation.

In one embodiment, the medical condition is a cancer, comprising amyloid protein aggregation, preferably comprising PTEN amyloid aggregation.

In one embodiment, the medical condition is a cancer, comprising amyloid protein aggregation, preferably comprising RAD51 amyloid aggregation.

In one embodiment, the medical condition is a cancer, comprising amyloid protein aggregation, preferably comprising Nup98 amyloid aggregation.

In one embodiment, the medical condition is an AGel-amyloidosis, preferably comprising amyloid protein aggregation of gelsolin.

Further medical conditions associated with and/or caused by protein aggregation are known to a skilled person and may be determined without undue effort.

In one embodiment, the invention relates to a method for treating a medical condition associated with protein aggregation in a subject, comprising administering a compound as described herein to a subject in need thereof. In one embodiment, the invention relates to a method for inhibiting protein aggregation in a subject, comprising administering a compound as described herein to a subject in need thereof. In one embodiment, the invention relates to a method for reducing and/or inhibiting amyloid protein aggregation in a subject. In one embodiment, the invention relates to a method for inhibiting protein aggregate formation and/or fibril elongation. In one embodiment, the invention relates to a method for inhibiting association between monomers of aggregating proteins, inhibition of protein oligomerization, inhibition of protein condensation, inhibition of secondary nucleation processes in fibril formation, disrupting replication of aggregate formation on the surface of an existing fibril, and/or inhibition of fibril elongation by capping fibril ends, in a subject in need thereof.

The invention further relates to methods of treating a subject, and/or to methods of reducing the risk of a medical condition as disclosed herein occurring or worsening.

All features described in the present specification may be employed to define any other embodiment or aspect of the invention, for example, features used to describe the medical use of one compound may be used to describe a medical use of another compound, and vice versa. Features used to describe any one or more compounds may be combined with any one or more of the various medical uses of the invention. Similarly, structural features of the compounds may be used to describe the methods of the invention or other compounds of the invention, according to the understanding of a skilled person.

### Preferred compounds of the invention

Table 1 shows exemplary compounds of the invention, suitable for the medical use described herein. Any structure may represent an embodiment of the invention and/or be employed in any of the aspects or embodiments of the invention described herein.

**Table. 1 Preferred compounds of the invention:**

| **Compoun d No**. | **Example** - **Compound Name** | **Structure** |
|---|---|---|
| 1 | Ramoplanin A2 | 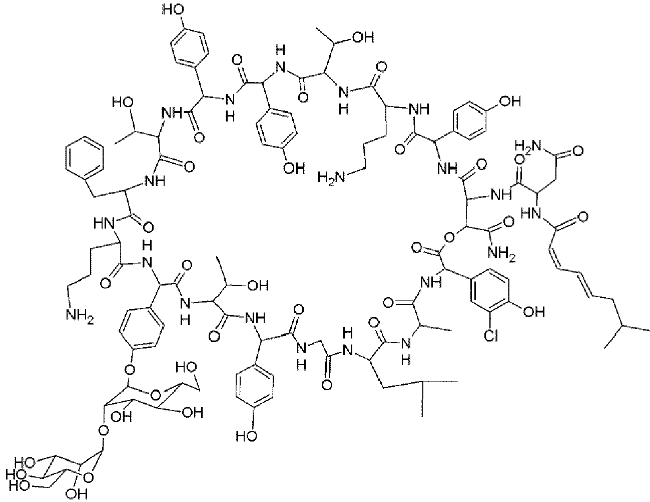 |
| 2 | Ramoplanin A1 | 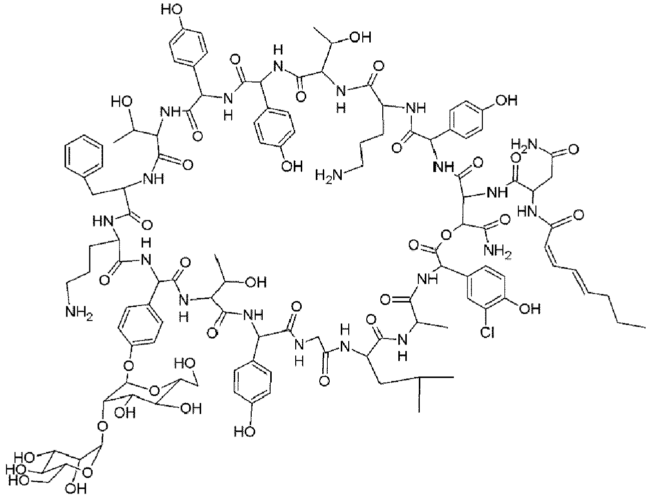 |
| 3 | Ramoplanin A3 | 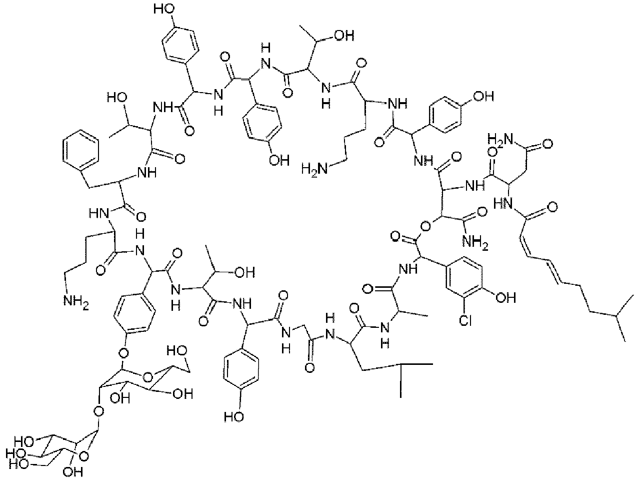 |
| 4 | Ramoplanos e | 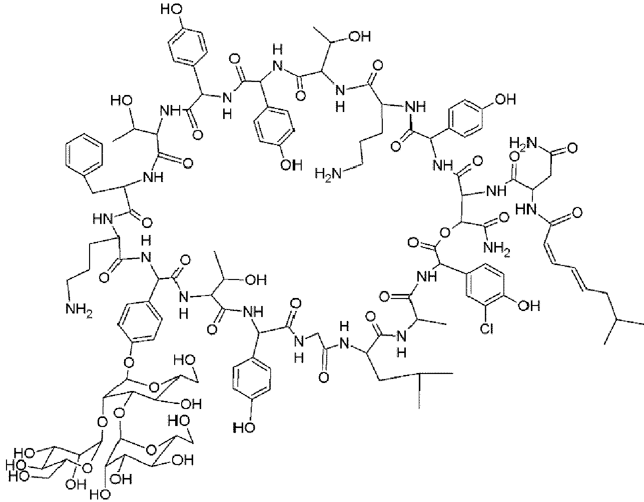 |
| 5 | Ramoplanin 2-methylphenyl derivative NAI-603 | 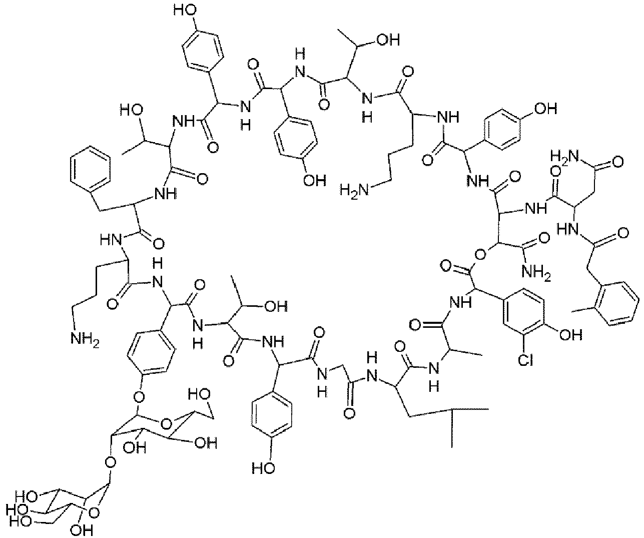 |
| 6 | Enduracidin A | 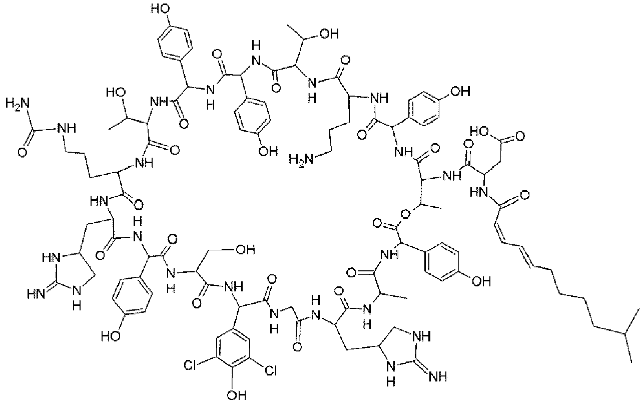 |
| 7 | Enduracidin B | 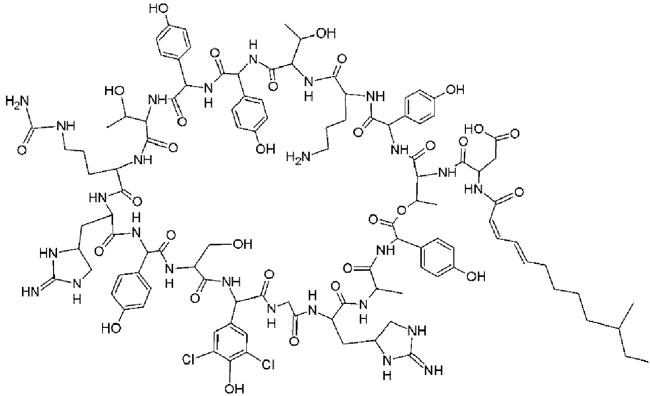 |
| 8 | Ramoplanin aglycon | 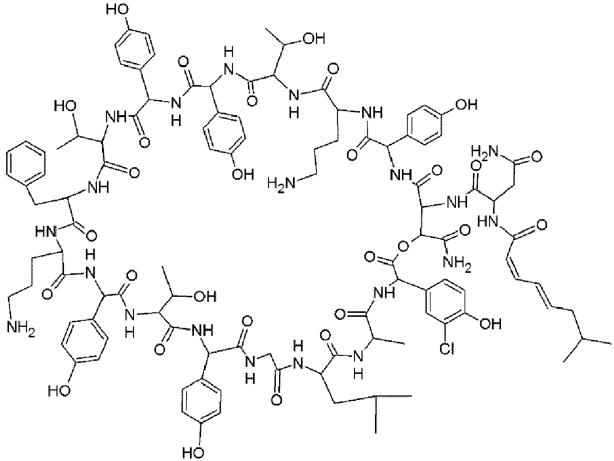 |
| 9 | Teicoplanin A2-1 | 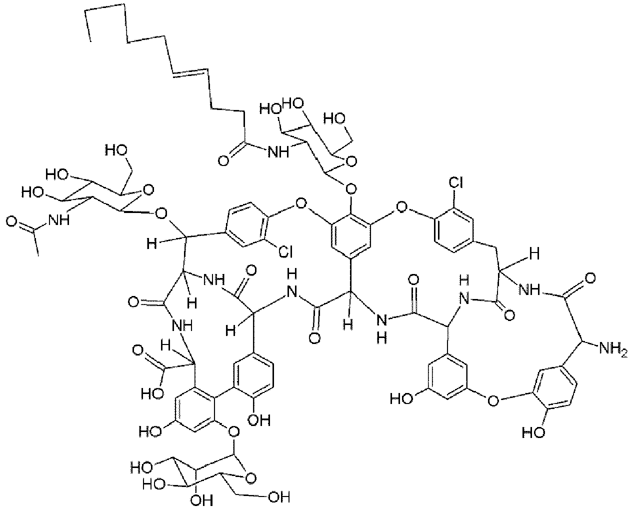 |
| 10 | Teicoplanin A2-2 | 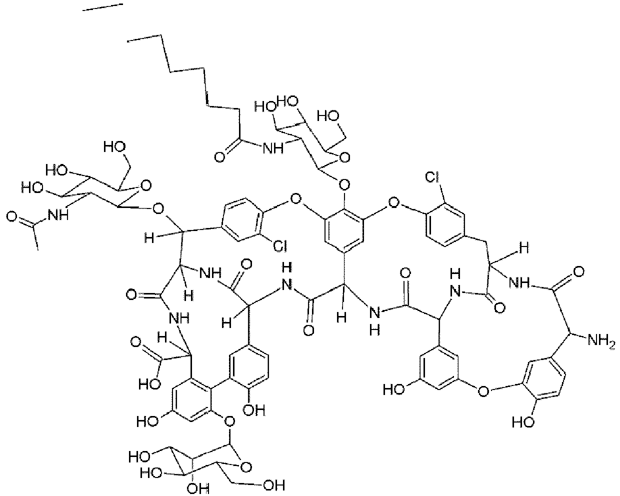 |
| 11 | Teicoplanin A2-3 | 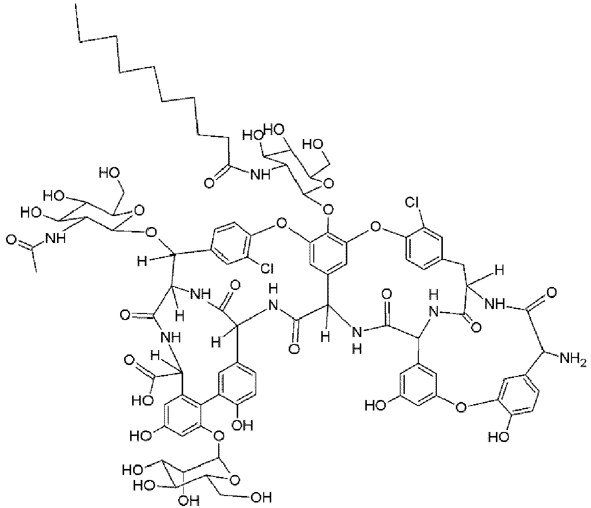 |
| 12 | Teicoplanin A2-4 | 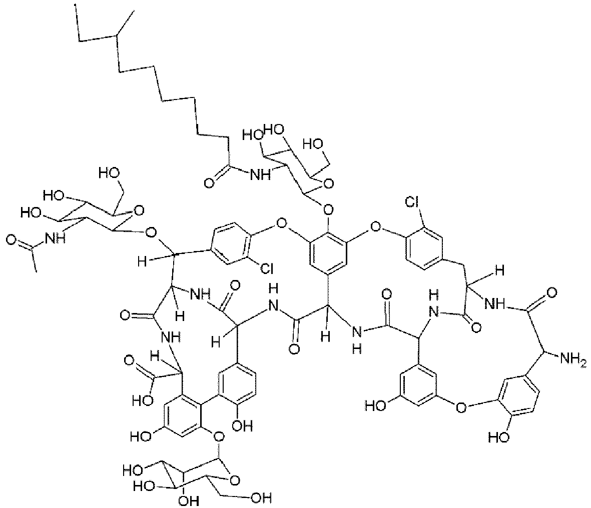 |
| 13 | Teicoplanin A2-5 | 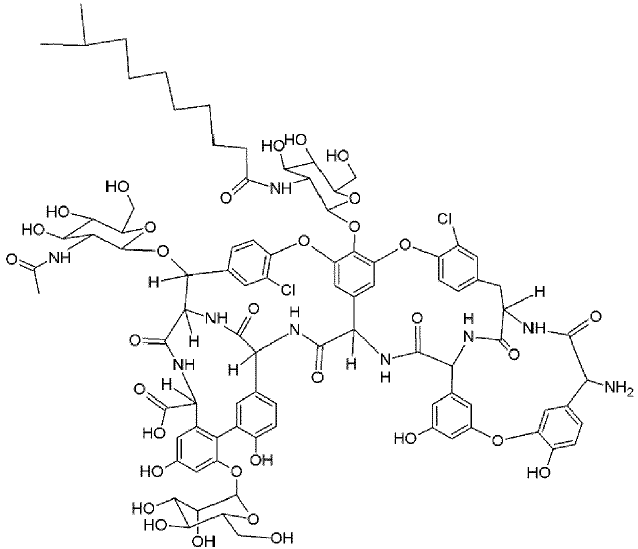 |
| 14 | Ristocetin A | 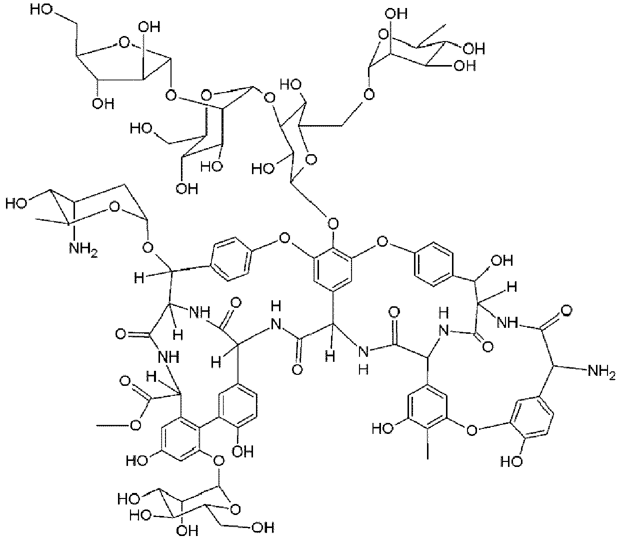 |
| 15 | Ristocetin B | 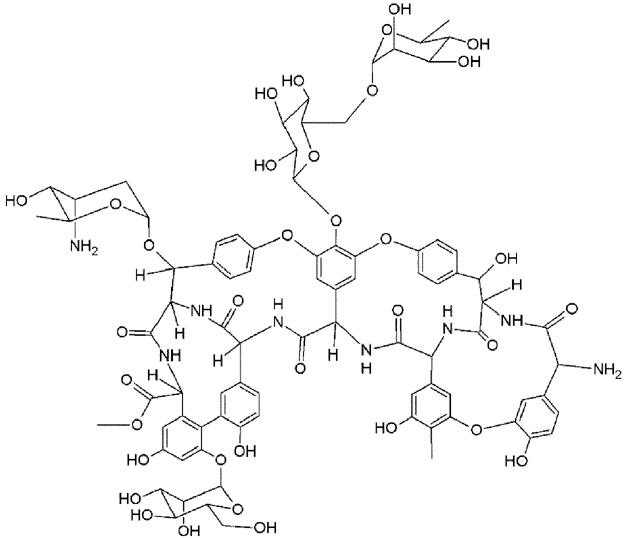 |
| 16 | Ristocetin aglycon | 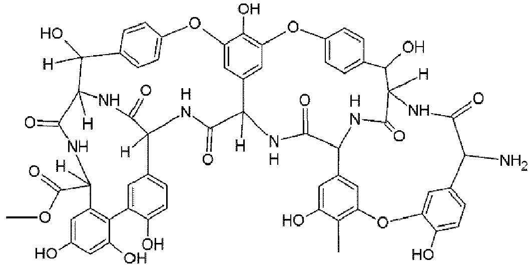 |
| 17 | Vancomycin | 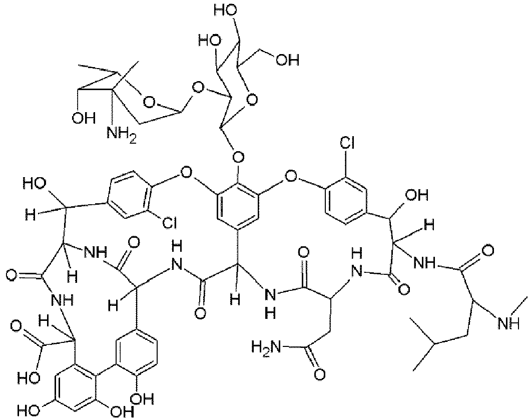 |

**Table 2: Further preferred derivatives of Ramoplanin**

| Any structure may represent an embodiment of the invention and/or be employed in any of the aspects or embodiments of the invention described herein. | | |
|---|---|---|
| **Compoun d No.** | **Example-Compoun d Name** | **Structure** |
| | General structure | 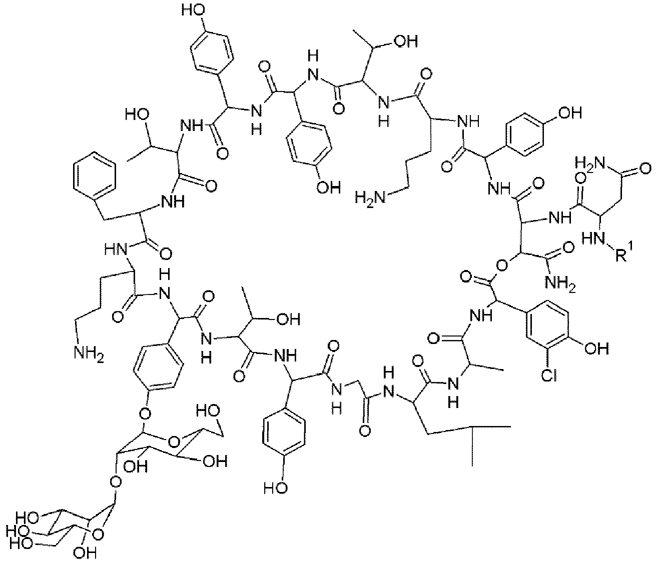 |
| 18 | R¹ | 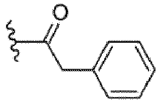 |
| 19 | R¹ | 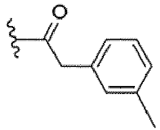 |
| 20 | R¹ | 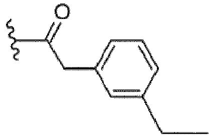 |
| 21 | R¹ | 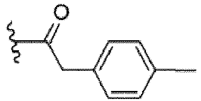 |
| 22 | R¹ | 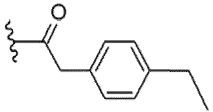 |
| 23 | R¹ | 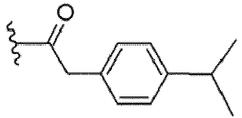 |
| 24 | R¹ | 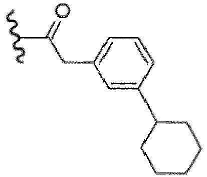 |
| 25 | R¹ | 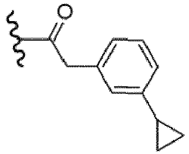 |
| 26 | R¹ | 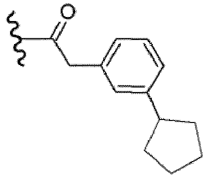 |
| 27 | R¹ | 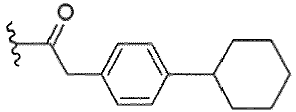 |
| 28 | R¹ | 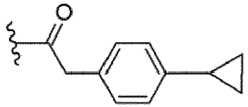 |
| 29 | R¹ | 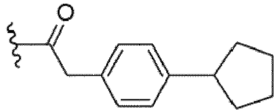 |

### DETAILED DESCRIPTION

As described in detail herein, the invention relates to a cyclic lipopeptide antibiotic compound, or derivative and/or salt thereof, for use in the treatment and/or prevention of a medical condition associated with protein aggregation.

### Compounds of the invention:

As used herein, a "peptide" "polypeptide", "polypeptide fragment" and "protein" are used interchangeably, unless specified to the contrary, and according to conventional meaning, i.e., as a sequence of amino acids. Polypeptides are not limited to a specific length, e.g., they may comprise a full-length protein sequence or a fragment of a full length protein, and may include post-translational modifications of the polypeptide, for example, glycosylations, acetylations, phosphorylations and the like, as well as other modifications known in the art, both naturally occurring and non-naturally occurring.

Protein or peptide sequence modifications, which may occur through substitutions, are also included within the scope of the invention. Substitutions as defined herein are modifications made to the amino acid sequence of the protein, whereby one or more amino acids are replaced with the same number of (different) amino acids, producing a protein which contains a different amino acid sequence than the primary protein. Substitutions may be carried out that preferably do not significantly alter the function of the peptide. Like additions, substitutions may be natural or artificial. It is well known in the art that amino acid substitutions may be made without significantly altering the protein's function. This is particularly true when the modification relates to a "conservative" amino acid substitution, which is the substitution of one amino acid for another of similar properties. Such "conserved" amino acids can be natural or synthetic amino acids which because of size, charge, polarity and conformation can be substituted without significantly affecting the structure and function of the protein. Frequently, many amino acids may be substituted by conservative amino acids without deleteriously affecting the protein's function.

In general, the non-polar amino acids Gly, Ala, Val, lie and Leu; the non-polar aromatic amino acids Phe, Trp and Tyr; the neutral polar amino acids Ser, Thr, Cys, Gin, Asn and Met; the positively charged amino acids Lys, Arg and His; the negatively charged amino acids Asp and Glu, represent groups of conservative amino acids. This list is not exhaustive. For example, it is well known that Ala, Gly, Ser and sometimes Cys can substitute for each other even though they belong to different groups.

Conservative amino acid substitutions are not limited to naturally occurring amino acids, but also include synthetic or non-proteinogenic amino acids. Commonly used synthetic amino acids are omega amino acids of various chain lengths and cyclohexyl alanine which are neutral non-polar analogues; citrulline and methionine sulfoxide which are neutral non-polar analogues, phenylglycine which is an aromatic neutral analog; cysteic acid which is a negatively charged analog and ornithine which is a positively charged amino acid analogue. Like the naturally occurring amino acids, this list is not exhaustive, but merely exemplary of the substitutions that are well known in the art.

As used herein, D amino acids are amino acids where the stereogenic carbon alpha to the amino group has the D configuration. For most naturally occurring amino acids, this carbon has the L-configuration. D amino acids are occasionally found in nature as residues in proteins. They may be formed from ribosomally-derived D amino acid residues. The compounds of the present invention may, in embodiments, comprise a mixture of D- and L-amino acids. The D or L configuration of the amino acids may however be modified, or amino acid substitutions carried out. In preferred embodiments the biological activity of the compound is maintained, namely the compound should exhibit inhibition of protein aggregation in a relevant assay, such as those described herein.

As used herein, non-proteinogenic amino acids are those that are not encoded in the human genome, and are termed unnatural amino acids. They either occur naturally (for example, in bacteria) or are chemically synthesized. Apart from glycine and the 20 natural L-amino acids, there is a variety of non-natural or unusual amino acids available that can be built into peptides. Examples include, without limitation, homo-amino acids, beta-homo-amino acids, n-methyl amino acids and alpha-methyl amino acids. Non-proteinogenic amino acids occur frequently in microbial peptides and proteins and are typically formed post-translationally. Other examples thereof are, without limitation, citrulline (Cit), hydroxyproline (Hyp), norleucine (Nle), 3-nitrotyrosine, nitroarginine, ornithine (Orn), naphtylalanine (Nal), Abu, DAB, methionine sulfoxide or methionine sulfone.

As used herein, a hydrophobic moiety is any group that is hydrophobic, i.e., hydrophobic refers to a physical property of a molecule that is seemingly repelled from a mass of water. Examples of hydrophobic molecules include, without limitation, alkanes, oils or fats. Chemical groups that tend to make substances hydrophobic include -CH2- chains and rings, such as straight chain or branched hydrocarbons and aromatic ring systems. Amino acids with a hydrophobic moiety include, without limitation, any amino acid linked to a lipid or lipid-like group.

As used herein, a lipid is a molecule (or substituent or group of a compound) that is soluble in nonpolar solvents, such as hydrocarbons used to dissolve other hydrocarbon lipid molecules that do not dissolve in water. Lipids may include fatty acids, waxes, sterols, fat-soluble vitamins (A, D, E, and K), monoglycerides, diglycerides, triglycerides, and phospholipids. Lipids may also include any hydrophobic or amphiphilic small molecule. Although the term "lipid" is sometimes used as a synonym for fats, fats are a subgroup of lipids called triglycerides. Lipids also encompass molecules such as fatty acids and their derivatives (including tri-, di-, monoglycerides, and phospholipids), as well as other sterol-containing metabolites such as cholesterol.

As used herein, a lipopeptide is any molecule comprising of a lipid connected to a peptide. Examples of lipopeptides are often bacterially produced, for example from the genus Bacillus, Pseudomonas and Streptomyces. Certain lipopeptides are used as antibiotics or as antifungal agents.

As used herein, a sugar moiety refers to its common meaning in the art, and includes monosaccharides, such as glucose, galactose, fructose, or xylose, and disaccharides, such as sucrose, lactose, maltose or trehalose, or sugar molecules with 1, 2, 3, 4, 5 or more monosaccharide units. Monosaccharides are the simplest carbohydrates in that they cannot be hydrolyzed to smaller carbohydrates. Two joined monosaccharides are called a disaccharide, and these are the simplest polysaccharides. Examples include sucrose and lactose. They are composed of two monosaccharide units bound together by a covalent bond known as a glycosidic linkage formed via a dehydration reaction. Saccharides with larger numbers of sugar units are known to a skilled person and may be incorporated in the compounds of the present invention. Trisaccharides are oligosaccharides composed of three monosaccharides with two glycosidic bonds connecting them. A tetra-saccharide is a carbohydrate which gives upon hydrolysis four molecules of the same or different monosaccharides. In embodiments amino acids of the compounds of the present invention may be substituted with one or more sugar moieties, wherein the sugar moieties comprise a mono-, di- or trisaccharides. In embodiments the one or more sugar moieties may be linked to the amino acids by an alpha- and/or beta-glycosidic linkage.

As used herein, a cyclic peptide is any polypeptide comprising a joined ring, or circular, sequence of bonds. This can be through a connection between the amino and carboxyl ends of the peptide, a connection between the amino end and a side chain, the carboxyl end and a side chain, or two side chains, or other arrangements leading to a circular structure.

As used herein, a non-ribosomal peptide (NRP) is any peptide structure produced by non-ribosomal peptide synthetases and/or including a synthetic amino acid. Regarding their structure, a non-ribosomal peptide can be identified by comprising one or more amino acids and/or peptidic structures not capable of synthesis via ribosomal translation of mRNA. Non-ribosomal peptides are typically synthesized by non-ribosomal peptide synthetases, which, unlike the ribosomes, are independent of messenger RNA. Non-ribosomal peptides typically have cyclic and/or branched structures, may contain non-proteinogenic amino acids including D-amino acids, carry modifications like N-methyl and N-formyl groups, or are glycosylated, acylated, halogenated, hydroxylated and/or otherwise modified.

As used herein, the term antibiotic refers to any antimicrobial substance active against bacteria. Antibiotics are well known agents useful for fighting bacterial infections, and antibiotic medications are widely used in the treatment and prevention of such infections. They may either kill or inhibit the growth of bacteria. In embodiments of the invention, the peptide may be antimicrobial and/or antibiotic.

A variety of laboratory methods can be used to evaluate or screen an in vitro antimicrobial or antibiotic activity of an extract or compound. The most known and basic methods are the diskdiffusion and broth or agar dilution methods. Furthermore, time-kill test and flow cytofluorometric methods are available to a skilled person, which provide information on the nature of the inhibitory effect (bactericidal or bacteriostatic) (time-dependent or concentration-dependent) and the cell damage inflicted to the test microorganism (Balouiri et al, J Pharm Anal. 2016 Apr; 6(2): 71-79). As used herein, the term "anti-microbial" or "antibiotic" describes the property of a compound or composition to reduce numbers of viable microbes, or bacteria, respectively. In embodiments, the microbe shows no or only a negligible increase in the numbers of viable microbes in the composition over a given time, for example over 14 or 28 days. In some embodiments, the term "anti-microbial" is defined according to the guidelines set out in the USP 51 test. Alternative tests may be employed, such as ASTM E2315-16, providing a standard guide for assessment of antimicrobial activity using a time-kill procedure. Antibiotic or antimicrobial susceptibility testing may also be employed to identify whether any given compound exhibits an antibiotic or antimicrobial effect on any given micro-organism, or bacteria, respectively, as reviewed in Gajic et al (Antibiotics 2022, 11, 427).

As used herein, the compounds preferably exhibit an antibiotic effect. In embodiments, the compounds or derivatives of the specific example compounds may or may not exhibit antibiotic properties. In preferred embodiments, the compound of the invention exhibits an antibiotic effect in a relevant assay, such as in an antimicrobial susceptibility test.

In embodiments, the compound of the invention exhibits an antibiotic and/or antimicrobial effect in a relevant assay of at least 0.1%, 1%, 2%, or preferably at least 5%, 10%, 20%, 30% or more, compared to one or more of ramoplanin, ramoplanose, ramoplanin aglycon, ramoplanin A1-A3, enduracidin A, NAI-603 and/or enduracidin B, teicoplanin, teicoplanin A2-1, teicoplanin A2-2, teicoplanin A2-3, teicoplanin A2-4, teicoplanin A2-5, ristocetin A, ristocetin B and/or vancomycin.

Many antibiotics exist in nature as cyclic lipopeptides, and notable examples are found in the glycolipodepsipeptide antibiotic family. For example, the antibiotic referred to as "Ramoplanin" is produced by Actinoplanes ATCC33076 and exists in three structurally related forms, A1, A2, and A2 of which A2 is the most common (Cavalleri et al., 1984; Parenti et al., 1990). These forms differ in their fatty acid chain substituents. The Ramoplanins A1-A3 are related to ramoplanose, which is sourced from Actinoplanes strain UK-71903 and differs from A2 by the addition of one mannose ring (Skelton et al., 1991).

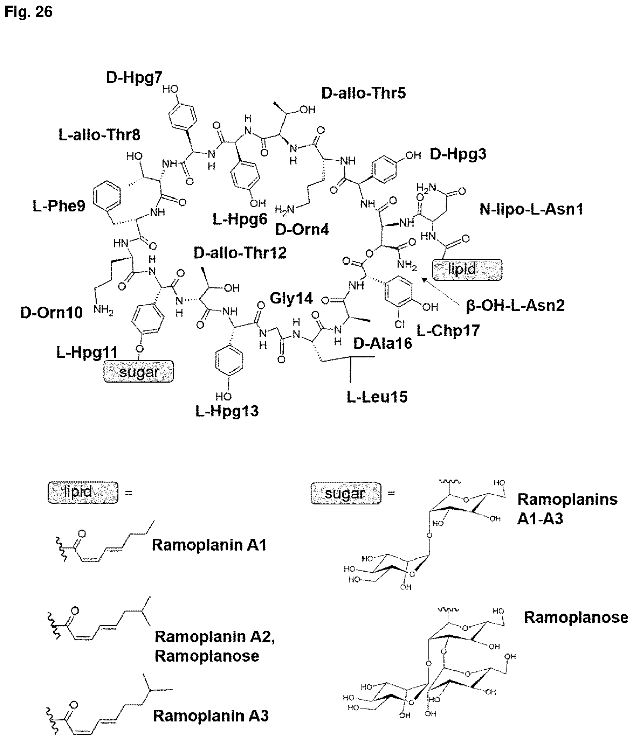

Ramoplanin (the complex of A1, A2, and A3) is known as an antibacterial agent against Gram-positive bacteria (Rolston et al., 1996) and has been studied as a candidate therapy for treating vancomycin- and β-lactam-resistant microbial infections (Reynolds et al., 1990), some of which were prevalent in hospital-acquired (nosocomial) infections in the last few decades (Swartz et al., 1994). Ramoplanin is active against vancomycin-resistant enterococci (VRE) and methicillin-resistant Staphylococcus aureus (MRSA) (von Nussbaum et al., 2006). The peptide core of Ramoplanin is a 49-membered ring made up of 17 amino acids, of which 12 are non-proteinogenic (Figure 26). Corrections regarding stereochemistry within the acyl side chain have been made through the years (Kurz et al., 1996). The peptide is cyclized via a lactone bond formed by the carboxyl end of residue 17 (L-3-chloro-4-hydroxyphenylglycine 17 or L-Chp-17) with the hydroxy group of residue 2 (β-L-hydroxyasparagine 2 or L-Asn-2). The mannose disaccharide moiety in A1-A3 is connected to the peptide via the side chain of residue 11 (L-hydroxyphenylglycine 11 or Hpg-11). The fatty acid chains are connected to the peptide core via the first residue.

Additionally, as described in the chemical formulae disclosed herein and/or in Figure 26, in embodiments, ramoplanins may exhibit structural variation at various positions of the formulae displayed in the figure. The positions indicated as "lipid" may exhibit any given lipid at said position, preferably those lipids disclosed in the text herein, more preferably those disclosed in Figure 26. The positions indicated as "sugar" may exhibit any given sugar at said position, preferably those sugars disclosed in the text herein, more preferably those disclosed in Figure 26. Also shown in the figure are the amino acids incorporated in the structure, variation in these amino acids, whilst preferably maintaining the desired biological property of the invention, also falls within the scope of the present invention.

Enduracidin lipopeptides are also part of the Ramoplanin family of antibiotics. Structurally similar to Ramoplanins A1-A3 are Enduracidin A and B, which differ from Ramoplanin in chlorination pattern, fatty acid side chain, and glycosylation Enduracidin A and B, which is produced by Streptomyces fungidicus B-5477 (Higashide et al., 1968) are additional members of Ramoplanin family that have been employed as feed additives and are known to inhibit Gram-positive bacterial cell wall biosynthesis by the same mechanism as the ramoplanins (Fang et al., 2006). It is known as an antibiotic against several Gram-positive bacteria. The antibiotic exists in two forms, Enduracidin A and Enduracidin B, which solely differ in the lipid chain substituent of the first residue (L-Asp1).

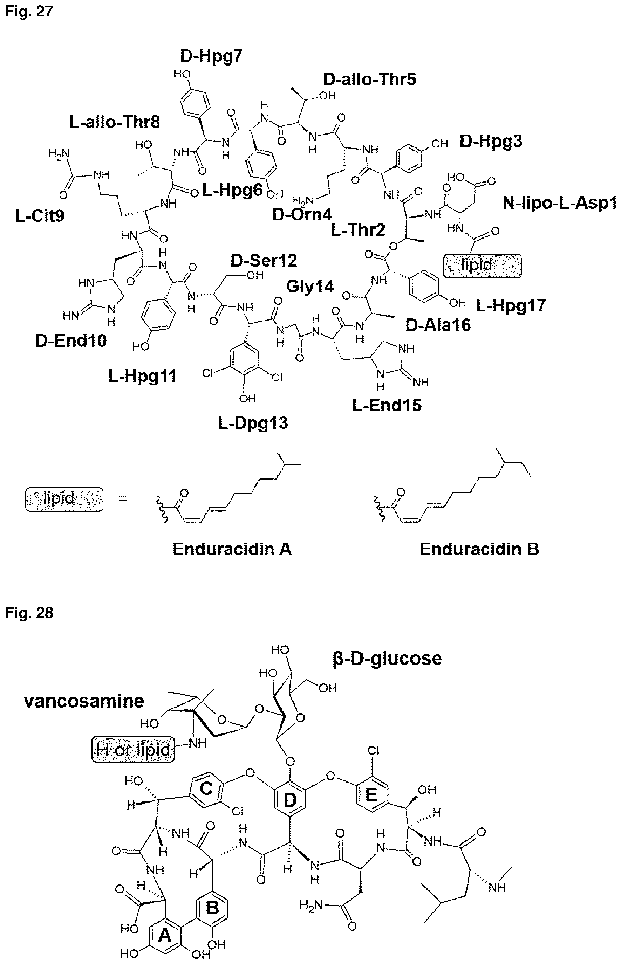

Additionally, as described in the chemical formulae disclosed herein and/or in Figure 27, in embodiments, enduracidins may exhibit structural variation at various positions of the formulae displayed in the figure. The positions indicated as "lipid" may exhibit any given lipid at said position, preferably those lipids disclosed in the text herein, more preferably those disclosed in Figure 27. Also shown in the figure are the amino acids incorporated in the structure, variation in these amino acids, whilst preferably maintaining the desired biological property of the invention, also falls within the scope of the present invention.

The Ramoplanins and Enduracidins share a high degree of structural similarity. Of note is the peptide segment from D-Hpg3 to L-*allo*-Thr8 common to Ramoplanin and Enduracidin, which is thought to function in Lipid I and II recognition and binding crucial to their bactericidal activity. Ramoplanins and Enduracidins have the same chirality at all 17 amino acids and bear the same charge (+2) at physiological pH. They both form two-antiparallel *β*-strands. Moreover, many of the remaining residues in the Enduracidins and Ramoplanins are identical (L-Hpg11, Gly14, D-Ala16) or represent conservative structural departures (D-Ser12 vs D-*allo*-Thr12, L-Dpg13 vs L-Hpg13 which differ by two chlorine atoms, L-Hpg17 vs L-Chp17 which differ by one chlorine atom, and L-Thr2 vs β-OH-L-Asn2). Even some of the significant departures (L-Cit9 vs L-Phe9 and D-End10 vs D-Orn10) represent changes that maintain the stereochemical and potential functional features of the Ramoplanins. The Enduracidins, however, do not contain a di- or trisaccharide at L-Hpg11 and the lipid substituents on the first residue are longer than those found in the Ramoplanins. Enduracidins also include an additional basic residue at position 15, L-End15 (vs L-Leu15) and an acidic residue at L-Asp1 (vs L-Asn1) that are proximal and engaged in a transannular salt bridge, as well as a flexible side chain at the L-Cit9, which is exposed to the solvent (under solution conditions H₂O-DMSO-*d*₆, 4:1). On the other hand, Ramoplanins incorporate a hydrophobic side chain at L-Phe9 forming a well-packed hydrophobic core along with other aromatic side chains (D-Hpg3, L-Chp17) and the lipid moiety (Kurz et al., 1996). Thus, the characteristic Ramoplanin hydrophobic core is disrupted within the Enduracidins (Castiglione et al., 2005). Although less well characterized, janiemycin represents an additional member of Ramoplanin family (Meyers et al., 1970).

Vancomycin, Teicoplanins, and Ristocetins share a common heptapeptide core and are members of the dalbaheptide family of cyclic antibiotics. For simplicity, these are collectively called the Vancomycin family of antibiotics.

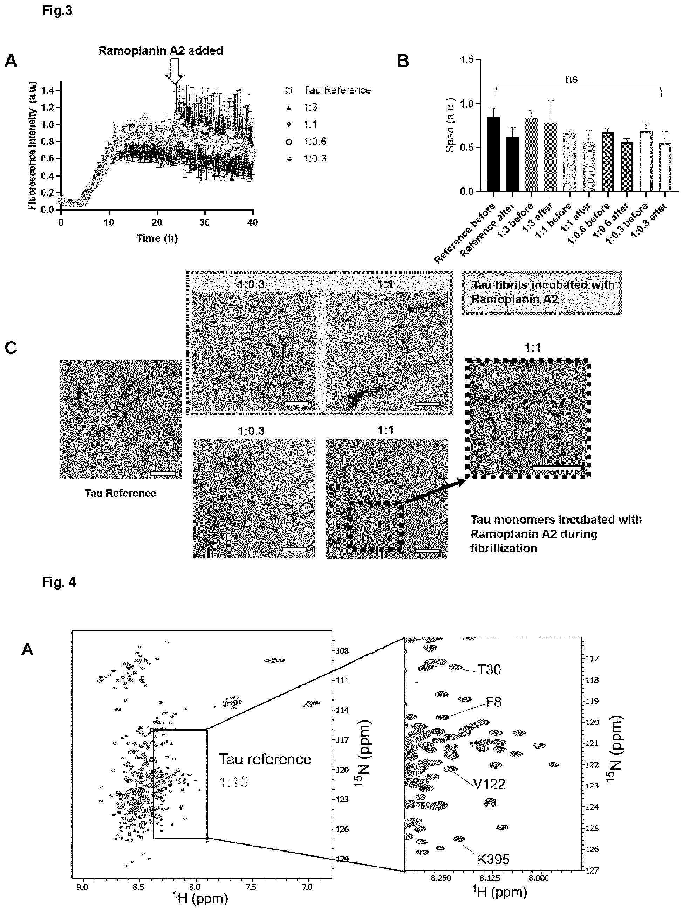

Vancomycin is a well-known glycopeptide antibiotic used in hospitals to treat infections caused by Gram-positive bacteria, especially for patients with allergies against penicillins and cephalosporins (Bruniera et al., 2015). In contrast to Teicoplanins, Vancomycin possesses one glycosylated site: a disaccharide consisting of one β-D-glucose unit bonded to the amino-sugar vancosamine (Figure 3). Although the parent structure of vancomycin does not include a lipid moiety, derivatization of the amine functional group in vancosamine with an acyl group or a lipid-like group gives rise to a lipopeptide derivative of vancomycin, such as those synthesized previously to increase the lipophilicity of the antibiotic (Ashford et al., 2012).

Additionally, as described in the chemical formulae disclosed herein and/or in Figure 28, in embodiments, vancomycin may exhibit structural variation at various positions of the formulae displayed in the figure. The position indicated as "lipid" may exhibit any given lipid at said position, preferably those lipids disclosed in the text herein. The "sugar" appended to the lipid may exhibit any given sugar at said position, preferably those sugars disclosed in the text herein, more preferably those disclosed in Figure 28.

Teicoplanin is produced by Actinoplanes teichomyceticus (Parenti et al., 1978) and exists in 5 forms: A2-1, A2-2, A2-3, A2-4, and A2-5. Teicoplanins were previously discovered to have antibacterial activity against multiple strains of streptococci and enterococci (Campoli-Richards et al., 1990). The mechanism of action of teicoplanin for exerting its bactericidal activity is similar to that of vancomycin - both types of antibiotics saturate the outer layers of the peptidoglycan structure of a bacterial cell wall, and bind to D-alanyl-D-alanine segments of peptidoglycan building blocks to inhibit cell wall biosynthesis (Campoli-Richards et al., 1990). Vancomycin and teicoplanin have been used in treatment of methicillin-resistant Staphylococcus aureaus (MRSA) and coagulase-negative staphylococci (Zeckel et al., 1994).

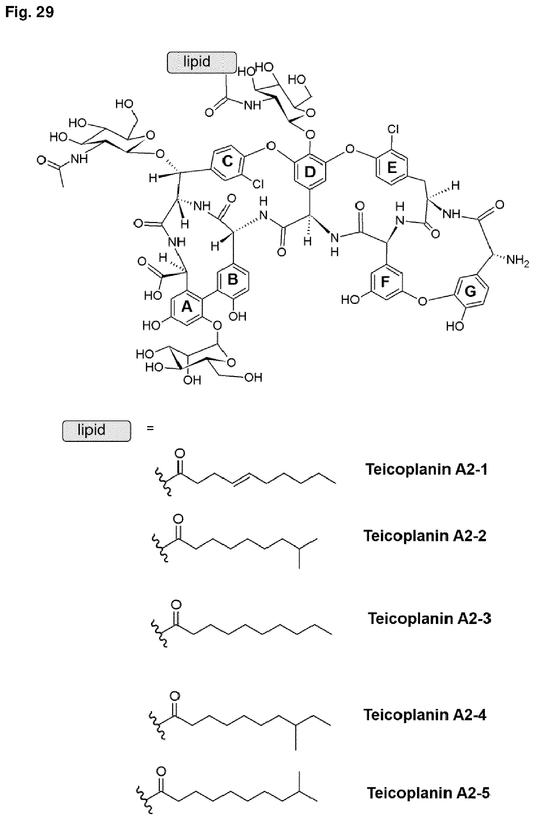

Additionally, as described in the chemical formulae disclosed herein and/or in Figure 29, in embodiments, teicoplanin may exhibit structural variation at various positions of the formulae displayed in the figure. The position indicated as "lipid" may exhibit any given lipid at said position, preferably those lipids disclosed in the text herein, more preferably those disclosed in Figure 29.

Ristocetin is an antibiotic produced by Nocardia lurida and has two structural forms, A and B (Philip et al., 1956). Similar to Teicoplanins, Ristocetins have 7 rings, A-G and have sugar groups attached to 3 separate sites within the compound. There are 6 monosaccharides in the structure: 2 mannose, one glucose, one arabinose, one rhamnose, and one ristosamine. Ristocetin is known to exert antibacterial activity through a mechanism involving binding to D-Ala-D-Ala peptide segments (Kang et al., 1987), however its use as antibiotic therapy is halted owing to its ability to cause hematologic complications (Newton et al., 1958; Gangarosa et al., 1958). Ristocetin is used as a diagnostic tool for von Willebrand's disease (Dowling et al., 1975).

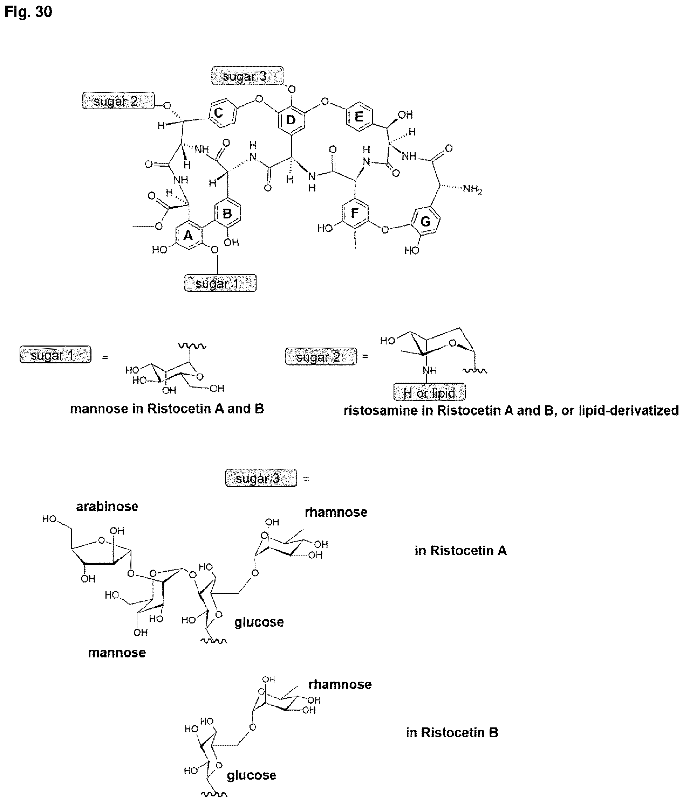

Additionally, as described in the chemical formulae disclosed herein and/or in Figure 30, in embodiments, ristocetins may exhibit structural variation at various positions of the formulae displayed in the figure. The positions indicated as "lipid" may exhibit any given lipid at said position, preferably those lipids disclosed in the text herein. The positions indicated as "sugar" may exhibit any given sugar at said position, preferably those sugars disclosed in the text herein, more preferably those disclosed in Figure 30.

Typically, the core of protein aggregates is enriched with hydrophobic side chains stacked together, and Ramoplanins/Enduracidins may associate with the exposed hydrophobic surface of the growing aggregate through pi-stacking interactions mediated by phenylglycine (hydroxy-, chlorohydroxy-, or dichlorohydroxyphenylglycine) or phenylalanine side chains. In the case of Teicoplanins, Vancomycin, and Ristocetins, the phenyl rings contribute to the hydrophobic interaction with a fibril surface.

In addition, polar moieties (amino, hydroxy, and carbonyl) in the backbone and side chains of the cyclic lipopeptides can form hydrogen bonds with exposed donor or acceptor atoms on a growing fibril, thereby inhibiting recruitment of monomers or oligomers on the fibril surface. Through these proposed mechanisms, the cyclic lipopeptides may block fibril elongation.

The candidate drug to be used in targeting protein aggregation can optionally be a cyclic lipopeptide belonging to the Ramoplanin/Vancomycin families that blocks fibril growth potentially at various stages. In the primary nucleation stage of fibrillization, monomeric proteins assemble into dimers, trimers, or other higher order structures in the process of building a β-sheet template. Such a template then recruits additional protein molecules and forms the fibril nucleus. By binding to the aggregation-prone conformation of the protein monomer, the peptide can block association between monomers or assist the formation of an off-pathway oligomer. It can also disrupt secondary nucleation processes in fibril formation, including the replication of the formation of new aggregates (including fibrils or oligomers) on the surface of an existing fibril. Moreover, the cyclic antibiotic peptide can block fibril elongation by capping the fibril ends.

### Chemical compounds, structures and substituents:

With respect to the chemical compounds described herein, the term "alkyl" refers to a branched or unbranched saturated hydrocarbon group of preferably 1 to 7 carbon atoms, such as methyl, ethyl, n-propyl, isopropyl, n-butyl, isobutyl, sec-butyl, pentyl, hexyl, heptyl, and the like. Preferred alkyl groups have 1-7 carbon atoms, more preferably 1-6, 1-5, 1-4 or 1-3, 2 or 1 carbon atoms. Any one or more of the alkyl groups described herein may be "substituted alkyls", wherein one or more hydrogen atoms are substituted with a substituent such as halogen, cycloalkyl, alkoxy, hydroxyl, aryl, or carboxyl.

The term "alkenyl" refers to a straight, branched or cyclic hydrocarbon configuration and combinations thereof, including preferably 2 to 7 carbon atoms, more preferably 2 to 4 carbon atoms, that would form if a hydrogen atom is removed from an alkene, for example resulting in ethenyl, or the like.

The term "cycloalkyl" refers to a configuration derived from a cycloalkane by removal of an atom of hydrogen, thereby forming preferably cyclopropyl, cyclobutyl, cyclopentyl, cyclohexyl or cycloheptyl, or the like.

The term "alkoxy" refers to a straight, branched or cyclic hydrocarbon configuration and combinations thereof, including preferably 1-7 carbon atoms, more preferably 1-6, 1-5, 1-4 or 1-3 carbon atoms, that include an oxygen atom at the point of attachment (such as O-alkyl). An example of an "alkoxy group" is represented by the formula -OR, or -ROR, where R can be an alkyl group, optionally substituted with halogen, aryl, cycloalkyl, halogenated alkyl. Suitable alkoxy groups include methoxy, ethoxy, n-propoxy, i-propoxy, n-butoxy, i-butoxy, sec-butoxy, cyclohexyloxy, and the like.

The term "aryl" refers to any carbon-based aromatic group including, but not limited to, benzene, naphthalene, and the like. The term "aromatic" also includes "heteroaryl group," which is defined as an aromatic group that has at least one heteroatom incorporated within the ring of the aromatic group. Examples of heteroatoms include, but are not limited to, nitrogen, oxygen, sulfur. The aryl group can be substituted with one or more groups including, but not limited to, alkyl, aryl, halogen, nitro, hydroxy, carboxylic acid, or alkoxy, or the aryl group can be unsubstituted.

The term "heteroaryl" is understood to mean saturated (heterocycloalkyl), partly unsaturated (heterocycloalkenyl) or unsaturated (heteroaryl) hydrocarbon rings containing from 3 to 15 carbon atoms in a mono- or bicyclic , fused, bridged or spirocyclic ring in which 1 to 5 carbon atoms of the 3 to 15 ring carbon atoms are replaced by heteroatoms such as nitrogen, oxygen or sulfur in which further the heteroatoms can be oxidized, for example N=O, S=O, SO2. Non-limiting examples of heterocycles are acridinyl, azaindole (1H-pyrrolopyridinyl), azabenzimidazolyl, azaspirodecanyl, azepinyl, azetidinyl, aziridinyl, benzimidazolyl, benzofuranyl, dihydrobenzofuranyl, benzothiofuranyl, benzothiophenyl, benzoxazolyl, benzthiazolyl, benztriazolyl, benztetrazolyl, benzisoxazolyl, benzisothiazolyl, carbazolyl, 4aH-carbazolyl, carbolinyl, chromanyl, chromenyl, cinnolinyl, decahydrochinolinyl, 4,5-dihydrooxazolinyl, dioxazolyl, dioxazinyl, 1,3-dioxolanyl, 1,3-dioxolenyl, 3,3-dioxo[1,3,4]oxathiazinyl, 6H-1,5,2-dithiazinyl, dihydrofuro[2,3-b]-tetrahydrofuranyl, furanyl, furazanyl, imidazolidinyl, imidazolinyl, imidazolyl, 1H-indazolyl, indolinyl, indolizinyl, indolyl, 3H-indolyl, isobenzofuranyl, isochromanyl, isoindazolyl, isoindolinyl, isoindolyl, isoquinolinyl (benzimidazolyl), isothiazolyl, isothiazolidinyl, isothiazolinyl, isoxazolyl, isoxazolinyl, isoxazolidinyl, 2-isoxazolinyl, ketopiperazinyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, 1,2-oxa-thiepanyl, 1,2-oxathiolanyl, 1,4-oxazepanyl, 1,4-oxazepinyl, 1,2-oxazinyl, 1,3-oxazinyl, 1,4-oxazinyl, oxazolidinyl, oxazolinyl, oxazolyl, oxetanyl, oxocanyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyrazolidinyl, pyrazolinyl, pyrazolyl, pyridazinyl, pyridooxazolyl, pyridoimidazolyl, pyridothiazolyl, pyridinyl, pyridyl, pyrimidinyl, pyrrolidinyl, pyrrolidinonyl, pyrrolinyl, 2H-pyrrolyl, pyrrolyl, quinazolinyl, quinolinyl, 4H-quinolizinyl, quinoxalinyl, quinuclidinyl, tetrahydrofuranyl, tetrahydroisoquinolinyl, tetrahydroquinolinyl, tetrahydrofuranyl, tetrahydropyranyl, tetrahydropyridinyl, tetrahydrothiophenyl, tetrazinyl, tetrazolyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, 1,2-thiazinyl, 1,3-thiazinyl, 1,4-thiazinyl, 1,3-thiazolyl, thiazolyl, thiazolidinyl, thiazolinyl, thienyl, thietanyl, thienothiazolyl, thienooxazolyl, thienoimidazolyl, thiomorpholinyl, thiophenolyl, thiophenyl, thiopyranyl, 1,2,3-triazinyl, 1,2,4-triazinyl, 1,3,5-triazinyl, 1,2,3-triazolyl, 1,2,3-triazolyl, 1,2,4-triazolyl, 1,2,5-triazolyl, 1,3,4-triazolyl and xanthenyl.

The term "amine" refers to a group of the formula -NRR', where R and R' can be, independently, hydrogen or an alkyl, alkenyl, alkynyl, aryl, aralkyl, cycloalkyl, halogenated alkyl, or heterocycloalkyl group described above.

The term "amide" or "amido" is represented by the formula -C(O)NRR', where R and R' independently can be a hydrogen, alkyl, alkenyl, alkynyl, aryl, aralkyl, cycloalkyl, halogenated alkyl, or heterocycloalkyl group described above. A suitable amido group is acetamido.

"Carbonyl" refers to a radical of the formula -C(O)-. Carbonyl-containing groups include any substituent containing a carbon-oxygen double bond (C=O), including acyl groups, amides, carboxy groups, esters, ureas, carbamates, carbonates and ketones and aldehydes, such as substituents based on -COR or -RCHO where R is an aliphatic, heteroaliphatic, alkyl, heteroalkyl, hydroxyl, or a secondary, tertiary, or quaternary amine, phenyl, a substituted phenyl (substituted with, for example, halogen, C1-C3 alkyl, alkoxy, amine), carboxyl, alkoxycarbonyl, amine, aryl.

The term "alkyl amino" refers to alkyl groups as defined above where at least one hydrogen atom is replaced with an amino group.

"Aminocarbonyl" alone or in combination, means an amino substituted carbonyl (carbamoyl) radical, wherein the amino radical may optionally be mono- or di-substituted, such as with alkyl, aryl, aralkyl, cycloalkyl, cycloalkylalkyl, alkanoyl, alkoxycarbonyl, aralkoxycarbonyl and the like. An aminocarbonyl group may be -N(R)-C(O)-R (wherein R is a substituted group or H) or -C(O)-N(R).

"Carboxyl" refers to a -COOH radical. Substituted carboxyl refers to -COOR where R is aliphatic, heteroaliphatic, alkyl, heteroalkyl, or a carboxylic acid or ester.

The term "hydroxyl" is represented by the formula -OH.

The term "hydroxyalkyl" refers to an alkyl group that has at least one hydrogen atom substituted with a hydroxyl group. The term "alkoxyalkyl group" is defined as an alkyl group that has at least one hydrogen atom substituted with an alkoxy group described above.

The term "aralkyl" refers to an aryl group having an alkyl group, as defined above, attached to the aryl group, as defined above. An example of an aralkyl group is a benzyl group.

Optionally substituted groups, such as "optionally substituted alkyl," refers to groups, such as an alkyl group, that when substituted, have from 1-5 substituents, typically 1, 2 or 3 substituents, selected from alkoxy, optionally substituted alkoxy, acyl, acylamino, acyloxy, amino, aminoacyl, aminoacyloxy, aryl, carboxyalkyl, optionally substituted cycloalkyl, optionally substituted cycloalkenyl, halogen, optionally substituted heteroaryl, optionally substituted heterocyclyl, hydroxy, sulfonyl, thiol and thioalkoxy.

In particular, optionally substituted alkyl groups include, by way of example, haloalkyl groups, such as fluoroalkyl groups, including, without limitation, trifluoromethyl groups. These potential optional substituents apply to any group of the formula disclosed herein where an optional substituent is recited. Preferable optional substituents are hydroxyl, alkyl, alkoxy, carbonyl, alkoxycarbonyl, NO2, amine.

The term "carbamate" is represented by the formula -NR-C(O)-O-R.

The term "aldehyde" is represented by the formula -CHO, consisting of a carbonyl center (a carbon double-bonded to oxygen) with the carbon atom also bonded to hydrogen and to an R group, preferably the backbone of the formula.

The term "carboxyester" is represented by the formula -C(O)-O-R.

The term "carboxamide" is represented by the formula -C(O)-N(R)-R.

The term "primary, secondary or tertiary amine" is represented by the formula -N(R)-R.

The term "amide amine" is represented by the formula -NH-C(O)-NH-R,

The term "carbamate" is represented by the formula -N(R)-C(O)-R.

For the definitions above, preferably the terms R, R' are independently selected from the group of H, alkyl, alkylhalo, alkoxy, or amine, and wherein X is halogen. The terms R, R' also comprise the possibility of any given group being appended to R.

Optionally substituted groups, such as "optionally substituted" refers to groups, such as an alkyl group, that when substituted, have from 1-5 substituents, typically 1, 2 or 3 substituents.

Where reference is made to "C1-C7, C1-C5 or C1-C3" alkyl, cycloalkyl, alkoxy, aryl, or the like, the number of carbon atoms C1-C7 preferably refers to each of the substituents mentioned, although in some embodiments the shorter substituents of C1-C5 or C1-C3 apply to the alkyl, cycloalkyl and/or alkoxy groups, whereby aryl may remain preferably C1-C7, such as C6 phenyl.

Further to the definitions provided herein, all chemical names and groups refer (also) to their standard meaning in the art.

Protected derivatives of the disclosed compound also are contemplated, for example for use in the synthesis of the disclosed compounds. A variety of suitable protecting groups for use with the disclosed compounds are disclosed in Greene and Wuts Protective Groups in Organic Synthesis; 3rd Ed.; John Wiley & Sons, New York, 1999. In general, protecting groups are removed under conditions which will not affect the remaining portion of the molecule. These methods are well known in the art and include acid hydrolysis, hydrogenolysis and the like.

Particular examples of the presently disclosed compounds include one or more asymmetric centers also termed chiral centers; thus these compounds can exist in different stereoisomeric forms also termed stereoisomers, including without limitation double-bond isomers (i.e., geometric isomers), regioisomers, enantiomers and/or diastereomers. The compounds of the present invention may be provided as individual pure stereoisomers (e.g., geometrically pure, enantiomerically pure or diastereomerically pure stereoisomer) or as stereoisomeric mixtures, including enantiomeric and racemic mixtures. Stereoisomeric mixtures can be resolved into their component stereoisomers using separation techniques or chiral synthesis techniques well known to the person skilled in the art. Representations of structures without specific stereochemistry encompasses any possible stereoisomeric form of the compound. Specific stereochemistry, as may be disclosed herein, represents a preferred and non-limiting embodiment of the compound structures.

The compounds of the present invention, may further exist in several tautomeric forms including the enol form, the keto form and mixtures thereof. In particular sugar moieties may be present in different tautomeric forms due to keto-enol tautomerization. This may result in the formation of 5- or 6-membered rings due to keto-enol tautomerization followed by cyclization. Accordingly, the chemical structures depicted herein encompass all possible tautomeric forms of the compound.

Specific tautomeric forms, as may be disclosed herein, represent a preferred and non-limiting embodiment of the compound structures.

The compounds of the invention may also exist in various polymorphous forms, for example as amorphous and crystalline polymorphous forms. All polymorphous forms of the compounds of the invention belong within the framework of the invention and are a further aspect of the invention.

The compound of the invention may also comprise deuterium replacing hydrogen. This replacement may in some circumstances lead to improved metabolic stability (Nature Reviews Drug Discovery 15, 219-221 (2016)).

It is understood that substituents and substitution patterns of the compounds described herein can be selected by one of ordinary skill in the art to provide compounds that are chemically stable and that can be readily synthesized by techniques known in the art and further by the methods set forth in this disclosure.

The present invention relates to pharmaceutically acceptable salts of the compounds described herein. The term "pharmaceutically acceptable salt" refers to salts or esters of the compounds described herein prepared by conventional means that include basic salts of inorganic and organic acids and bases. "Pharmaceutically acceptable salts" are also inclusive of the free acid, base, and zwitterionic forms. Descriptions of suitable pharmaceutically acceptable salts can be found in Handbook of Pharmaceutical Salts, Properties, Selection and Use, Wiley VCH (2002). For therapeutic use, salts of the compounds are those wherein the counter-ion is pharmaceutically acceptable. However, salts of acids and bases which are non-pharmaceutically acceptable may also find use, for example, in the preparation or purification of a pharmaceutically acceptable compound.

### Medical conditions:

As used herein, a medical condition associated with protein aggregation, or a protein aggregation disease, is considered any medical condition in which unwanted (pathologic) protein aggregation occurs, for example those diseases that comprise misfolding and aggregation of endogenous proteins in an affected tissue.

Notable examples of protein aggregation diseases are neurodegenerative disorders. These neurodegenerative disorders include but are not limited to Alzheimer's disease (AD), Lewy body dementia, Down syndrome, progressive supranuclear palsy, hereditary cerebral hemorrhage with amyloidosis (Dutch type), Guam Parkinson Dementia, Parkinson's disease, HIV-associated dementia, amyotrophic lateral sclerosis (ALS), and multiple sclerosis (MS). Ocular diseases associated with amyloid deposition include but are not limited to corneal lattice amyloidosis and gelsolin amyloidosis. The metabolic disorder type 2 diabetes (T2D) is also characterized by amyloid deposits, specifically derived from the islet amyloid polypeptide.

Amyloidosis refers to a subclass of disorders in which protein aggregation is an underlying disease mechanism.

Alzheimer's disease is a neurodegenerative disorder characterized by pathological hallmarks such as the accumulation of senile plaques mainly composed of amyloid β (Aβ) fibrils, and neurofibrillary tangles (NFTs) made up of tau fibrils (Serrano-Pozo et al., 2011). The formation and spread of these protein aggregates in the brain are implicated in the impairment of neurotransmitter systems and the loss of neurons. In the early stages of the disease, neural connections involved in memory, including the entorhinal cortex and hippocampus, are affected. As the disease progresses the pathology spreads to other areas of the brain such as the cerebral cortex important for language, logic, and social interactions.

The amyloid β proteins Aβ40 and Aβ42 are the main constituents of extracellular plaque in the AD brain. Serial proteolytic cleavage of the amyloid precursor protein (APP) by enzymes β- and γ- secretase yields a 37-43 residue Aβ peptide, whose length depends on the γ-secretase cleavage site (Zhang et al., 2007). APP is a transmembrane protein that functions in neuronal development, axonal transport, and neurite outgrowth (Kang et al., 1987). Aβ42 is more prevalent than Aβ40 in amyloid plaques and is also more aggregation-prone presumably owing to its highly hydrophobic residues in the carboxy-terminal region of the peptide. The main component of neurofibrillary tangles (NFTs) in AD is the microtubule-associated protein tau. Multiple lines of structural evidence have revealed that tau self-assembles through various intermediates into β-sheet-rich amyloid structures (Nizynski et al., 2017). Tau aggregates can be in the form of paired helical filaments (PHFs) modeled as two intertwined fibrils, straight filaments (SFs), and ribbonlike fibrils (Goedert et al., 1998). The aggregation of Tau into oligomers, protein-rich condensates or highly ordered amyloid/amyloid-like structures characterizes a class of neurodegenerative disorders termed tauopathies, which includes AD. Other examples of tauopathies include but are not limited to Pick's disease (PiD), progressive supranuclear palsy (PSP), corticobasal degeneration (CBD), and argyrophilic grain disease (AGD). Previous studies based on tau aggregate uptake or "seeding" in cultured cells suggest that amyloid fibril seeds of Tau mediate the propagation of amyloid pathology in the brain (Frost et al., 2009; Guo et al., 2011). The mechanisms of propagation may resemble those of pathological prion protein (PrP) (Sander et al., 2014).

Prion diseases, also termed transmissible spongiform encephalopathies or TSEs, are a class of diseases in animals and humans characterized by the accumulation of cerebral aggregates composed of misfolded prion proteins. Examples of prion diseases include but are not limited to Creutzfeldt-Jakob disease (CJD), bovine spongiform encephalopathy, chronic wasting disease, and scrapie (Soto et al., 2018). In such diseases the infectious agent is an aggregated form of PrP called a prion. Exposure to a prion initiates the process of protein misfolding and aggregation in the infected host, and eventually the prion pathology spreads in the host by cell-to-cell contact or through biological fluids (Soto et al., 2012). The most common form of human TSE is Creutzfeldt-Jakob disease (Ladogana et al., 2005). CJD may be classified as sporadic, genetic, or acquired CJD. Clinical symptoms of CJD include ataxia, mutism, and rapidly progressing dementia (Manix et al., 2015). Neuropathology of CJD is characterized by loss of neurons, spongiform change, and astrocytic gliosis (Knight et al., 2004). Genetic CJD is caused by a mutation in the PRNP gene encoding normal cellular prion protein (PrP^{c}). In sporadic CJD, the infectious seed is the scrapie form of the cellular prion protein that causes a posttranslational modification in PrP^{c}, resulting in the disease related form, PrP^{Sc}. Variant CJD can be acquired by eating beef infected with bovine spongiform encephalopathy (BSE, commonly called "mad cow disease" (Knight et al., 2004)).

Parkinson's disease (PD) is a debilitating neurodegenerative disease that is clinically characterized by impaired movement, including tremors, loss of balance and coordination, and slurred speech. The pathological hallmark of PD is the formation of Lewy bodies - lesions of misfolded, aggregated α-synuclein (Asyn) protein that accumulate in neurons (Dickson et al., 2012). PD belongs to a group of diseases collectively referred to as "synucleinopathies" since their pathogenesis involves Asyn aggregates. Human α-synuclein is a 140-residue protein encoded by the SNCA gene (Jakes et al., 1994; Lavendan et al., 1998). Asyn under physiological conditions exists in an equilibrium between a cytoplasmic disordered state and a membranebound helical form whereas in the pathological state, Asyn can form oligomers and amyloid fibrils that accumulate into insoluble deposits (Burre et al, 2018)

Examples of ocular amyloidosis include corneal lattice dystrophy and gelsolin amyloidosis. Corneal lattice dystrophy is an inherited disease caused by mutations in the transforming growth factor-beta induced (TGFBI) gene, characterized by linear, "lattice-like" amyloid deposits in the cornea that leads to loss of vision (Moshirfar et al., 2021). The primary type of corneal lattice dystrophy is Type I or Biber-Haab-Dimmer dystrophy (Klinthworth et al., 2003; Weiss et al., 2015). The *TGFBI* gene encodes for keratoepithelin, an extracellular matrix protein expressed in the corneal epithelium and other tissues (Skonier et al., 1992; Escribano et al., 1994). Other related diseases overlapping with the corneal lattice dystrophy classification are familial amyloid polyneuropathy (FAP) type IV (formerly called corneal lattice dystrophy type II), FAP Finnish type, Meretoja's syndrome, and granular corneal dystrophy type II (Weiss et al., 2015). Gelsolin amyloidosis, also called Meretoja syndrome is characterized by the deposition of gelsolin-based amyloid fibrils composed of misfolded gelsolin fragments (Solomon et al., 2012). It is a hereditary disease associated with pathogenic mutations in the GSN gene, which encodes the regulatory protein gelsolin (Solomon et al., 2012). Gelsolin is a calcium-sensitive actin-regulating protein that functions in cell signaling and motility (Kwiatkowski et al., 1999; Bucki et al., 2008).

Amyloidogenic proteins can accumulate in the myocardium to cause cardiac amyloidosis (Kittleson et al., 2020). The two main amyloid proteins implicated in this class of diseases are monoclonal immunoglobulin light chains (AL) or transthyretin (TTR). AL cardiac amyloidosis is caused by misfolded AL produced by clonal plasma cells and affects the heart and/or kidneys (Grogan et al., 2017). Transthyretin amyloidosis (ATTR) can be caused by pathogenic mutation in the TTR gene encoding transthyretin or by the accumulation of wild-type transthyretin protein (Kittleson et al., 2020). ATTR is a multisystemic disease and can spread to the nervous system. The potential of interplay between amyloid-related diseases is manifested in the involvement of TTR in glaucoma, in cases of transthyretin-related familial amyloidotic polyneuropathy (Colligris et al., 2018). The amyloid precursor lactadherin (also called milk fat globule EGF factor 8, MFG-E8) is expressed in multiple cell types including vascular smooth cells. Processing of lactadherin forms medin, the most common amyloid in humans. Medin has been found to form amyloid in arteries or middle-aged individuals (Peng et al., 2005) and in human brain vasculature (Wagner et al., 2020). Medin amyloid formation is implicated in cerebral β-amyloidosis, AD, and vascular dementia (Wagner et al., 2020; Migrino et al., 2020).

The accumulation of islet amyloid deposits, of which islet amyloid polypeptide (IAPP) is the main component, is a key pathological feature of the pancreas in type 2 diabetes (Kahn et al., 1999). IAPP is also known to form fibril aggregates in insulinoma (Westermark et al., 1987). IAPP is found in pancreatic islet β-cells together with insulin, and is co-secreted with insulin as a result of glucose-induced stimulation of β-cells (Kahn et al., 1990). IAPP functions in carbohydrate metabolism although its role is not yet clearly delineated (Marzban et al., 2003). Several studies support the correlation between islet amyloid deposition with the development and progression of type 2 diabetes (Westermark et al., 1994; Clark et al., 1988).Notably, islet amyloid has been detected postmortem in type 2 diabetes patients. The precursor of IAPP, prolAPP (67 residues) is synthesized mainly by pancreatic β-cells and cleaved to form IAPP in β-cell secretory granules by convertase enzymes (Kahn et al., 1999; Wang et al., 2001).

Recent evidence shed light on the role of mutant p53 aggregation in tumor growth (Kanapathipilla et al., 2018). Mutants such as R248Q, R248W, and R175H have been reported to form aggregates in tumor samples (Sorangi et al., 2016; De Smet et al., 2017) and the aggregation behavior may follow a prion-like mechanism (Poduslo et al., 1999).

Prion and prion like diseases, which result from the misfolding and aggregation of prion proteins, have been demonstrated in transmissible spongiform encephalopathies and various neurodegenerative diseases. This concept has been extended to the field of oncology, and a variety of important proteins, such as the tumor suppressors p53, PTEN and RAD51 have been found to be pathologically aggregated in tumors, leading to functional alterations and tumor progression. It has been found that malignant tumors characterized by p53 mutations may share a common propagation mechanism with neurodegenerative diseases, and p53 can aggregate into structures such as oligomers or amyloid fibrils, which in turn can affect its normal suppressor function. p53 aggregation has been found in various tumors, and wild-type p53 was found to be aggregated in neuroblastoma, breast cancer, colon cancer, and retinoblastoma. The accumulation of p53 aggregates was detected in paraffin-embedded breast tumor biopsies and basal cell carcinoma (BCC) samples (Li et al, Experimental Hematology & Oncology, 2022, 11:66). p53 mutations are common in tumors, and more than 200 different single-site mutations have been reported successively. For example, sites R175, G245, R248, R249 and R282 are known as structural mutations because of the importance for the structural stability of p53. Such mutations may be associated with p53 protein aggregation in cancer. The three functional domains of p53, transactivation domain (TAD), DBD, and OD, have been demonstrated to form amyloid aggregates in vitro, and the highest aggregation propensity remains in the DBD region, which has the most hotspot mutations. Cancers with p53 amyloid aggregation may be treated by the compounds described herein.

Other proteins may form amyloid aggregates in cancer. For example, RAD51 is a central protein of homologous recombination (HR) and homologous DNA repair processes. RAD51 has a natural tendency to form heterogeneous aggregates, but its filament behavior in the cell seems to be tightly controlled. Studies now show that RAD51 can form amyloid-like aggregates in vitro. RAD51 aggregates exhibit all the properties of amyloids, including resistance to detergents, birefringence in polarized light, binding to ThT and the X-ray diffraction pattern typical for amyloids (Kachkin et al, Int. J. Mol. Sci. 2022, 23, 11657). Cancers with RAD51 amyloid aggregation may be treated by the compounds described herein.

Other forms of protein association between monomers of aggregation-prone proteins occur in aggregates and/or condensates of the nucleoporin Nup98. In certain types of leukaemia, the FG-repeat domain of Nup98 is fused to a chromatin-binding domain as a result of recurrent chromosomal translocations. The oncogenic properties of the Nup98 fusion proteins are related to their ability to concentrate into condensates and/or aggregates. Site-directed mutagenesis demonstrates that the ability to self-associate and form oncogenic transcription factor condensates critically depends on the FG repeats of Nup98. Consistent with the formation of Nup98 condensates and/or aggregates in cells, the FG repeats of several nucleoporins phase separate in vitro into liquid-like droplets and solid-like condensates above critical concentrations as low as 20 nM. The FG-repeat domain of Nup98 also facilitates the aggregation of the protein tau associated with Alzheimer's disease in vitro and accumulates in the cell bodies of neurons that contain tau aggregates.

### Compositions and modes of treatment:

Another aspect of the disclosure includes pharmaceutical compositions prepared for administration to a subject and which include a therapeutically effective amount of one or more of the compounds disclosed herein.

Within the pharmaceutical compositions the compounds of the present invention are referred to as "active ingredient". The term "active ingredient" or "API" herein refers to a pharmaceutically active molecule as well as a pro-drug transformed to the pharmaceutically active molecule in a subject and a pharmaceutically acceptable and/or therapeutically active salt thereof. The term further refers to pharmaceutically acceptable and therapeutically active hydrates, esters, amides, metabolites, stereoisomers such as enantiomers, polymorphs, analogs, etc. that induce a desired pharmacological or physiological effect or induce a desired pharmacological or physiological effect upon transformation to a pharmaceutically active molecule in the subject. Terms like "active agent", "active pharmaceutical ingredient", "drug substance", "active drug", "pharmacologically active agent", "pharmaceutically active molecule", "therapeutically active molecule", "therapeutically active drug" may be used synonymously for "active ingredient"

The therapeutically effective amount of a disclosed compound will depend on the route of administration, the species of subject and the physical characteristics of the subject being treated. Specific factors that can be taken into account include disease severity and stage, weight, diet and concurrent medications. The relationship of these factors to determining a therapeutically effective amount of the disclosed compounds is understood by those of skill in the art.

Pharmaceutical compositions for administration to a subject can include at least one further pharmaceutically acceptable additive such as carriers, thickeners, diluents, buffers, preservatives, surface active agents and the like in addition to the molecule of choice. Pharmaceutical compositions can also include one or more additional active ingredients such as antimicrobial agents, anti-inflammatory agents, anesthetics, and the like. The pharmaceutically acceptable carriers useful for these formulations are conventional. Remington's Pharmaceutical Sciences, by E. W. Martin, Mack Publishing Co., Easton, PA, 19th Edition (1995), describes compositions and formulations suitable for pharmaceutical delivery of the compounds herein disclosed.

In general, the nature of the carrier will depend on the particular mode of administration being employed. For instance, parenteral formulations usually contain injectable fluids that include pharmaceutically and physiologically acceptable fluids such as water, physiological saline, balanced salt solutions, aqueous dextrose, glycerol or the like as a vehicle. For solid compositions (for example, powder, pill, tablet, or capsule forms), conventional non-toxic solid carriers can include, for example, pharmaceutical grades of mannitol, lactose, starch, or magnesium stearate. In addition to biologically-neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

The pharmaceutical compositions can be administered to subjects by a variety of mucosal administration modes, including by oral, rectal, intraocular, intranasal, intrapulmonary, or transdermal delivery, or by topical delivery to other surfaces. Optionally, the compositions can be administered by non-mucosal routes, including by intramuscular, intraocular, subcutaneous, intravenous, intra-arterial, intra-articular, intraperitoneal, intrathecal, intracerebroventricular, or parenteral routes.

The compositions of the disclosure can alternatively contain as pharmaceutically acceptable carrier substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, and triethanolamine oleate. For solid compositions, conventional nontoxic pharmaceutically acceptable vehicles can be used which include, for example, pharmaceutical grades of mannitol, lactose, starch, magnesium stearate, sodium saccharin, talcum, cellulose, glucose, sucrose, magnesium carbonate, and the like.

In one embodiment the invention comprises the topical and/or local administration of a compound as described herein and/or a composition comprising a compound as described herein to a subject. The term "topical administration" refers to the delivery of a pharmacologically active agent to the skin or mucosa of a patient. Topical administration can provide a local rather than a systemic effect. The terms "topical administration" and "transdermal administration" are used interchangeably to mean administration of a pharmacologically active agent to the skin or mucosa of a patient to achieve a therapeutic effect in treating or preventing a medical disorder of the invention or discomfort at the site of topical or transdermal administration. Preferred administration modes relate to a topical solution, lotion, shake lotion, cream, ointment, gel, foam, transdermal patch, powder, solid form, sponge, tape, paste or tincture. Preferred embodiments relate to creams, foams, gels, lotions, and ointments.

Various additives, known to those skilled in the art, may be included in topical compositions of the present disclosure. For example, solvents, including relatively small amounts of alcohol, may be used to solubilize a compound of the invention. Other optional additives include antioxidants, fragrances, colorant, gelling agents, emulsifiers, thickening agents, stabilizers, surfactants, buffers, cooling agents (e.g., menthol) and the like. Other agents may also be added, such as antimicrobial agents, to prevent spoilage upon storage, i.e., to inhibit growth of microbes such as yeasts and molds. Examples of suitable antimicrobial agents include methyl and propyl esters of p-hydroxybenzoic acid (i.e., methyl and propyl paraben), sodium benzoate, sorbic acid, imidurea, and the like. When applied to skin, a topical composition of the present disclosure can be covered with an occlusive or non-occlusive dressing, which may be porous or non-porous, so as to protect the composition from mechanical removal during the period of treatment, e.g. a plastic film food wrap or other non-absorbent film. Various inert coverings may be employed. Non-woven or woven coverings may be employed, particularly elastomeric coverings, which allow for heat and vapor transport. These coverings can allow for cooling of the diseased site, which can provide for greater comfort, while protecting the composition from mechanical removal.

In accordance with the various treatment methods of the disclosure, the compound can be delivered to a subject in a manner consistent with conventional methodologies associated with management of the disorder for which treatment or prevention is sought. In accordance with the disclosure herein, a prophylactically or therapeutically effective amount of the compound and/or other biologically active agent is administered to a subject in need of such treatment for a time and under conditions sufficient to prevent, inhibit, and/or ameliorate a selected disease or condition or one or more symptom(s) thereof.

"Administration of" and "administering a" compound should be understood to mean providing a compound, a prodrug of a compound, or a pharmaceutical composition as described herein. The compound or composition can be administered by another person to the subject (e.g., intravenously, gel, cream, spray) or it can be self-administered by the subject (e.g., tablets, gel, cream, spray).

Dosage can be varied by the attending clinician to maintain a desired concentration at a target site (for example, the lungs or systemic circulation). Higher or lower concentrations can be selected based on the mode of delivery, for example, trans-epidermal, rectal, oral, pulmonary, or intranasal delivery versus intravenous or subcutaneous delivery. Dosage can also be adjusted based on the release rate of the administered formulation, for example, of an intrapulmonary spray versus powder, sustained release oral versus injected particulate or transdermal delivery formulations, and so forth.

The term "subject" includes both human and veterinary subjects. The term "treatment" refers to a therapeutic intervention that ameliorates a sign or symptom of a disease or pathological condition after it has begun to develop.

As used herein, the term "ameliorating", with reference to a disease or pathological condition, refers to any given beneficial effect of the treatment. The beneficial effect can be evidenced, for example, by a delayed onset of clinical symptoms of the disease in a subject, a reduction in severity of some or all clinical symptoms of the disease, a slower progression of the disease, an improvement in the overall health or well-being of the subject, or by other parameters well known in the art that are specific to the particular disease.

The present invention encompasses both therapeutic treatment and preventative (prophylactic) treatment of a subject. A "preventative" or "prophylactic" treatment is a treatment administered to a subject, who does not exhibit signs of the medical condition or who preferably exhibits indications of developing or developing further any given medical condition, for the purpose of decreasing the risk of developing pathology or clinical symptoms. A prophylactic administration may comprise the administration of the compounds in advance of developing symptoms, thereby avoiding or reducing the subsequent occurrence of a disease. The present invention also relates to a method of treatment of subjects suffering from the various medical conditions disclosed herein. The method of treatment comprises preferably the administration of a therapeutically effective amount of a compound disclosed herein to a subject in need thereof.

A "therapeutically effective amount" refers to a quantity of a specified agent sufficient to achieve a desired effect in a subject being treated with that agent. For example, this may be the amount of a compound disclosed herein useful in alleviating the symptoms of one or more of the medical conditions described herein in a subject. The therapeutically effective amount or diagnostically effective amount of an agent will be dependent on the subject being treated, the severity of illness, and the manner of administration of the therapeutic composition. Dosage regimens can be adjusted to provide an optimum prophylactic or therapeutic response. A therapeutically effective amount is also one in which any toxic or detrimental side effects of the compound and/or other biologically active agent is outweighed in clinical terms by therapeutically beneficial effects. A non-limiting range for a therapeutically effective amount of a compound and/or other biologically active agent within the methods and formulations of the disclosure is about 0.001 mg/kg body weight to 50 mg/kg body weight, 0.01 mg/kg body weight to about 20 mg/kg body weight, such as about 0.05 mg kg to about 5 mg/kg body weight, or about 0.2 mg/kg to about 2 mg/kg body weight.

The instant disclosure also includes kits, packages and multi-container units containing the herein described pharmaceutical compositions, active ingredients, and/or means for administering the same for use in the prevention and treatment of diseases and other conditions in mammalian subjects.

### FIGURES

The invention is further described by the following figures. These are not intended to limit the scope of the invention but represent preferred embodiments of aspects of the invention provided for greater illustration.

### Brief description of the figures:

**Figure 1****: Solution structure of Ramoplanin A2.**
**Figure 2****:** Ramoplanin A2 inhibits seeded Tau fibrillization in vitro.
**Figure 3****:** Ramoplanin A2 interferes with Tau fibril elongation and does not dissociate Tau fibrils.
**Figure 4****:** Interaction of Ramoplanin A2 with monomeric Tau.
**Figure 5****:** NMR resonance assignment of Ramoplanin A2.
**Figure 6****:** Quantification of Ramoplanin A2 chemical shift perturbations (CSPs) for the determination of epitopes for binding to monomeric Tau.
**Figure 7****:** Ramoplanin A2 side chain and amide backbone epitopes for binding to monomeric Tau. NMR chemical shift perturbations (CSPs, from Figure 6) of Ramoplanin A2 side chain and amide.
**Figure 8****:** The peptide backbone of Ramoplanin A2 is involved in binding to monomeric Tau.
**Figure 9****:** Ramoplanin A2 epitopes for binding to Tau fibrils from saturation transfer difference (STD) NMR spectroscopy.
**Figure 10****:** Ramoplanin A2 epitopes for binding to Tau fibrils mapped to the structure of the peptide.
**Figure 11****:** Inhibition of seeded Tau aggregation by cyclic lipopeptide antibiotic compounds in HEK biosensor cells.
**Figure 12****:** Ramoplanin A2 concentration needed to reach 50% inhibitory activity against seeded Tau aggregation in HEK biosensor cells.
**Figure 13****:** Ramoplanin A2 inhibits the aggregation of Tau in HEK biosensor cells when Tau aggregation is induced by exposure of HEK cells to Tau^{P301L} fibrils.
**Figure 14****:** Ramoplanin A2 inhibits the aggregation of Tau in HEK biosensor cells when Tau aggregation is induced by exposure of HEK cells to brain homogenate of PS19 mice.
**Figure 15****:** Ramoplanin A2 rescues Tau-induced degeneration of Drosophila eyes.
**Figure 16****:** Ramoplanin A2 inhibits the seeded aggregation of the NM region of the Sup35 prion protein (named "NM") in cells.
**Figure 17****:** Ramoplanin A2 prevents prion infection in cellular model.
**Figure 18****:** Ramoplanin A2 and the Ramoplanin A2 Aglycon derivative inhibit the in vitro aggregation of the Parkinson's disease-associated protein alpha-synuclein (Asyn).
**Figure 19****:** Enduracidin A and B inhibit the in vitro aggregation of the Parkinson's disease-associated protein alpha-synuclein (Asyn).
**Figure 20****:** Teicoplanin inhibits the in vitro aggregation of the Parkinson's disease-associated protein alpha-synuclein (Asyn).
**Figure 21****:** Ramoplanin A2 inhibits the in vitro aggregation of medin protein.
**Figure 22****:** Ramoplanin A2 inhibits the in vitro aggregation of TGFBI peptide.
**Figure 23****:** Ramoplanin A2, NAI-603, and Enduracidin B inhibit the in vitro aggregation of human IAPP or amylin.
**Figure 24****:** Proposed mechanism of action of cyclic lipopeptide antibiotic compounds in pathogenic protein aggregation inhibition.
**Figure 25****:** Synthesis scheme of compounds 5 and 18 to 29.
**Figure 26****.** Structural features of Ramoplanins and Ramoplanose.
**Figure 27****.** Structural forms of Enduracidin.
**Figure 28****.** Chemical structure of Vancomycin and lipopeptide derivatives of Vancomycin.
**Figure 29****.** Structural forms of Teicoplanin.
**Figure 30****.** Structural forms of Ristocetin and lipopeptide derivatives of Ristocetin.

### Detailed description of the figures

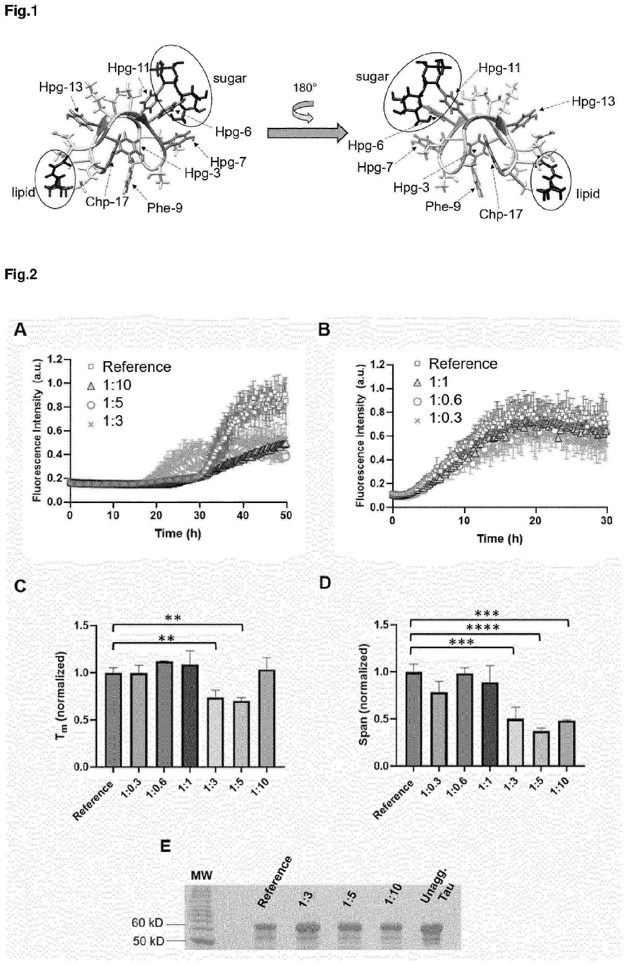

**Figure 1****:** Solution structure of Ramoplanin A2. A Stereoview of the Ramoplanin A2 solution structure (PDB entry 1DSR) shows two antiparallel β-strands (HAsn2-D-Hpg7 and D-Orn10-Gly14) stabilized by six transannular hydrogen bonds (H-bonds) and a cluster of hydrophobic aromatic side chains (D-Hpg3, Phe9, and Chp17) providing a U-shape topology to the β-sheet with a reverse β-turn (aThr8-Phe9) at one end and a more flexible connecting loop (Leu15-Chp17) at the other end in the solution structure. Lipid and sugar atoms are colored black and encircled. Aromatic residues are colored grey and non-aromatic residues are colored white. Aromatic rings of residues 3, 17, and 9 form a hydrophobic core stabilized by pi-stacking interactions.

**Figure 2****:** Ramoplanin A2 inhibits seeded Tau fibrillization in vitro. (A,B) Ramoplanin A2 was incubated with 25 µM 2N4R Tau monomers, 1% (v/v) 2N4R Tau fibril seeds, and 50 µM Thioflavin T (ThT) in 25 mM HEPES at pH 7.2 with 5 mM MgCl₂, 10 mM KCI while mixing at 37 °C. During incubation at 37 °C, ThT fluorescence was monitored. and an increase in ThT fluorescence indicates fibrillization. Two batches of experiments were performed spanning Tau: Ramoplanin A2 mole ratios of 1:3-1:10 (A) and 1:0.3-1:1 (B) with two replicates per condition. For comparison, Tau fibrillization in the absence of Ramoplanin A2 (named "Reference") was also followed. (C) Influence of Ramoplanin A2 on the kinetics of Tau fibrillization. (D) The span of the fluorescence curves for all mole ratios are compared to the span of Tau reference curves. For panels C and D, significant differences between group means were determined by one-way ANOVA analysis with Dunnett's multiple comparison test (Reference Tm or span values serve as the control groups). Significant differences corresponding to p≤0.002(**), p≤0.0002 (***), and p≤0.0001 (****) are indicated. (E) SDS-PAGE gel of aggregation assays.

**Figure 3****:** Ramoplanin A2 interferes with Tau fibril elongation and does not dissociate Tau fibrils. (A) Tau aggregation was carried out by incubating 25 µM 2N4R Tau monomers with 1% (v/v) 2N4R Tau fibril seeds and 50 µM ThT at 37°C for -25 h, after which Ramoplanin A2 (Tau:Ramoplanin mole ratios are 1:0.3 and 1:1) or buffer (Reference) were added. Two replicates were performed per condition. (B) The span of the fluorescence curves from (A) was quantified before and after addition of Ramoplanin A2. The change in span induced by Ramoplanin A2 was determined by one-way ANOVA analysis with Dunnett's method of multiple comparisons (Reference before addition of Ramoplanin A2 was considered as the control group). (C) Transmission electron micrographs of Tau fibrils formed with and without Ramoplanin A2 (Scale bars are 500 nm).

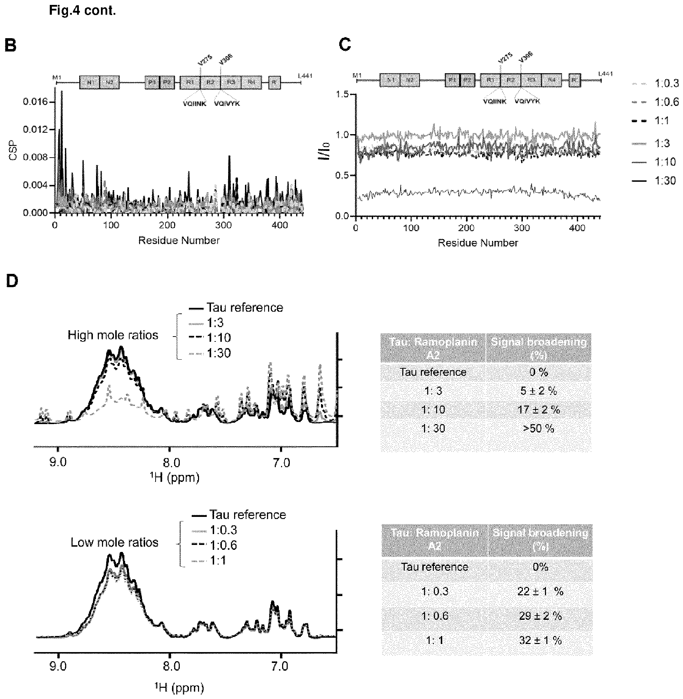

**Figure 4****:** Interaction of Ramoplanin A2 with monomeric Tau. ¹⁵N-labeled 2N4R Tau (18 µM in 50 mM sodium phosphate pH 6.8 with 0.01% w/v sodium azide at 278 K) was titrated with increasing amounts of Ramoplanin A2 (mole ratios 1:0.3 to 1:30) while the 2D and 1D NMR spectra of Tau were monitored. (A) Superposition of 2D ¹H-¹⁵N correlation NMR spectra of Tau in the absence (black) and in presence of a 10-fold excess of Ramoplanin A2 (grey). Changes in peak positions (termed chemical shift perturbations, CSPs) induced by Ramoplanin A2 are highlighted in the zoomed-in region (right). (B,C) Residue-specific chemical shift perturbations (CSP; B) and signal changes (I/I₀, C) induced by addition of Ramoplanin A2. (D) Quantification of amide proton signal broadening from 1 D ¹H NMR spectra of Tau titrated with Ramoplanin A2. Error estimates are based on signal-to-noise in the spectra.

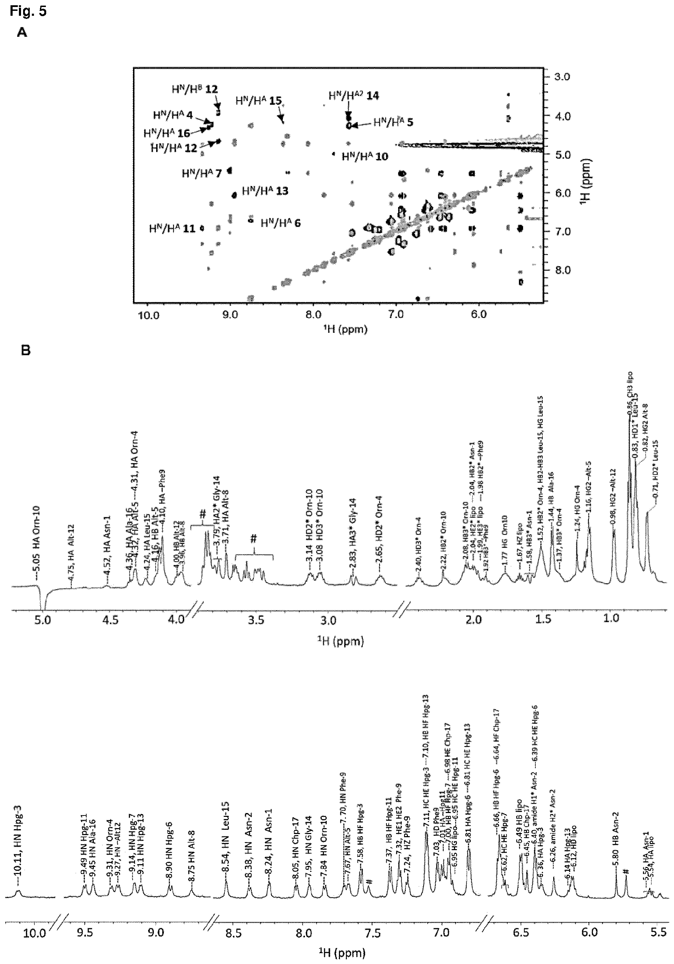

**Figure 5****:** NMR resonance assignment of Ramoplanin A2. (A) Assigned HN/HA and HN/HB cross-peaks in 2D TOCSY (black) and 2D NOESY (grey) NMR spectra of Ramoplanin A2 in 50 mM sodium phosphate, 0.01% (w/v) sodium azide at pH 6.8, 25 °C. The assignments were determined by a sequential walk following the NOE connectivities between HN and HA-1 protons. (B) 1D ¹H NMR spectrum of Ramoplanin A2 labeled with the resonance assignment of side chain and backbone protons. The spectrum was recorded at 5 °C in 50 mM sodium phosphate, 0.01% (w/v) sodium azide at pH 6.8. Ambiguous assignments corresponding to geminal atoms or geminal methyl groups are marked with an asterisk (*) and unassigned peaks corresponding to sugar protons are marked with a pound sign (#). The backbone and side chain assignments were used to facilitate structure-activity-relationship (SAR) studies of Ramoplanin A2.

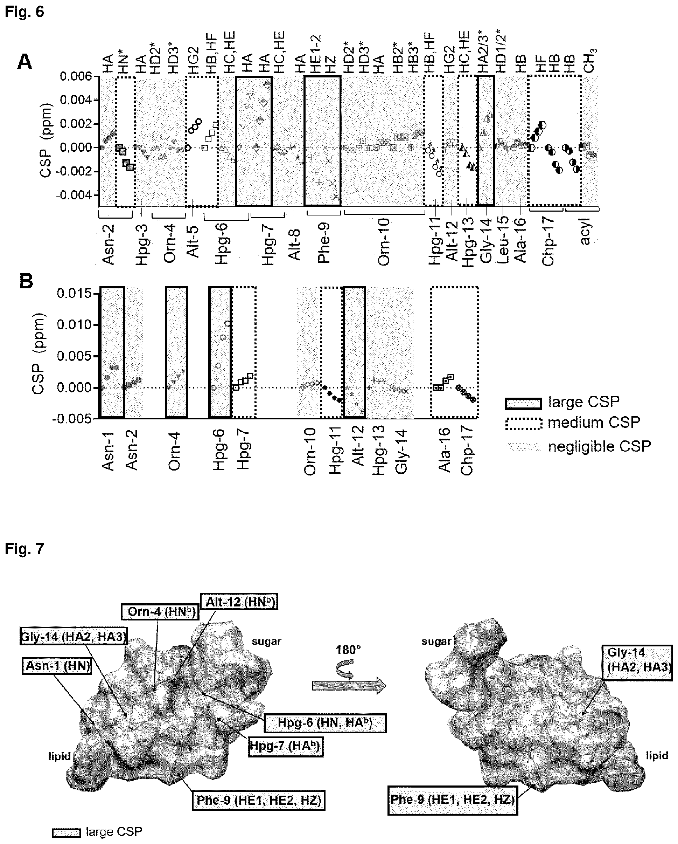

**Figure 6****:** Quantification of Ramoplanin A2 chemical shift perturbations (CSPs) for the determination of epitopes for binding to monomeric Tau. NMR chemical shift perturbations of Ramoplanin A2 protons (side chain/A, amide backbone/B) observed in the presence of 2N4R Tau. 200 µM Ramoplanin A2 was titrated with 10, 20, and 30 µM Tau at 37 °C in 50 mM sodium phosphate (pH 6.8) and CSPs of assigned peaks in the peptide at the mole ratio 200:30 (Ramoplanin A2:Tau) were ranked according the cut-offs for "large" CSPs (0.0025 ppm) and "medium" CSPs (0.0015 ppm). Negligible CSPs are <0.0015 ppm. CSPs of some protons are not determined due to peak overlap, peak broadening, or lack of assignment.

**Figure 7****:** Ramoplanin A2 side chain and amide backbone epitopes for binding to monomeric Tau. NMR chemical shift perturbations (CSPs, from Figure 6) of Ramoplanin A2 side chain and amide. NMR chemical shift perturbations (CSPs, from Figure 6) of Ramoplanin A2 side chain and amide backbone protons observed in the presence of 2N4R Tau are mapped to the molecular surface of Ramoplanin A2 (PDB entry 1DSR). 200 µM Ramoplanin A2 was titrated with 10, 20, and 30 µM Tau at 37 °C in 50 mM sodium phosphate (pH 6.8) and CSPs of assigned peaks in the peptide at the mole ratio 200:30 (Ramoplanin A2:Tau) were determined. Protons corresponding to "large" CSPs (at least 0.0025 ppm) are labeled. Protons with large CSPs represent binding epitopes of Ramoplanin A2 for monomeric Tau. The superscript "b" corresponds to protons that are buried in the structure, i.e., not solvent-excluded. Solvent-excluded molecular surface construction were performed using Chimera version 1.14.

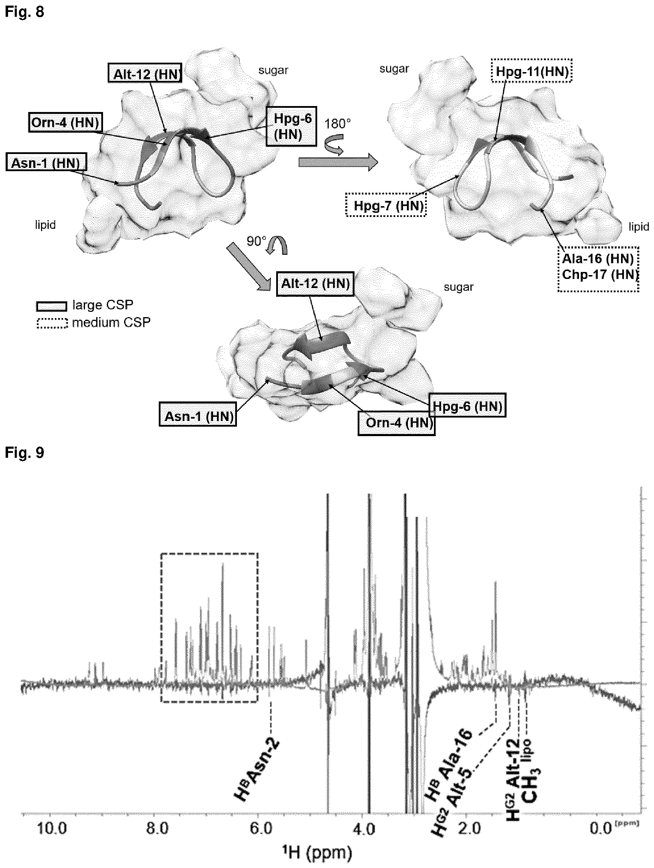

**Figure 8****:** The peptide backbone of Ramoplanin A2 is involved in binding to monomeric Tau. Large (≥ 0.0025 ppm) and medium (0.0025 > CSP ≥ 0.0015 ppm) CSPs are mapped to the ribbon representation of the peptide backbone (PDB entry 1DSR). Secondary structure determination and solvent-excluded molecular surface construction were performed using Chimera version 1.14.

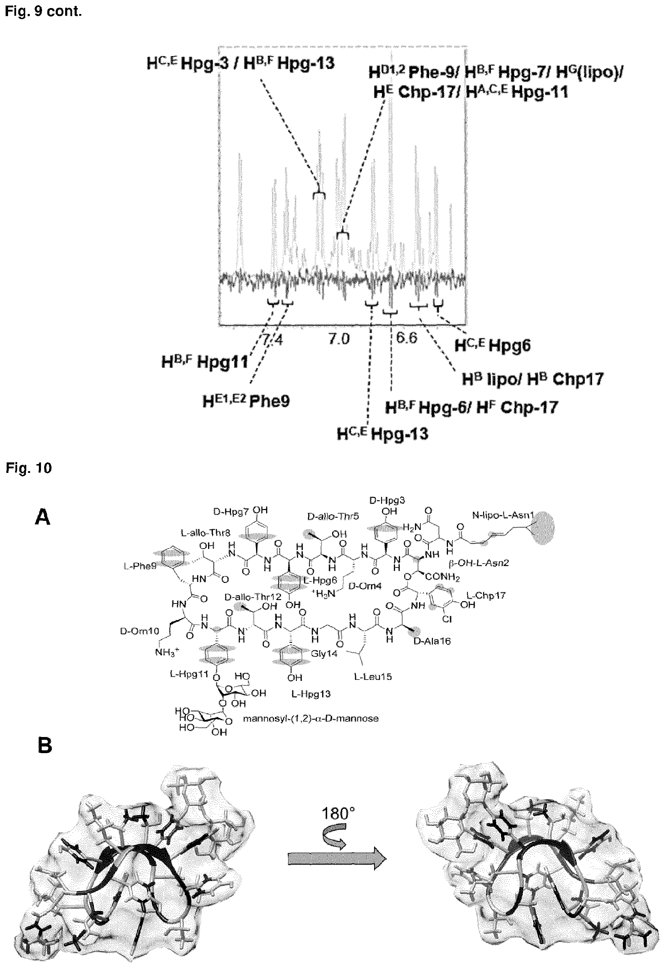

**Figure 9****:** Ramoplanin A2 epitopes for binding to Tau fibrils from saturation transfer difference (STD) NMR spectroscopy. The 1D STD NMR spectrum of Ramoplanin A2 (negatively phased peaks) was recorded in the presence of Tau fibrils (48-fold excess of Ramoplanin A2 over 2N4R Tau fibrils). The STD experiment was performed in an interleaved manner, alternating between off-resonance irradiation at 60 ppm and on-resonance irradiation at -2 ppm. The regular 1D ¹H spectrum of Ramoplanin A2 is shown with positively phased peaks. The inset on the bottom panel corresponds to the boxed region in the spectrum (-6.0 ppm - 8.0 ppm). The spectra were recorded at 37°C in 25 mM HEPES, pH 7.2, 100 mM NaCl. In the STD analyses, sugar protons were excluded owing to peak overlap with HEPES protons (3.6 ppm - 4.0 ppm).

**Figure 10****:** Ramoplanin A2 epitopes for binding to Tau fibrils mapped to the structure of the peptide. Protons showing STD effects are encircled in grey in the chemical structure (Panel A) of Ramoplanin A2. The C-H pairs showing STD effects are marked black in the molecular model of Ramoplanin A2 (Panel B, PDB entry 1DSR), which has the same orientation as in Figure 1. They represent Tau fibril binding epitopes of Ramoplanin A2.

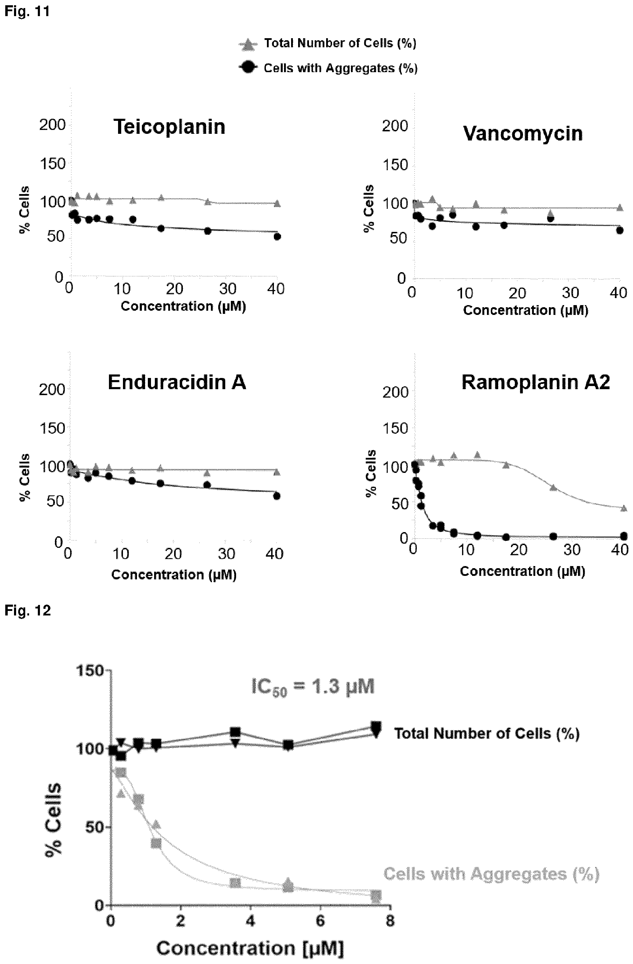

**Figure 11****:** Inhibition of seeded Tau aggregation by cyclic lipopeptide antibiotic compounds in HEK biosensor cells. Tau aggregation in cells was induced by incubation of HEK biosensor cells with cell lysate, which was obtained from a previous HEK batch containing Tau aggregates. Cells were incubated with the compounds (0.25 µM to 40 µM) for 22h in the presence of lipofectamine.

**Figure 12****:** Ramoplanin A2 concentration needed to reach 50% inhibitory activity against seeded Tau aggregation in HEK biosensor cells. Tau aggregation in HEK biosensor cells was induced by incubation with lysate from HEK cells containing Tau aggregates. Cells were incubated with Ramoplanin A2 (0.25 µM to 7.5 µM) for 22 h in the presence of lipofectamine.

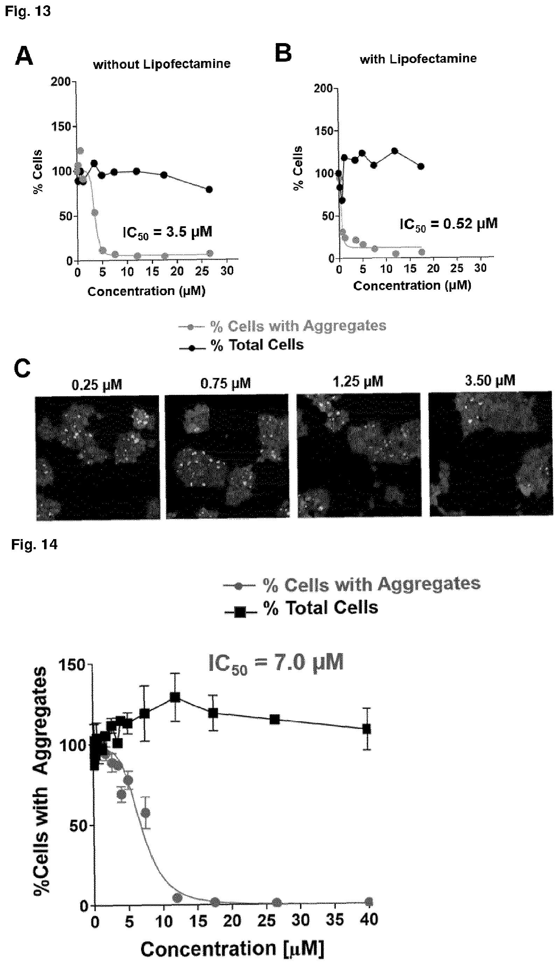

**Figure 13****:** Ramoplanin A2 inhibits the aggregation of Tau in HEK biosensor cells when Tau aggregation is induced by exposure of HEK cells to Tau^{P301L} fibrils. (A-B) Cells were incubated with the drug (0.25 µM to 40 µM) for 48 hours in the presence or absence of lipofectamine. (C) The percentages of total cells and cells with aggregates (appearing as white spots) were quantified by microscopy.

**Figure 14****:** Ramoplanin A2 inhibits the aggregation of Tau in HEK biosensor cells when Tau aggregation is induced by exposure of HEK cells to brain homogenate of PS19 mice. Cells were incubated with the drug (0.25 µM to 26.5 µM) for 24 hours in the presence of lipofectamine. Error bars correspond to the standard deviation of the % cells (total cells or cells with aggregates) from two replicates.

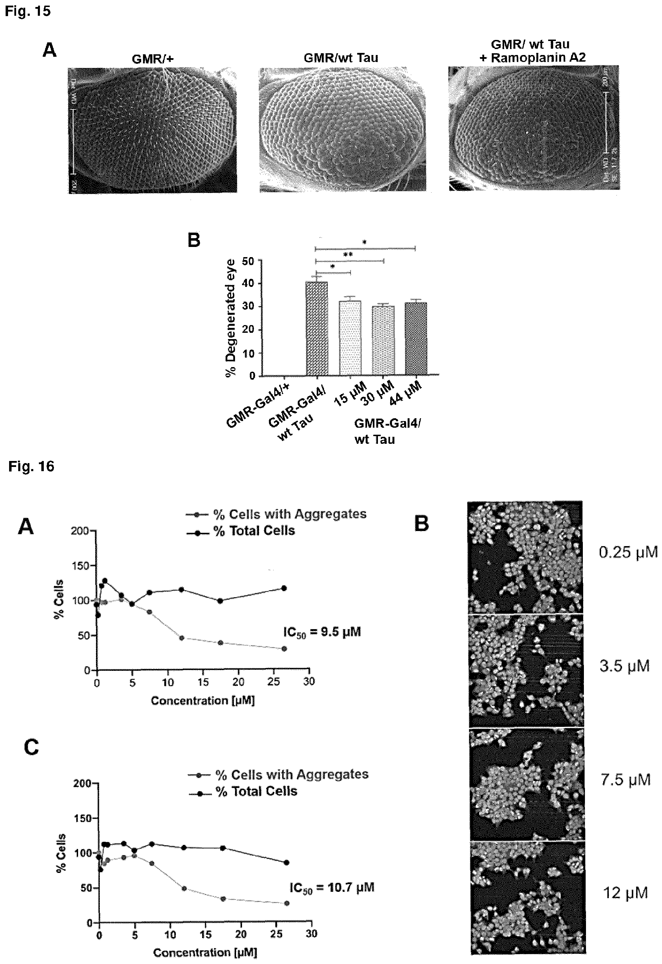

**Figure 15****:** Ramoplanin A2 rescues Tau-induced degeneration of Drosophila eyes. Flies with (GMR-Gal4/WT Tau) and without (GMR-Gal4/+) Tau expression were treated with Ramoplanin A2 from the larvae stage up to the adult stage, with 5 replicates. (A, B) Percent degeneration was determined by SEM imaging of Drosophila eyes. Statistically different groups were determined by unpaired t-test with p≤ 0.05(*), p ≤0.01 (**), p≤0.001 (***), p≤0.0001 (****).

**Figure 16****:** Ramoplanin A2 inhibits the seeded aggregation of the NM region of the Sup35 prion protein (named "NM") in cells. HEK (A, B) or N2a cells (C) expressing soluble NM-GFP or HA-epitope tagged NM, respectively, were incubated with increasing concentrations of Ramoplanin for 1 h. 500 nM of sonicated, recombinant NM fibrils was added and cells were incubated for a total of 24 h. Cell nuclei were detected using Hoechst, NM-HA was stained using anti-HA antibodies. Cells with aggregates (aggregates appear as white spots) were analyzed by automated microscopy and image analysis using CellVoyager 6000 and image analysis. (A,C) Statistical analysis of aggregate induction assay. (B) CellVoyager images of HEK NM-GFP cells exposed to Ramoplanin A2 and recombinant NM fibrils.

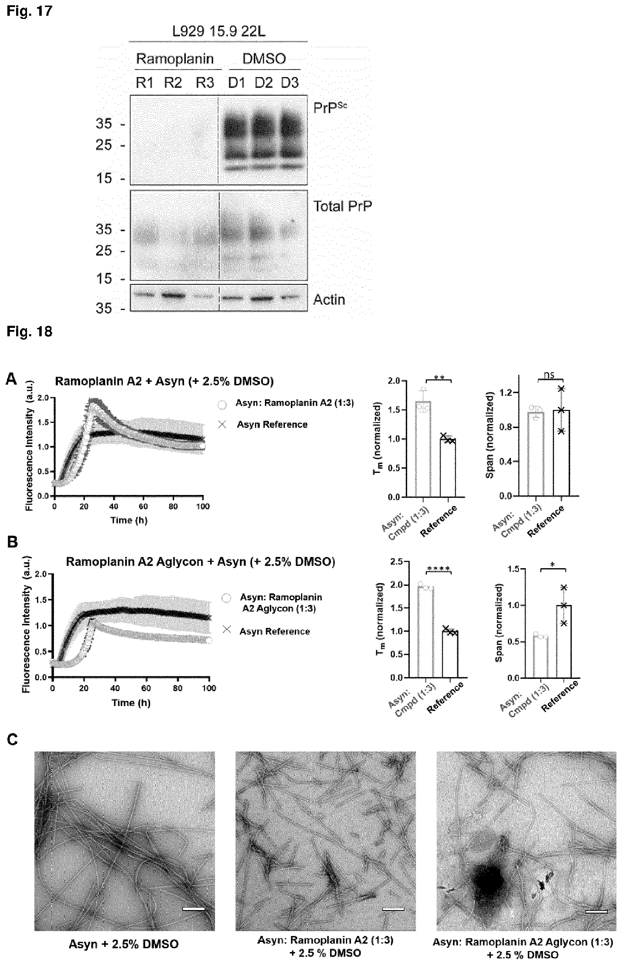

**Figure 17****:** Ramoplanin A2 prevents prion infection in cellular model. Prion-susceptible fibroblast cell clone L929 15.9 was pre-incubated with 3 µM Ramoplanin A2 or solvent control for 1 h and subsequently exposed to 1 % 22L scrapie brain homogenate. Cells were grown for 9 d in the presence of Ramoplanin A2 before cell lysates were analyzed for pathogenic prion protein PrP^{Sc}. Shown are Western blots of total cell lysates (total PrP and actin) and proteinase K resistant PrP^{Sc}. PrP was detected using anti-PrP antibody 4H11. Additional lanes were excised for presentation purposes (dashed lines). Shown are triplicates.

**Figure 18****:** Ramoplanin A2 and the Ramoplanin A2 Aglycon derivative inhibit the in vitro aggregation of the Parkinson's disease-associated protein alpha-synuclein (Asyn). (A, B) Thioflavin-T (ThT) fluorescence curves of N-terminally acetylated alpha-synuclein (Asyn) in the absence (cross) and presence of a 3-fold excess (white circles) of compound. The aggregation assay was performed by incubating 25 µM Asyn monomers with 1% (v/v) Asyn fibril seeds, 50 µM ThT, and aggregation buffer (50 mM HEPES, 100 mM NaCl, pH 7.4) containing 2.5 % v/v dimethyl sulfoxide (DMSO), since the compound stock solutions were prepared in DMSO. Incubation was performed at 37°C with double orbital shaking and in the presence of two microbeads per microwell (100-uL mixture). Quantitative analysis of Asyn aggregation kinetics was performed by nonlinear regression with sigmoidal fitting to determine the midpoint value (Tm) of the fluorescence curve as well as the fluorescence span. Unpaired two-tailed t-test was performed to test the null hypothesis (p≤0.0332(*), p≤0.002(**), p≤0.0002 (***), p≤0.0001 (****)). Significantly larger Tm and lower span indicate inhibition of Asyn aggregation. (C) TEM imaging analysis of the compound/Asyn mixtures. Scale bars correspond to 200 nm.

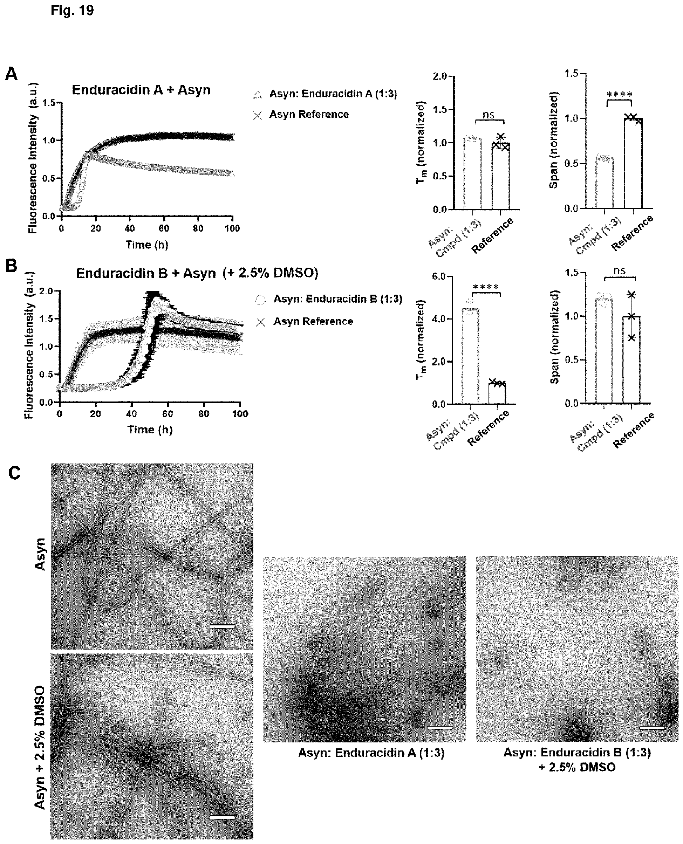

**Figure 19****:** Enduracidin A and B inhibit the in vitro aggregation of the Parkinson's disease-associated protein alpha-synuclein (Asyn). (A, B) ThT fluorescence curves of N-terminally acetylated alpha-synuclein (Asyn) in the absence (cross) and presence of a 3-fold excess (white circles or triangles) of compound. The aggregation assay was performed by incubating 25 µM Asyn monomers with 1% (v/v) Asyn fibril seeds, 50 µM ThT and plain aggregation buffer (50 mM HEPES, 100 mM NaCl, pH 7.4) or aggregation buffer containing 2.5 % v/v dimethyl sulfoxide (DMSO). Incubation was performed at 37°C with double orbital shaking and in the presence of two microbeads per microwell (100-uL mixture). Quantitative analysis of Asyn aggregation kinetics was performed by nonlinear regression with sigmoidal fitting to determine the midpoint value (Tm) of the fluorescence curve as well as the fluorescence span. Unpaired two-tailed t-test was performed to test the null hypothesis (p≤0.0332(*), p≤0.002(**), p≤0.0002 (***), p≤0.0001 (****)). Significantly larger Tm and lower span indicate inhibition of Asyn aggregation. (C) TEM imaging analysis of the compound/Asyn mixtures. Scale bars correspond to 200 nm.

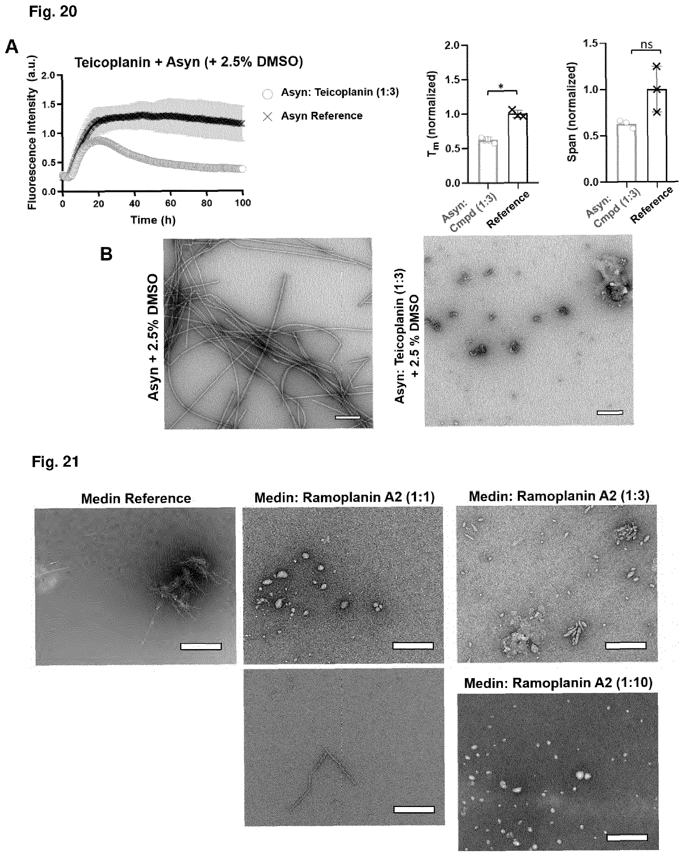

**Figure 20****:** Teicoplanin inhibits the in vitro aggregation of the Parkinson's disease-associated protein alpha-synuclein (Asyn). (A) ThT fluorescence curves of N-terminally acetylated alpha-synuclein (Asyn) in the absence (cross) and presence of a 3-fold excess (white circles) of compound. The aggregation assay was performed by incubating 25 µM Asyn monomers with 1% (v/v) Asyn fibril seeds, 50 µM ThT and aggregation buffer (50 mM HEPES, 100 mM NaCl, pH 7.4) containing 2.5 % v/v dimethyl sulfoxide (DMSO). Incubation was performed at 37°C with double orbital shaking and in the presence of two microbeads per microwell (100-uL mixture). Quantitative analysis of Asyn aggregation kinetics was performed by nonlinear regression with sigmoidal fitting to determine the midpoint value (Tm) of the fluorescence curve as well as the fluorescence span. Unpaired two-tailed t-test was performed to test the null hypothesis (p≤0.0332(*), p≤0.002(**), p≤0.0002 (***), p≤0.0001 (****). Significantly larger Tm and lower span indicate inhibition of Asyn aggregation. (B) TEM imaging analysis of the compound/Asyn mixture. Scale bars correspond to 200 nm.

**Figure 21****:** Ramoplanin A2 inhibits the in vitro aggregation of medin protein. The aggregation assay was performed by incubating 20 µM medin monomers with 20 µM ThT and aggregation buffer (20 mM sodium phosphate, 150 mM NaCl, pH 7.4) containing 2.5 % v/v dimethyl sulfoxide (DMSO). Incubation was performed at 30°C with double orbital shaking and without microbeads. B. TEM imaging analysis of the compound/medin mixture. Scale bars correspond to 200 nm.

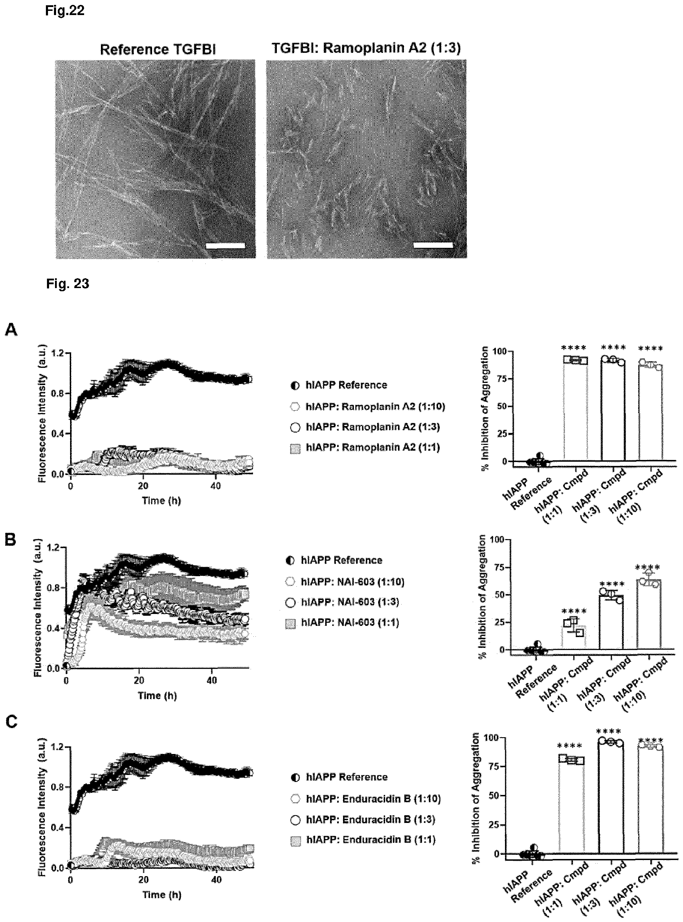

**Figure 22****:** Ramoplanin A2 inhibits the in vitro aggregation of TGFBI peptide. The aggregation assay was performed by incubating 85 µM TGFBI monomers with 30 µM ThT and aggregation buffer (phosphate buffered saline: 137 mM NaCl, 2.7 mM KCI, 10 mM Na₂HPO₄, 1.8 mM KH₂PO₄, pH 7.4) containing 2.5 % v/v dimethyl sulfoxide (DMSO). Incubation was performed at 37°C with double orbital shaking and in the presence of two microbeads per microwell (100-uL mixture). TEM imaging analysis of the compound/TGFBI mixture. Scale bars correspond to 200 nm.

**Figure 23****:** Ramoplanin A2, NAI-603, and Enduracidin B inhibit the in vitro aggregation of amylin. The aggregation assay was performed by incubating 5 µM amylin (human islet amyloid polypeptide or hIAPP) monomers with 10 µM ThT and aggregation buffer (10 mM Tris-HCI with 100 mM NaCl, pH 7.4) containing 2.5 % v/v dimethyl sulfoxide (DMSO). The hIAPP monomers were incubated with various doses of cyclic lipopeptide antibiotic compounds (A - Ramoplanin A2, B - NAI-603 or the 2-methylphenyl derivative of Ramoplanin, C - Enduracidin B). Incubation was performed at 37°C with double orbital shaking and no microbeads. The percent of amylin aggregation inhibition was determined from the final ThT fluorescence values relative to the reference at the end of the assay (t = 50 h). One-way ANOVA with Dunnett's multiple comparisons was performed to test the null hypothesis (p≤0.0332(*), p≤0.002(**), p≤0.0002 (***), p≤0.0001 (****) while comparing the % inhibition in the presence of the compounds with the % inhibition in the reference condition.

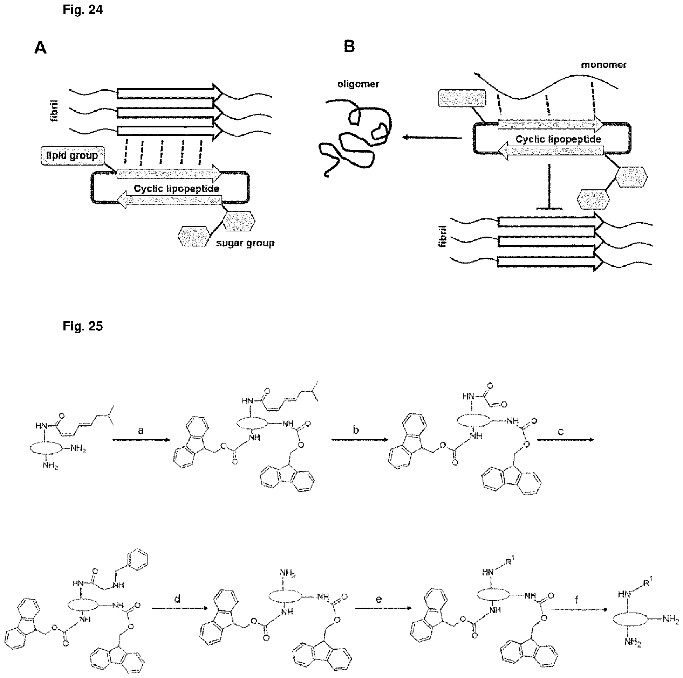

**Figure 24****:** Proposed mechanism of action of cyclic lipopeptide antibiotic compounds in pathogenic protein aggregation inhibition. (A) To block fibril elongation, the compounds (in grey) engage in hydrophobic and hydrogen bonding interactions (dashed lines) with the exposed surface of a growing amyloid fibrils (in black outlines), thereby inhibiting recruitment of monomer proteins to the fibril. (B) The compounds also block fibril formation by binding to the monomeric state of the protein thereby trapping the protein in an oligomeric state (combination of cyclic lipopeptide and protein) and disfavoring the formation of amyloid fibrils.

**Figure 25****:** Synthesis scheme of compounds 5 and 18 to 29. Displayed is the general synthesis scheme for compounds No. 5 and 18 to 29. The peptide core and mannosyl-(1,2)-α-D-mannose moieties are represented as an oval, with only the ornithine amino moieties and R1 attached to the first residue illustrated. Steps a to f are explained in detail within the example section.

**Figure 26****.** Structural features of Ramoplanins and Ramoplanose. Ramoplanins A1, A2, A3 are distinguished from each other by the lipid moiety attached to the first residue, L-asparagine 1 (L-Asn1). Ramoplanins A1-A3 and Ramoplanose have a common peptide core of 17 amino acids. Ramoplanins A1-A3 have a disaccharide moiety, mannosyl-1,2-α-D-mannose, attached to L-hydroxyphenylglycine 11 (L-Hpg11). Ramoplanose differs from Ramoplanin A2 by the sugar substituent on L-Hpg11: Ramoplanose has 3 instead of 2 mannose units. Specific stereochemical representations of the structures are non-limiting.

**Figure 27****.** Structural forms of Enduracidin. Enduracidin is a mixture of components A and B which differ in the lipid substituent of L-Asp1. Enduracidin has 17 amino acids, and 13 of which are non-proteinogenic amino acids. Unlike Ramoplanin, Enduracidin does not contain sugar moieties. Specific stereochemical representations of the structures are non-limiting.

**Figure 28****.** Chemical structure of Vancomycin and lipopeptide derivatives of Vancomycin. Vancomycin is glycosylated on ring D and does not contain a lipid group (vancosamine in the form -NH₂). Common to Teicoplanins, Ristocetins, and the parent structure of Vancomycin are rings A-E. Lipopeptide derivatives of vancomycin may be produced by attachment of a lipid or lipid-like substituent to the amino nitrogen of vancosamine (-NH-lipid). Specific stereochemical representations of the structures are non-limiting.

**Figure 29****.** Structural forms of Teicoplanin. Teicoplanins exist in 5 forms A2-1 to A2-5 which differ in the lipid substituent of N-acyl glucosamine. There are 3 sugar moieties in teicoplanins attached to separate sites within the compound: N-acylglucosamine linked to the phenolic oxygen of ring D, N-acetylglucosamine preceding ring C, and D-mannose linked to the phenolic oxygen of ring A. Specific stereochemical representations of the structures are non-limiting.

**Figure 30****.** Structural forms of Ristocetin and derivatives. Ristocetins A and B only differ in the sugar substituent bonded to the phenolic oxygen of ring D. In Ristocetin A, the substituent is a tetrasaccharide comprising arabinose, mannose, glucose, and rhamnose whereas in B the disaccharide substituent comprises glucose and rhamnose. The preferred potential site of derivatization to convert Ristocetins to lipopeptides is the amino group of ristosamine. Specific stereochemical representations of the structures are non-limiting.

### EXAMPLES

### Example 1: Inhibition of seeded Tau fibrillization in vitro by Ramoplanin A2 (compound No. 1)

As shown in Figure 2, Ramoplanin A2 inhibits seeded Tau fibrillization in vitro. Ramoplanin A2 was incubated with 25 µM 2N4R Tau monomers, 1% (v/v) 2N4R Tau fibril seeds at 37 °C and ThT fluorescence was monitored. The fluorescence curves in panels A and B show the increase in Thioflavin T fluorescence over time, which is a sign of Tau fibrillization. Further, the influence of Ramoplanin A2 on the kinetics of Tau fibrillization was investigated. Thereby, the midpoint values (Tm) of the fluorescence curves in Figure 2C show that at 1:3 and 1:5 mole ratios, Ramoplanin A2 accelerates Tau fibrillization (lower Tm corresponds to faster aggregation). Mole ratios of 1:3, 1:5, and 1:10 show significantly lower span values indicating that the total amount of Tau fibrils at chemical equilibrium was decreased in the presence of excess concentrations of Ramoplanin A2 (Figure 2D). Additionally, the SDS-PAGE gel shows that the amount of soluble Tau was increased in the aggregation assays (from Figure 2A) when Tau was incubated with Ramoplanin A2 at mole ratios 1:3 and 1:5 (Figure 2E). The data indicate the overall amount of aggregated Tau is decreased by the cyclic lipopeptide Ramoplanin A2.

### Example 2: Interference of Ramoplanin A2 with Tau fibril elongation

Ramoplanin A2 interferes with Tau fibril elongation and does not dissociate Tau fibrils (Figure 3). Tau aggregation was carried out according to the methods section, after which Ramoplanin A2 (Tau:Ramoplanin mole ratios are 1:0.3 and 1:1) or buffer (Reference) were added. As displayed in Figure 3 A and B, the change in span of the fluorescence curves induced by Ramoplanin A2 was not statistically significant. This shows that Tau fibrils were not dissolved by Ramoplanin A2. Tau fibrils were further investigated by transmission electron microscopy, revealing that Tau fibrils formed in the absence of Ramoplanin A2 (Reference) feature morphologies similar to that of pre-formed Tau fibrils incubated further (for 15 h) with Ramoplanin A2 (Figure 3 C in grey boxes). On the other hand, when Tau fibrils were formed starting from Tau monomers incubated with Ramoplanin A2, fibrils appeared much shorter (<100 nm) and thicker. This suggests that Ramoplanin A2 disrupts Tau fibril elongation, but does not modify already formed Tau fibrils.

### Example 3: Interaction of Ramoplanin A2 with monomeric Tau

Figure 4 shows interaction of Ramoplanin A2 with monomeric Tau. Therefore, superposition of 2D ¹H-¹⁵N correlation NMR spectra of Tau in the absence (black) and in presence of a 10-fold excess of Ramoplanin A2 (grey) were measured. The low chemical shift dispersion along the ¹H dimension indicates that Tau remains disordered in the presence of Ramoplanin A2 (Figure 4 A). Residue-specific chemical shift perturbations (CSP; Figure 4 B) and signal changes (I/I₀, Figure 4 C) induced by addition of Ramoplanin A2. CSPs above ~0.0025 ppm together with signal broadening (I/I₀<1 in (C)) suggest binding of Ramoplanin A2 to specific regions/residues in the monomeric form of Tau. Notably, CSPs (B), I/I₀ ratios (Figure 4 C), and signal broadening of amide protons (Figure 4 D) do not correlate with the dose of Ramoplanin A2, suggesting that Ramoplanin A2 may form large assemblies (e.g., oligomers or micelles) together with Tau in solution.

### Example 4: Binding of Ramoplanin A2 to monomeric Tau

NMR chemical shift perturbations (CSPs, from Figure 6) of Ramoplanin A2 side chain and amide backbone protons observed in the presence of 2N4R Tau were mapped to the molecular surface of Ramoplanin A2 (PDB entry 1DSR) (Figure 7). Protons with large CSPs represent binding epitopes of Ramoplanin A2 for monomeric Tau. On the lipid side, the large CSPs mapped to the solvent-excluded molecular surface are HN Asn-1, HE1-2 and HZ Phe-9, and HN Hpg-6. Rotation by 180 degrees shows the sugar side which has large CSPs for HA2-3 Gly-14 and HE1-2 HZ Phe-9. Figure 8 shows that the peptide backbone of Ramoplanin A2 is involved in binding to monomeric Tau. Large (≥ 0.0025 ppm) and medium (0.0025 > CSP ≥ 0.0015 ppm) CSPs are mapped to the ribbon representation of the peptide backbone (PDB entry 1DSR). Amide protons HN Alt-12, HN Orn-4, HN Hpg-6 and HN Asn-1 showed large CSPs (in boxes with solid lines). Amide protons HN Hpg-7, HN Hpg-11, HN Ala-16 and HN Chp-17 showed medium CSPs (in boxes with dashed lines). HN Alt-12, HN Orn-4, and HN Hpg-6 appear on the antiparallel betastrands formed by residues 3-6 and 11-13. HN Alt-12 and HN Orn-4 showing large CSPs despite being buried in the structure suggests movement of the beta-strand backbone upon binding to monomeric Tau.

### Example 5: Inhibition of seeded Tau aggregation by cyclic lipopeptide antibiotic compounds in HEK biosensor cells.

Tau aggregation in HEK biosensor cell was induced using a various modalities: induction by incubation with cell lysate containing Tau aggregates, induction by incubation with Tau^{P301L} fibrils, and induction by exposure to brain homogenate of PS19 mice (mouse model for tauopathy).

For the first modality, Tau aggregation in cells was induced by incubation of HEK biosensor cells with the lysate of a previous generation of HEK cells containing Tau aggregates. Cells were incubated with the compounds (0.25 µM to 40 µM) for 22 h in the presence of lipofectamine (Figure 11). Teicoplanin, Vancomycin, Enduracidin A and Ramoplanin A2 showed inhibition of seeded Tau aggregation. Among the tested compounds, Ramoplanin A2 showed the strongest dose-dependent inhibition of seeded Tau aggregation. In Figure 12, cells were further incubated with different concentrations of Ramoplanin A2 (0.25 µM to 7.5 µM) for 22 h in the presence of lipofectamine to assess the concentration needed to reach 50% inhibitory activity against seeded Tau aggregation. The percentage of total cells remained constant in the span of Ramoplanin A2 concentrations tested, indicating that Ramoplanin A2 exerts inhibitory activity without being cytotoxic.

Further, it was shown that Ramoplanin A2 inhibits the aggregation of Tau in HEK biosensor cells when Tau aggregation is induced by the second modality, which is exposure of HEK cells to Tau^{P301L} fibrils (Figure 13). Cells were incubated with the drug (0.25 µM to 40 µM) for 48 hours in the presence or absence of lipofectamine (Figure 13 A and B). The percentages of total cells and cells with aggregates (appearing as white spots) were quantified by microscopy (Figure 13 C). Cells incubated with a higher concentration of Ramoplanin A2 showed decreased aggregate formation. The IC₅₀ values were determined for data points corresponding to Ramoplanin A2 concentrations at which the percentage of total cells stayed above 70%.

Additionally, Figure 14 shows that Ramoplanin A2 inhibits the aggregation of Tau in HEK biosensor cells when Tau aggregation is induced by the third modality, which is exposure of HEK cells to brain homogenate of PS19 mice.

### Example 5: Ramoplanin A2 rescues Tau-induced degeneration of Drosophila eves

Fruit flies with (GMR-Gal4/WT Tau) and without (GMR-Gal4/+) Tau overexpression were treated with Ramoplanin A2 from the larvae stage up to the adult stage, with 5 replicates. Percent degeneration was determined by SEM imaging of Drosophila eyes (Figure 15 A). Ramoplanin A2 treatment lowers the percent degeneration of Drosophila eyes (Figure 15 B).

### Example 6: Inhibition of seeded aggregation of the NM region of the Sup35 prion protein (named "NM") bv Ramoplanin A2

HEK (Figure 16 A, B) or N2a cells (Figure 16 C) expressing soluble NM-GFP or HA-epitope tagged NM, respectively, were incubated with increasing concentrations of Ramoplanin for 1 h. 500 nM of sonicated, recombinant NM fibrils was added and cells were incubated for a total of 24 h. Cells with aggregates (aggregates appear as white spots) were analyzed by automated microscopy and image analysis. Figure 16 B shows images of HEK NM-GFP cells exposed to Ramoplanin A2 and recombinant NM fibrils. The percent of cells with aggregates of the prion protein was reduced with increasing concentration of Ramoplanin A2 in both cell lines.

### Example 7: Prevention of prion infection bv Ramoplanin A2

Prion-susceptible fibroblast cell clone L929 15.9 was pre-incubated with 3 µM Ramoplanin A2 or solvent control for 1 h and subsequently exposed to 1 % 22L scrapie brain homogenate (Figure 17). Cells were grown for 9 d in the presence of Ramoplanin A2 before cell lysates were analyzed for pathogenic prion protein PrP^{Sc}. Western blots of total cell lysates (total PrP and actin) and proteinase K resistant PrP^{Sc} show a reduction of pathogenic prion protein PrP^{Sc} and total PrP.

### Example 8: Inhibition of the in vitro aggregation of the Parkinson's disease-associated protein alpha-svnuclein (Asvn) by Ramoplanin A2 and the Ramoplanin A2 Aglycon derivative

The aggregation assay was performed by incubating 25 µM Asyn monomers with 1% (v/v) Asyn fibril seeds, 50 µM ThT, and aggregation buffer (50 mM HEPES, 100 mM NaCl, pH 7.4) containing 2.5 % v/v dimethyl sulfoxide (DMSO). Quantitative analysis of Asyn aggregation kinetics was performed by nonlinear regression with sigmoidal fitting to determine the midpoint value (Tₘ) of the fluorescence curve as well as the fluorescence span. Significantly larger Tₘ and lower span indicate inhibition of Asyn aggregation (Figure 18 A, B). TEM imaging analysis of the compound/Asyn mixtures show that in the presence of Ramoplanin A2, Asyn generally forms shorter fibrils thereby suggesting that Ramoplanin A2 blocks fibril elongation (Figure 18 C). On the other hand, Ramoplanin A2 Aglycon favors the formation of Asyn oligomers, which is a process that competes with fibril formation.

### Example 9: Inhibition of the in vitro aggregation of the Parkinson's disease-associated protein alpha-svnuclein (Asyn) bv Enduracidin A and B

The aggregation assay was performed by incubating 25 µM Asyn monomers with 1% (v/v) Asyn fibril seeds, 50 µM ThT and plain aggregation buffer (50 mM HEPES, 100 mM NaCl, pH 7.4) or aggregation buffer containing 2.5 % v/v dimethyl sulfoxide (DMSO). Quantitative analysis of Asyn aggregation kinetics was performed by nonlinear regression with sigmoidal fitting to determine the midpoint value (Tm) of the fluorescence curve as well as the fluorescence span. Significantly larger Tm and lower span indicate inhibition of Asyn aggregation (Figure 19 A, B). TEM imaging analysis of the compound/Asyn mixtures show that in the presence of Enduracidins A and B, Asyn generally forms shorter fibrils with a rougher surface (Figure 19 C). In addition, in the presence of Enduracidin B, Asyn oligomer formation also competes with Asyn fibril formation.

### Example 10: Inhibition of the in vitro aggregation of the Parkinson's disease-associated protein alpha-svnuclein (Asyn) by Teicoplanin

The aggregation assay was performed by incubating 25 µM Asyn monomers with 1% (v/v) Asyn fibril seeds, 50 µM ThT and aggregation buffer (50 mM HEPES, 100 mM NaCl, pH 7.4) containing 2.5 % v/v dimethyl sulfoxide (DMSO). Quantitative analysis of Asyn aggregation kinetics was performed by nonlinear regression with sigmoidal fitting to determine the midpoint value (Tm) of the fluorescence curve as well as the fluorescence span. Significantly larger Tm and lower span indicate inhibition of Asyn aggregation (Figure 20 A). TEM imaging analysis of the compound/Asyn mixture shows that in the presence of Teicoplanin, Asyn oligomer formation competes with Asyn fibril formation (Figure 20 B).

### Example 11: Inhibition of medin protein aggregation by Ramoplanin A2

The aggregation assay was performed by incubating 20 µM medin monomers with 20 µM ThT and aggregation buffer (20 mM sodium phosphate, 150 mM NaCl, pH 7.4) containing 2.5 % v/v dimethyl sulfoxide (DMSO) (Figure 21). TEM imaging analysis of the compound/medin mixtures show that in the presence of Ramoplanin A2, medin oligomer formation competes with medin fibril formation.

### Example 12: Inhibition of corneal lattice dystrophy-associated TGFBI peptide aggregation by Ramoplanin A2

The aggregation assay was performed by incubating 85 µM TGFBI monomers with 30 µM ThT and aggregation buffer (phosphate buffered saline: 137 mM NaCl, 2.7 mM KCI, 10 mM Na₂HPO₄, 1.8 mM KH₂PO₄, pH 7.4) containing 2.5 % v/v dimethyl sulfoxide (DMSO) (Figure 22). TEM imaging analysis of the compound/TGFBI mixture shows that in the presence of Ramoplanin A2, TGFBI forms shorter fibrils, indicating that Ramoplanin A2 inhibits fibril elongation.

### Example 13: Inhibition of diabetes-associated amvlin aggregation bv Ramoplanin A2, NAI-603 and Enduracidin B

The aggregation assay was performed by incubating 5 µM amylin (human islet amyloid polypeptide or hIAPP) monomers with 10 µM ThT and aggregation buffer (10 mM Tris-HCI with 100 mM NaCl, pH 7.4) containing 2.5 % v/v dimethyl sulfoxide (DMSO) (Figure 23). The hIAPP monomers were incubated with various doses of cyclic lipopeptide antibiotic compounds (Figure 23 A - Ramoplanin A2, Figure 23 B - NAI-603 or the 2-methylphenyl derivative of Ramoplanin, Figure 23 C - Enduracidin B). The percent of amylin aggregation inhibition was determined from the final ThT fluorescence values relative to the reference at the end of the assay (t = 50 h). All compounds tested effectively inhibit amylin fibrillization *in vitro.*

### Example 14: Structural features relevant to activity and proposed mechanism of action of cyclic lipopeptide antibiotic compounds in protein aggregation inhibition

As shown in Figure 24 A, to block fibril elongation, the compounds (in grey) engage in hydrophobic and hydrogen bonding interactions (dashed lines) with the exposed surface of a growing amyloid fibrils (in black outlines), thereby inhibiting recruitment of monomer proteins to the fibril. The compounds also block fibril formation by binding to the monomeric state of the protein thereby trapping the protein in an oligomeric state (combination of cyclic lipopeptide compound and protein) and disfavoring the formation of amyloid fibrils (Figure 24 B).

Important for binding to aggregation-prone proteins or to growing amyloid fibrils are aromatic pi-stacking interactions mediated by the aromatic side chains in ramoplanins, enduracidins, teicoplanins (examples of such side chains in hydroxyphenylglycine, chlorohydroxyphenylglycine, phenylalanine, dichlorohydroxyphenylglcine residues). At the level of a growing or elongating fibril, the hydrogen-bond acceptors and donors from the peptide backbones of these cyclic lipopeptides participate in hydrogen-bonds that stabilize a cross beta-sheet structure. Further, sugar and lipid moieties modulate solubility and promote cell uptake of the compounds.

### Methods:

### Abbreviations:

DMSO - dimethylsulfoxide
NMR - nuclear magnetic resonance
HMQC - heteronuclear multiple quantum coherence
TOCSY - total correlation spectroscopy
NOESY - nuclear Overhauser effect spectroscopy
STD - saturation transfer difference
ThT - Thioflavin T
TCEP - (tris(2-carboxyethyl)phosphine)
HEK - human embryonic kidney
GFP - green fluorescent protein
SDS-PAGE - sodium dodecyl sulfate polyacrylamide gel electrophoresis
TFA - trifluoroacetic acid
FCC - flash column chromatography
DMF - N,N-dimethylformamide
Fmoc - fluorenylmethyloxycarbonyl
Fmoc-ONSu - 9-fluorenylmethyl-succinimidyl carbonate
TEA - triethylamine
TFA - trifluoroacetic acid
PyBOP - benzotriazol-1-yloxytripyrrolidinophosphonium hexafluorophosphate
HCl - hydrochloric acid
HF - hydrofluoric acid
HPLC - high performance liquid chromatography

*Synthesis*/*Preparation of compounds No. 5 and 18 to 29:* Compounds 5 and 18 to 29 were prepared using a general scheme according to Figure 25 based on a published method (Ciabatti et al., 2007) that uses carboxylic acid precursors of the lipid moiety (Table 2, R¹).

In Step **a,** Orn-4 and Orn-10 side chain amino groups were protected by capping with fluorenylmethyloxycarbonyl (Fmoc) groups. Ramoplanin A2 (Compound No. 1) was dissolved in DMF (500 mg or 0.2 mmol in 2.5 mL solvent) at 0°C under a constant stream of nitrogen. To the solution, 0.1 mmol of 9-fluorenylmethyl-succinimidyl carbonate (Fmoc-ONSu) and 0.2 mmol TEA were added. After 10 minutes, another batch of 0.3 mmol Fmoc-ONSu and 0.2 mmol TEA were added and the mixture was brought to room temperature. The crude product was washed in methanol:water (1:9) and adjusted to pH 5.0 using acetic acid. The solid residue was separated by filtration and dried in vacuo. In Step **b,** 0.2 mmol of the purified product (4,10-diFmoc protected Ramoplanin A2) from Step **a** was dissolved in a mixture of methanol/DMF (3:1, 13 mL) and cooled to -80°C. Ozone (5% ozone in oxygen gas) was introduced into the mixture while stirring constantly for 30 min. Ozonolysis was quenched by bubbling nitrogen gas into the mixture. Triphenylphosphine (0.4 mmol) was added, and the mixture was brought to room temperature. The solvents were removed in vacuo and the precipitate (4,10-diFmoc protected Ramoplaninaldehyde derivative) was washed with ethyl acetate and dried. In Step **c,** 0.2 mmol of the purified product from step **b** was added to DMF (5 mL) together with benzylamine hydrobromide (0.8 mmol) and NaCNBH₃ (0.3 mmol) with constant stirring. The mixture was incubated for 2 h at room temperature and subsequently transferred in 50 mL water. The solid residue was filtered and dried at room temperature in vacuo. The dried residue was redissolved in 1:1 acetonitrile/acidified water mixture (brought to pH 2.0 using hydrochloric acid) and subjected to silica flash column chromatography (FCC) eluting with a gradient of water/acetonitrile. The eluted product (4,10-diFmoc protected Ramoplanin-benzylamine derivative) was filtered, washed with water, and dried in vacuo. In Step **d,** the purified product (0.05 mmol) from step c was dissolved in a solution of 1:1 pyridine/water (1 mL) with phenyl isothiocyanate (0.06 mmol) with continuous stirring at room temperature. The solvent was removed by evaporation and the residue was washed in toluene. The solid was added to dichloromethane (1 mL), after which TFA (1 mL) was introduced, and the mixture was incubated at 40°C. The solvents were removed by evaporation and the resulting oily residue was combined with diethyl ether (1 mL) and dried under reduced pressure, to yield a solid product (4,10-diFmoc protected Ramoplanin-amine derivative). The solid was washed in water, filtered, and dried. The product of Step **d** could in principle react with various carboxylic acids containing R¹ groups in Table 2. The condensation reaction in Step **e** generally follows the procedure in which the product of Step **d** (0.3 mmol) was combined with triethylamine (1.0 mmol) and the suitable carboxylic acid precursor (0.5 mmol) in DMF (10 mL). To this mixture, PyBOP (0.5 mmol) was added, and the mixture was stirred and incubated at room temperature for 5 h to yield the product 4,10-diFmoc protected Ramoplanin-R¹ derivative. Deprotection in Step **f** was performed with 2 mL 2,2,6,6-tetramethylpiperidine with a 30 min incubation period. The reaction mixture was acidified with 6 mL of 1 M HCI and the final product (examples include Compounds No. 5, 18-29) was purified by reversed-phase HPLC and lyophilized.

*Synthesis*/*Preparation of Aglycon Derivatives of Ramoplanin and Ramoplanose:* Removal of the sugar substituents was accomplished based on a published method (Wanner et al., 2003). Anhydrous HF (5 mL) was added to Ramoplanins A1-A3 (compound No. 1 to 3) or Ramoplanose (compound No. 4, 0.04 mmol) and anisole (0.5 mL) at -78°C. The reaction mixture was brought to 0°C and continuously stirred for 1.5 h. HF was evaporated using a stream of nitrogen gas. Ethanol (1 mL) was used to wash the residue, and the solvent was removed under reduced pressure. The solid was dissolved in 0.1 M HCI in water and lyophilized. Reversed-phase HPLC was used to purify the aglycon using a gradient of 20-50% acetonitrile/ water with 0.05 M ammonium formate.

*NMR spectroscopy:* Stocks of the compounds for NMR experiments were prepared by dissolving the powder directly in NMR buffer (50 mM sodium phosphate buffer at pH 6.8, 0.01% NaN₃), in HEPES buffer (25 mM HEPES), or as 10 mM compound in deuterated DMSO. To obtain residue-specific information about the interaction between Tau and the compounds, ¹H-¹⁵N correlation spectra of [U-¹⁵N]-Tau (2N4R) were monitored over the course of a titration with increasing amounts of compound. The ¹H-¹⁵N SOFAST-HMQC spectra (Schanda et al., 2005) of [U-¹⁵N]-Tau were acquired at 5°C on a Bruker 800 MHz spectrometer equipped with a triple-resonance cryoprobe. Tau samples (18 µM) were prepared in NMR buffer containing 10% (v/v) D₂O, and with compound concentrations that corresponded to Tau: compound mole ratios of 1:0.3 up to 1:30. All NMR samples were incubated overnight (~16h) at 37°C prior to spectral acquisition. Tau amide assignments were taken from a previous study (Mukrasch et al., 2009) supplemented by backbone assignments in the repeat region of Tau, represented by [U-¹³C,¹⁵N]-4R Tau (K18 construct). Backbone assignments were determined by analyzing 3D HNCA, 3D HNCOCA, and 3D CBCACONH spectra of [U-¹³C,¹⁵N]-4R Tau. The normalized chemical shift perturbations (CSP) were calculated as CSP = √ [0.5 [(Δδ H)² + (Δδ N)²/25]. NMR peak intensity ratios, I/I₀ (in which I represents the peak intensity of Tau amide backbone resonances in the presence of the compounds, and I₀ corresponds to the peak intensity in the absence of the compounds), were calculated for each titration condition. To facilitate structure-activity relationships between Ramoplanin A2 (compound No. 1) and Tau, assignments of non-labile protons of Ramoplanin A2 were determined by 1D ¹H NMR, 2D ¹H-¹H TOCSY with 80 ms mixing time, and 2D ¹H-¹H NOESY with 200 ms mixing time. The spectra were recorded at 5°C in NMR buffer. The unambiguous proton assignments were identified by a sequential walk following NOE connectivities between backbone amide protons (H^{N}) and the H^{A-1} protons. Experiments were performed at 5°C using a Bruker 600 MHz spectrometer equipped with a triple resonance cryoprobe. The assignments were subsequently transferred to spectra at 25°C and 37°C facilitated by a temperature titration experiment, in which the 1D ¹H NMR spectra of Ramoplanin A2 were collected at 5°C, 12°C, 17°C, 22°, 27°C, 32°C, 37°C.

To determine binding epitopes of the Ramoplanin A2 for monomeric 2N4R Tau, 1D ¹H-NMR titrations were performed in which the spectra of Ramoplanin A2 (200 µM compound) were recorded with unlabeled 2N4R Tau (0, 10, 20 and 30 µM) at 37°C in NMR buffer. NMR spectra were recorded at 37°C using a Bruker 600 MHz spectrometer equipped with a triple resonance cryoprobe. Chemical shift perturbations (CSPs) of protons in Ramoplanin A2 were determined by subtracting the chemical shift of the proton in the absence of Tau from the corresponding chemical shift in the presence of Tau (proton CSP = δ H_{compound+Tau} - δ H_{compound} reference). Protons with large CSPs represent binding epitopes of Ramoplanin A2 for monomeric Tau. The proton CSPs were mapped onto the structural model of Ramoplanin A2 (PDB entry 1DSR). Secondary structure determination and solvent-excluded molecular surface construction were performed using Chimera version 1.14 (Goddard et al., 2018). To determine binding epitopes of the compounds for fibrillar Tau, 1D saturation-transfer difference (STD) experiments were performed with Ramoplanin A2 and sonicated 2N4R Tau fibrils (circa 100 nm fibril length). 2N4R Tau fibrils were prepared using the aggregation procedure described in the section *In vitro assay* 1without Thioflavin T. The mole ratio of Ramoplanin A2 to fibrils was 48:1. The STD NMR samples were prepared in 25 mM HEPES, pH 7.2, 100 mM NaCl and spectra were recorded at 37°C using a Bruker 700 MHz spectrometer equipped with a triple resonance cryoprobe. 1D ¹H NMR STD spectra were acquired in an interleaved manner, alternating between off-resonance irradiation at 60 ppm and on-resonance irradiation at -2 ppm. NMR spectra were processed using Topspin 3.6.2 (Bruker) and analyzed using NMRFAM-Sparky (Lee et al., 2015).

### In vitro assay procedures:

*In vitro assay 1: de novo and seeded Tau aggregation assays:* Aggregation of 2N4R Tau (residues 1-441 of human Tau, Uniprot entry P10636-8) was performed using a modified version of a published co-factor-free aggregation protocol (Chakraborty et al., 2021). 2N4R Tau protein stock solutions were prepared in 25 mM HEPES pH 7.4, 1 mM TCEP. Stocks of compounds were prepared in aggregation buffer (25 mM HEPES, 10 mM KCI, 5 mM MgCl₂, pH 7.2). Tau fibril seeds were prepared by *de novo* aggregation at 37°C, in which 2N4R Tau (monomer concentration of 25 µM) was combined with 50 µM Thioflavin T (ThT) in aggregation buffer and incubated in a 96-well microplate (Greiner Bio-one). Each well contained 100 µL Tau-ThT solution and two polytetrafluoroethylene beads. The microplate was periodically subjected to intervals of double orbital shaking and ThT fluorescence emission measurement using a program implemented in a Tecan Spark plate reader. The excitation wavelength for ThT was set as 430 nm and the emission wavelength was 485 nm. After about 3-6 days of incubation, ThT fluorescence emission reached saturation, indicating that Tau fibrils were in chemical equilibrium with other non-fibrillar species. The fibril-containing aggregation mixture collected from the microplate was used as a "fibril seed" mixture for the further aggregation assays. Seeded aggregation assays were performed as described above but with 1.0% of the well volume occupied by the fibril seed mixture sonicated for 1 minute at room temperature. The assays were performed in the presence and absence of compounds, with working concentrations ranging 0 - 250 µM. Analyses of the ThT fluorescence profile were performed using Graphpad PRISM version 8. Fluorescence data were fit to a sigmoid function. The number of hours corresponding to half-maximal ThT fluorescence (Tm) as well as the span of fluorescence intensity (Span) were determined for each curve. Statistically significant differences were determined by one-way ANOVA analysis with Dunnett's multiple comparison test.

*In vitro assay 2: Tau fibril dissolution assay:* To observe the effect of compounds on pre-formed Tau fibrils, seeded aggregation assays with 2N4R Tau in the absence of compounds was performed according to *in vitro assay 1.* After 25 h, Ramoplanin A2 (compound No. 1) (with Tau: compound mole ratios of 1:0.3 and 1:1) or buffer (Reference) were added to the pre-formed Tau fibrils in the aggregation assay mixture. Incubation with the fibrils was continued for 12 h while performing double orbital shaking and fluorescence measurement. After incubation, the span of fluorescence curves was quantified before and after addition of the compound or buffer control. A statistically significant decrease in fluorescence span is indicative of fibril dissolution. Retention of the fluorescence span indicates no fibril dissolution. Statistically significant differences were determined by one-way ANOVA analysis with Dunnett's multiple comparison test.

*In vitro assay 3: Tau pelleting assay:* Aggregation assays were performed as described in the section *In vitro assay 1.* The aggregation mixtures were harvested and ultracentrifuged (55000 rpm using a JLA 100.3 rotor, Optima MAX-XP) for 30 minutes at 37°C to separate the soluble (supernatant) and insoluble (pellet) fractions. Soluble fractions were analyzed by SDS-PAGE using a 12% or 15% acrylamide resolving gel and a 5% acrylamide stacking gel. Band intensities of soluble Tau protein were quantified by ImageLab (Bio-Rad Laboratories).

*In vitro assay 4: de novo and seeded α-synuclein aggregation assays:* Aggregation of α-synuclein (Asyn, residues 1-140 of human α-synuclein, Uniprot Entry P37840, acetylated at the N-terminus) was performed using a protocol similar to *in vitro assay 1.* Asyn protein stock solutions were prepared in 50 mM HEPES at pH 7.4 with 100 mM NaCl. Stocks of compounds were prepared in the same buffer (500 µM) or as 10 mM compound in DMSO. Asyn fibril seeds were prepared by *de novo* aggregation at 37°C, in which Asyn (monomer concentration of 25 µM) was combined with 50 µM Thioflavin T (ThT) in 50 mM HEPES at pH 7.4 with 100 mM NaCl and incubated in a 96-well microplate as a 100-µL sample with two polytetrafluoroethylene beads. The microplate was likewise subjected to the incubation and fluorescence measurement program used in *in vitro assay 1.* After about 2-4 days of incubation, ThT fluorescence emission reached the maximal value and the Asyn fibril-containing mixture was collected as "fibril seed" mixture for subsequent Asyn aggregation experiments. Seeded aggregation assays were performed as described above but with 1.0% of the well volume occupied by the Asyn fibril seed mixture sonicated for 1 minute at room temperature. The assays were performed in the presence and absence of compounds, with working concentrations ranging 0 - 250 µM. Analyses of the ThT fluorescence profile were performed as described in *in vitro assay 1.*

*Negative stain transmission electron microscopy:* Fibril samples were negative stained using glow discharged 400 mesh carbon-coated copper grids. After staining using a 1% uranyl acetate solution, images were acquired on a Talos L120C transmission electron microscope (Thermo Fisher, Eindhoven, The Netherlands).

### Biological assay procedures

*Biological assay 1: HEKbiosensor cell Tau seeding assay:* Compound stock solutions were prepared as 10 mM compound in DMSO. HEK293T biosensor cells expressing the human tau repeat domain (RD) containing the P301L mutation and fused to a C-terminal GFP tag (named TauRD^{LM}-GFP) were engineered as described in a published method (Liu et al., 2021). HEK Cells were incubated with pre-sonicated Tau^{P301L} fibrils to induce Tau aggregation in cells. Cells were harvested and the resulting cell lysate was used to seed Tau aggregation in further experiments. In a typical seeded assay, HEK cells were incubated with cell lysate, compounds (0-40 µM) and lipofectamine for 22-48 hours. Negative control experiments in the absence of compounds (only with vehicle DMSO) and in the presence of cell lysate were performed in parallel. The number of cells with aggregates for each compound concentration were plotted as dose response curves. The curves were fitted with a sigmoid function using Graphpad PRISM version 8. The inhibitory concentration at 50% activity (IC₅₀) was determined for each compound. The HEK cell Tau seeding assay was likewise performed with the abovementioned protocol using brain homogenate of PS19 mice (mouse model of tauopathy) as seeding mixture.

*Biological assay 2: HEK biosensor cell NM seeding assay:* HEK cells were engineered to express soluble NM-GFP (in which "NM" refers to the NM region of the Sup35 prion protein) (Liu et al., 2021). Cells were incubated with Ramoplanin A2 (compound No. 1) or solvent control (DMSO) for 1 h using working concentrations of 0-26.5 µM Ramoplanin A2. 500 nM of sonicated, recombinant NM fibrils was added and cells were incubated for 24 h. Nuclei were stained with Hoechst. Cells with aggregates were counted by automated microscopy and image analysis using CellVoyager 6000. Statistical analysis and IC₅₀ determination were performed using GraphPad Prism version 8.

*Biological assay 3: N2a biosensor cell NM seeding assay:* N2a cells were engineered to express soluble HA-epitope tagged NM (Liu et al., 2021) Cells were incubated with Ramoplanin A2 (compound No. 1) or solvent control (DMSO) for 1 h using working concentrations of 0-26.5 µM Ramoplanin A2. 500 nM of sonicated, recombinant NM fibrils was added and cells were incubated for 24 h. Cell nuclei were stained with Hoechst and NM-HA was stained using anti-HA antibodies. Cells with aggregates were counted by automated microscopy and image analysis using CellVoyager 6000. Statistical analysis and IC₅₀ determination were performed using GraphPad Prism version 8.

*Biological assay 4: Prion infection cell assay:* Prion-susceptible fibroblast cell clone 15.9 (Fehlinger et al., 2017). was incubated with 3 µM Ramoplanin A2 (compound No. 1) or solvent control (DMSO) for 1 h. Cells were afterwards exposed to 1% 22L scrapie brain homogenate. Cells were grown for 9 d with Ramoplanin A2, after which cell lysates were analyzed by Western blotting for pathogenic prion protein PrP^{Sc}. Total PrP, actin control, and proteinase K resistant PrP^{Sc} were quantified. PrP was detected using anti-PrP antibody 4H11 (Ertmer et al., 2004). Western blot analysis was performed in triplicate.

### Biological assay 5: Drosophila neurodegeneration model

*Drug treatments in Drosophila:* Stock solutions of Ramoplanin A2 (compound No. 1) were prepared by dissolving 10 mg Ramoplanin in 100 µl of 100% DMSO. Ramoplanin A2 was orally administered to the flies (GMR-Gal4/WT Tau) by mixing the stock solution with food. The working concentration of Ramoplanin A2 was selected based on recommended concentrations for human use, adjusted based on body weight. Three concentrations of Ramoplanin A2 were tested (15, 30, and 44 µM) with 5 replicates. After hatching, first instar larvae were transferred onto the food containing Ramoplanin A2. Food mixed with compound was replaced every 48 h.

*Light Microscopy*: Fly heads were mounted on a glass slide. Full-eye images were recorded using an Infinity Analyze camera attached to an Olympus SZX7 microscope with a halogen light source. Total eye surface area and degenerated eye surface area were quantified using Infinity Analyze software (Lumenera Corporation). The percentage of degenerated area was calculated and plotted using GraphPad Prism version 8.

*Scanning Electron Microscopy*: After the eclosion, treated and control flies (2-3 days old) were fixed in 1% formaldehyde for two hours, followed by serial dehydration steps of 12 h each in 25%, 50%, 75% and 100% ethanol. Flies were stored in 100% ethanol and dried with CPD before mounting on the stab for imaging. Eye Images were captured at 150X and analyzed using Infinity Analyze software.

### REFERENCES

Valastyan, J. S. & Lindquist, S. Mechanisms of protein-folding diseases at a glance. Dis. Model. Mech. 7, 9-14 (2014).
Kahn, S. E., Andrikopoulos, S. & Verchere, C. B. Islet amyloid: a long-recognized but underappreciated pathological feature of type 2 diabetes. Diabetes 48, 241-253 (1999).
Sipe, J. D. & Cohen, A. S. Review: History of the Amyloid Fibril. J. Struct. Biol. 130, 88-98 (2000).
Benson, M. D. et al. Amyloid nomenclature 2020: update and recommendations by the International Society of Amyloidosis (ISA) nomenclature committee. Amyloid Int. J. Exp. Clin. Investig. Off. J. Int. Soc. Amyloidosis 27, 217-222 (2020).
Burré, J., Sharma, M. & Südhof, T. C. Cell Biology and Pathophysiology of α-Synuclein. Cold Spring Harb. Perspect. Med. 8, (2018).
Colligris, P., Perez de Lara, M. J., Colligris, B. & Pintor, J. Ocular Manifestations of Alzheimer's and Other Neurodegenerative Diseases: The Prospect of the Eye as a Tool for the Early Diagnosis of Alzheimer's Disease. J. Ophthalmol. 2018, 8538573 (2018).
Perez-Garmendia, R. et al. Interplay between Oxidative Stress, Inflammation, and Amyloidosis in the Anterior Segment of the Eye; Its Pathological Implications. Oxid. Med. Cell. Longev. 2020, 6286105 (2020).
Koronyo-Hamaoui, M. et al. Identification of amyloid plaques in retinas from Alzheimer's patients and noninvasive in vivo optical imaging of retinal plaques in a mouse model. Neuroimage 54 Suppl 1, S204-17 (2011).
Koronyo, Y., Salumbides, B. C., Black, K. L. & Koronyo-Hamaoui, M. Alzheimer's disease in the retina: imaging retinal aβ plaques for early diagnosis and therapy assessment. Neurodegener. Dis. 10, 285-293 (2012).
Jiang, J., Wang, H., Li, W., Cao, X. & Li, C. Amyloid Plaques in Retina for Diagnosis in Alzheimer's Patients: a Meta-Analysis. Front. Aging Neurosci. 8, 267 (2016).
La Morgia, C. et al. Melanopsin retinal ganglion cell loss in Alzheimer disease. Ann. Neurol. 79, 90-109 (2016).
Koronyo, Y. et al. Retinal amyloid pathology and proof-of-concept imaging trial in Alzheimer's disease. JCI insight 2, (2017).
Goldstein, L. E. et al. Cytosolic beta-amyloid deposition and supranuclear cataracts in lenses from people with Alzheimer's disease. Lancet (London, England) 361, 1258-1265 (2003).
Schön, C. et al. Long-Term In Vivo Imaging of Fibrillar Tau in the Retina of P301S Transgenic Mice. PLoS One 7, e53547 (2013).
Johnson, L. V et al. The Alzheimer's A beta -peptide is deposited at sites of complement activation in pathologic deposits associated with aging and age-related macular degeneration. Proc. Natl. Acad. Sci. U. S. A. 99, 11830-11835 (2002).
Kittleson, M. M. et al. Cardiac Amyloidosis: Evolving Diagnosis and Management: A Scientific Statement from the American Heart Association. Circulation 142, e7-e22 (2020).
Westermark, P. Amyloid and polypeptide hormones: What is their interrelationship? Amyloid 1, 47-60 (1994).
Clark, A. et al. Islet amyloid, increased A-cells, reduced B-cells and exocrine fibrosis: quantitative changes in the pancreas in type 2 diabetes. Diabetes Res. 9, 151-159 (1988).
Kanapathipillai, M. Treating p53 Mutant Aggregation-Associated Cancer. Cancers (Basel). 10, (2018).
Soragni, A. et al. A Designed Inhibitor of p53 Aggregation Rescues p53 Tumor Suppression in Ovarian Carcinomas. Cancer Cell 29, 90-103 (2016).
De Smet, F. et al. Nuclear inclusion bodies of mutant and wild-type p53 in cancer: a hallmark of p53 inactivation and proteostasis remodelling by p53 aggregation. J. Pathol. 242, 24-38 (2017).
Ano Bom, A. P. D. et al. Mutant p53 aggregates into prion-like amyloid oligomers and fibrils: implications for cancer. J. Biol. Chem. 287, 28152-28162 (2012).
Poduslo, J. F., Curran, G. L., Kumar, A., Frangione, B. & Soto, C. Beta-sheet breaker peptide inhibitor of Alzheimer's amyloidogenesis with increased blood-brain barrier permeability and resistance to proteolytic degradation in plasma. J. Neurobiol. 39, 371-382 (1999).
Estrada, L. D. & Soto, C. Inhibition of protein misfolding and aggregation by small rationallydesigned peptides. Curr. Pharm. Des. 12, 2557-2567 (2006).
Bett, C. K., Serem, W. K., Fontenot, K. R., Hammer, R. P. & Garno, J. C. Effects of peptides derived from terminal modifications of the aβ central hydrophobic core on aβ fibrillization. ACS Chem. Neurosci. 1, 661-678 (2010).
Griffin, M. D. W. et al. A Cyclic Peptide Inhibitor of ApoC-II Peptide Fibril Formation: Mechanistic Insight from NMR and Molecular Dynamics Analysis. J. Mol. Biol. 416, 642-655 (2012).
Kapurniotu, A. et al. Conformational restriction via cyclization in beta-amyloid peptide Abeta(1-28) leads to an inhibitor of Abeta(1-28) amyloidogenesis and cytotoxicity. Chem. Biol. 10, 149-159 (2003).
Zheng, J. et al. Macrocyclic β-Sheet Peptides That Inhibit the Aggregation of a Tau-Protein-Derived Hexapeptide. J. Am. Chem. Soc. 133, 3144-3157 (2011).
Luo, J. & Abrahams, J. P. Cyclic peptides as inhibitors of amyloid fibrillation. Chemistry 20, 2410-2419 (2014).
Ciabatti, R. et al. Synthesis and Preliminary Biological Characterization of New Semisynthetic Derivatives of Ramoplanin. J. Med. Chem. 50, 3077-3085 (2007).
Wanner, J. et al. A new and improved method for deglycosidation of glycopeptide antibiotics exemplified with vancomycin, ristocetin, and ramoplanin. Bioorg. Med. Chem. Lett. 13, 1169-1173 (2003).
Schanda, P., Kupce, E. & Brutscher, B. SOFAST-HMQC experiments for recording twodimensional heteronuclear correlation spectra of proteins within a few seconds. J. Biomol. NMR 33, 199-211 (2005).
Mukrasch, M. et al. Structural polymorphism of 441-residue tau at single residue resolution. PLoS Biol. 7, e34 (2009).
Goddard, T. D. et al. UCSF ChimeraX: Meeting modern challenges in visualization and analysis. Protein Sci. 27, 14-25 (2018).
Lee, W., Tonelli, M. & Markley, J. L. NMRFAM-SPARKY: enhanced software for biomolecular NMR spectroscopy. Bioinformatics 31, 1325-1327 (2015).
Chakraborty, P. et al. Co-factor-free aggregation of tau into seeding-competent RNAsequestering amyloid fibrils. Nat. Commun. 12, 4231 (2021).
Liu, S. et al. Highly efficient intercellular spreading of protein misfolding mediated by viral ligandreceptor interactions. Nat. Commun. 12, 5739 (2021).
Fehlinger, A. et al. Prion strains depend on different endocytic routes for productive infection. Sci. Rep. 7, 6923 (2017).
Ertmer, A. et al. The Tyrosine Kinase Inhibitor STI571 Induces Cellular Clearance of PrPSc in Prion-infected Cells*. J. Biol. Chem. 279, 41918-41927 (2004).
Serrano-Pozo, A., Frosch, M. P., Masliah, E. & Hyman, B. T. Neuropathological Alterations in Alzheimer Disease. Cold Spring Harb. Perspect. Med. 1, (2011).
Zhang, C. & Saunders, A. J. Therapeutic targeting of the alpha-secretase pathway to treat Alzheimer's disease. Discov. Med. 7, 113-117 (2007).
Kang, J. et al. The precursor of Alzheimer's disease amyloid A4 protein resembles a cell-surface receptor. Nature 325, 733-736 (1987).
Nizynski, B., Dzwolak, W. & Nieznanski, K. Amyloidogenesis of Tau protein. Protein Sci. 26, 2126-2150 (2017).
Goedert, M., Crowther, R. A. & Spillantini, M. G. Tau mutations cause frontotemporal dementias. Neuron 21, 955-958 (1998).
Frost, B., Jacks, R. L. & Diamond, M. I. Propagation of tau misfolding from the outside to the inside of a cell. J. Biol. Chem. 284, 12845-12852 (2009).
Guo, J. L. & Lee, V. M.-Y. Seeding of normal Tau by pathological Tau conformers drives pathogenesis of Alzheimer-like tangles. J. Biol. Chem. 286, 15317-15331 (2011).
Sanders, D. W. et al. Distinct tau prion strains propagate in cells and mice and define different tauopathies. Neuron 82, 1271-1288 (2014).
Soto, C. & Pritzkow, S. Protein misfolding, aggregation, and conformational strains in neurodegenerative diseases. Nat. Neurosci. 21, 1332-1340 (2018).
Soto, C. Transmissible proteins: expanding the prion heresy. Cell 149, 968-977 (2012).
Ladogana, A. et al. Mortality from Creutzfeldt-Jakob disease and related disorders in Europe, Australia, and Canada. Neurology 64, 1586-1591 (2005).
Manix, M. et al. Creutzfeldt-Jakob disease: updated diagnostic criteria, treatment algorithm, and the utility of brain biopsy. Neurosurg. Focus 39, E2 (2015).
Knight, R. S. G. & Will, R. G. Prion diseases. J. Neurol. Neurosurg. Psychiatry 75 Suppl 1, i36-42 (2004).
Dickson, D. W. Parkinson's disease and parkinsonism: neuropathology. Cold Spring Harb. Perspect. Med. 2, a009258 (2012).
Jakes, R., Spillantini, M. G. & Goedert, M. Identification of two distinct synucleins from human brain. FEBS Lett. 345, 27-32 (1994).
Lavedan, C. The synuclein family. Genome Res. 8, 871-880 (1998).
Moshirfar, M., West, W. & Ronquillo, Y. Lattice Corneal Dystrophy. (StatPearls [Internet], 2021).
Klintworth, G. K. The molecular genetics of the corneal dystrophies--current status. Front. Biosci. 8, d687-713 (2003).
Weiss, J. S. et al. IC3D classification of corneal dystrophies--edition 2. Cornea 34, 117-159 (2015).
Skonier, J. et al. cDNA cloning and sequence analysis of beta ig-h3, a novel gene induced in a human adenocarcinoma cell line after treatment with transforming growth factor-beta. DNA Cell Biol. 11, 511-522 (1992).
Escribano, J., Hernando, N., Ghosh, S., Crabb, J. & Coca-Prados, M. cDNA from human ocular ciliary epithelium homologous to beta ig-h3 is preferentially expressed as an extracellular protein in the corneal epithelium. J. Cell. Physiol. 160, 511-521 (1994).
Solomon, J. P., Page, L. J., Balch, W. E. & Kelly, J. W. Gelsolin amyloidosis: genetics, biochemistry, pathology and possible strategies for therapeutic intervention. Crit. Rev. Biochem. Mol. Biol. 47, 282-296 (2012).
Kwiatkowski, D. J. Functions of gelsolin: motility, signaling, apoptosis, cancer. Curr. Opin. Cell Biol. 11, 103-108 (1999).
Bucki, R., Levental, I., Kulakowska, A. & Janmey, P. A. Plasma gelsolin: function, prognostic value, and potential therapeutic use. Curr. Protein Pept. Sci. 9, 541-551 (2008).
Grogan, M., Dispenzieri, A. & Gertz, M. A. Light-chain cardiac amyloidosis: strategies to promote early diagnosis and cardiac response. Heart 103, 1065-1072 (2017).
Peng, S., Glennert, J. & Westermark, P. Medin-amyloid: a recently characterized age-associated arterial amyloid form affects mainly arteries in the upper part of the body. Amyloid Int. J. Exp. Clin. Investig. Off. J. Int. Soc. Amyloidosis 12, 96-102 (2005).
Wagner, J. et al. Medin amyloid forms age-associated aggregates in the brain vasculature and may contribute to cerebral β-amyloidosis. Alzheimer's Dement. 16, e042861 (2020).
Migrino, R. Q. et al. Cerebrovascular medin is associated with Alzheimer's disease and vascular dementia. Alzheimer's Dement. Diagnosis, Assess. Dis. Monit. 12, e12072 (2020).
Westermark, P. et al. Amyloid fibrils in human insulinoma and islets of Langerhans of the diabetic cat are derived from a neuropeptide-like protein also present in normal islet cells. Proc. Natl. Acad. Sci. U. S. A. 84, 3881-3885 (1987).
Kahn, S. E. et al. Evidence of cosecretion of islet amyloid polypeptide and insulin by beta-cells. Diabetes 39, 634-638 (1990).
Marzban, L., Park, K. & Verchere, C. B. Islet amyloid polypeptide and type 2 diabetes. Exp. Gerontol. 38, 347-351 (2003).
Wang, J. et al. The prohormone convertase enzyme 2 (PC2) is essential for processing pro-islet amyloid polypeptide at the NH2-terminal cleavage site. Diabetes 50, 534-539 (2001).
Cavalleri, B., Pagani, H., Volpe, G., Selva, E. & Parenti, F. A-16686, a new antibiotic from Actinoplanes. I. Fermentation, isolation and preliminary physico-chemical characteristics. J. Antibiot. (Tokyo). 37, 309-317 (1984).
Parenti, F., Ciabatti, R., Cavalleri, B. & Kettenring, J. Ramoplanin: a review of its discovery and its chemistry. Drugs Exp. Clin. Res. 16, 451-455 (1990).
Skelton, N. J. et al. Structure elucidation and solution conformation of the glycopeptide antibiotic ramoplanose (UK-71,903): a cyclic depsipeptide containing an antiparallel .beta.-sheet and a .beta.-bulge. J. Am. Chem. Soc. 113, 7522-7530 (1991).
Rolston, K. V et al. In-vitro activity of ramoplanin (a novel lipoglycopeptide), vancomycin, and teicoplanin against gram-positive clinical isolates from cancer patients. J. Antimicrob. Chemother. 38, 265-269 (1996).
Reynolds, P. E. & Somner, E. A. Comparison of the target sites and mechanisms of action of glycopeptide and lipoglycodepsipeptide antibiotics. Drugs Exp. Clin. Res. 16, 385-389 (1990). Swartz, M. N. Hospital-acquired infections: diseases with increasingly limited therapies. Proc. Natl. Acad. Sci. 91, 2420-2427 (1994).
von Nussbaum, F., Brands, M., Hinzen, B., Weigand, S. & Häbich, D. Antibacterial natural products in medicinal chemistry--exodus or revival? Angew. Chem. Int. Ed. Engl. 45, 5072-5129 (2006).
Kurz, M. & Guba, W. 3D structure of ramoplanin: a potent inhibitor of bacterial cell wall synthesis. Biochemistry 35, 12570-12575 (1996).
Higashide, E., Hatano, K., Shibata, M. & Nakazawa, K. Enduracidin, a new antibiotic. I. Streptomyces fungicidicus No. B5477, an enduracidin producing organism. J. Antibiot. (Tokyo). 21, 126-137 (1968).
Fang, X. et al. The mechanism of action of ramoplanin and enduracidin. Mol. Biosyst. 2, 69-76 (2006).
Castiglione, F., Marazzi, A., Meli, M. & Colombo, G. Structure elucidation and 3D solution conformation of the antibiotic enduracidin determined by NMR spectroscopy and molecular dynamics. Magn. Reson. Chem. 43, 603-610 (2005).
Meyers, E. et al. Janiemycin, a new peptide antibiotic. J. Antibiot. (Tokyo). 23, 502-507 (1970).
Bruniera, F. R. et al. The use of vancomycin with its therapeutic and adverse effects: a review. Eur. Rev. Med. Pharmacol. Sci. 19, 694-700 (2015).
Ashford, P.-A. & Bew, S. P. Recent advances in the synthesis of new glycopeptide antibiotics. Chem. Soc. Rev. 41, 957-978 (2012).
Parenti, F., Beretta, G., Berti, M. & Arioli, V. Teichomycins, new antibiotics from Actinoplanes teichomyceticus Nov. Sp. I. Description of the producer strain, fermentation studies and biological properties. J. Antibiot. (Tokyo). 31, 276-283 (1978).
Campoli-Richards, D. M., Brogden, R. N. & Faulds, D. Teicoplanin. A review of its antibacterial activity, pharmacokinetic properties and therapeutic potential. Drugs 40, 449-486 (1990).
Zeckel, M. L. & Woodworth, J. R. Vancomycin: a clinical overview. in Glycopeptide Antibiotics (ed. Nagarajan, R.) (CRC Press, 1994). doi:https://doi.org/10.1201/9781003067269.
PHILIP, J. E., SCHENCK, J. R. & HARGIE, M. P. Ristocetins A and B, two new antibiotics; isolation and properties. Antibiot. Annu. 699-705.
Newton, R. M. & Ward, V. G. LEUKOPENIA ASSOCIATED WITH RISTOCETIN (SPONTIN) ADMINISTRATION: REPORT OF TWO CASES. J. Am. Med. Assoc. 166, 1956-1959 (1958).
Gangarosa, E. J., Landerman, N. S., Rosch, P. J. & Herndon, E. G. Hematologic Complications Arising during Ristocetin Therapy. N. Engl. J. Med. 259, 156-161 (1958).
Dowling, S. V, Muntz, R. H., D'Souza, S. & Ekert, H. Ristocetin in the diagnosis of von willebrand's disease: a comparison of rate and percent of aggregation with levels of the plasma factor(s) necessary for ristocetin aggregation. Thromb. Diath. Haemorrh. 34, 465-474 (1975).

## Claims

1. A cyclic lipopeptide antibiotic compound, or derivative and/or salt thereof, for use in inhibiting protein aggregation in the treatment and/or prevention of a medical condition associated with protein aggregation.

2. The compound for use according to claim 1, wherein the treatment comprises the reduction and/or inhibition of amyloid protein aggregation in a subject.

3. The compound for use according to any one of the preceding claims, wherein the treatment comprises:
a. binding of said compound to a protein and inhibiting protein aggregate formation and/or fibril elongation, and/or
b. inhibition of association between monomers of aggregating proteins, inhibition of protein oligomerization, inhibition of protein condensation, inhibition of secondary nucleation processes in fibril formation, such as disrupting replication of aggregate formation on the surface of an existing fibril, and/or inhibition of fibril elongation by capping fibril ends.

4. The compound for use according to any one of the preceding claims, wherein the compound comprises
a. 5 to 20 amino acids, preferably 7 to 17 amino acids,
b. 3 or more non-proteinogenic amino acids,
c. 5 or more amino acids with a side chain comprising an aromatic ring, preferably selected from the group consisting of tyrosine, phenylalanine, phenylglycine and derivatives thereof,
d. one to four cyclic structures, and
e. at least one amino acid is substituted with a hydrophobic moiety.

5. The compound for use according to the preceding claim, wherein the hydrophobic moiety is a carbonyl group C=OR, wherein R is selected from the group consisting of alkyl, alkenyl, aryl, heteroaryl, alkyl aryl, and alkyl heteroaryl (preferably C5 to C20 alkyl, C5 to C20 alkenyl, C3 to C6 cycloalkyl, or benzyl), wherein R is optionally substituted with C1-C5 alkyl, C1-C5 alkenyl or C3 to C6 cycloalkyl.

6. The compound for use according to any one of the preceding claims, wherein 1 to 5 amino acids, preferably 1 to 3 amino acids, are substituted with a sugar moiety, wherein the sugar moiety comprises a mono-, di- or trisaccharide.

7. The compound for use according to any one of the preceding claims, wherein:
a. the compound comprises 17 amino acids (numbered 1 to 17), preferably comprising 6 to 8 amino acids with an aromatic side chain, 14 to 15 non-proteinogenic amino acids,
b. the compound forms a single ring structure and is cyclized via a lactone bond formed between a carboxyl end of a terminal amino acid residue 17 with a hydroxy group of amino acid residue 2,
c. amino acid 11 of the compound is substituted with a mono-, di- or trisaccharide sugar moiety, and
d. amino acid 1 is substituted with a hydrophobic moiety, wherein the hydrophobic moiety is a carbonyl group C=OR, wherein R is selected from the group consisting of alkyl, alkenyl, aryl, heteroaryl, alkyl aryl, and alkyl heteroaryl (preferably C5 to C20 alkyl, C5 to C20 alkenyl, C3 to C6 cycloalkyl, or benzyl), wherein R is optionally substituted with C1-C5 alkyl, C1-C5 alkenyl or C3 to C6 cycloalkyl.

8. The compound for use according to the preceding claims, according to **Formula I** 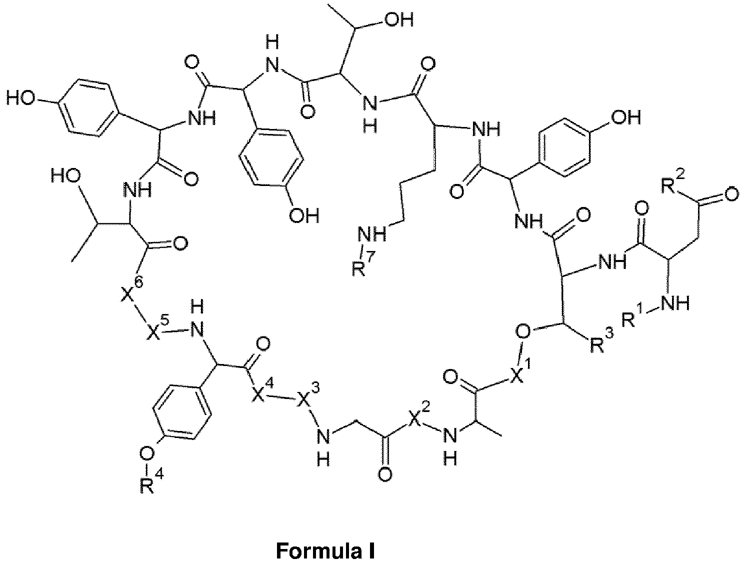 wherein:
**R¹** is a carbonyl group C=OR, wherein R is alkyl, alkenyl, cycloalkyl, aryl, heteroaryl, alkyl cycloalkyl, alkyl aryl, and alkyl heteroaryl (preferably C5 to C20 alkyl, C5 to C20 alkenyl, C3 to C6 cycloalkyl, or benzyl), wherein R is optionally substituted with C1-C5 alkyl, C1-C5 alkenyl or C3 to C6 cycloalkyl,
wherein **R¹** is preferably 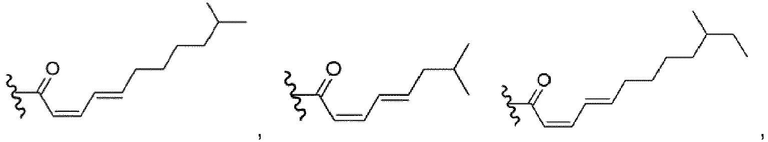 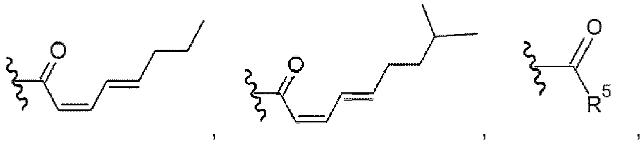 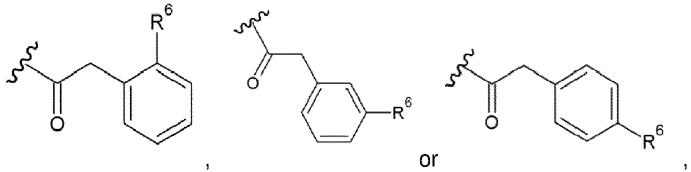
**R²** is H, -OH, an amine, an alkoxy group -OR, or a carbonyl group C=OR, wherein R is alkyl, alkenyl, cycloalkyl or aryl (preferably C1 to C12 alkyl, C3 to C6 cycloalkyl),
**R³** is H, alkyl, amide, carboxyl, carboxylate ester or an amine (preferably C1-C5 alkyl or an amide),
**R⁴** is H or a sugar moiety comprising 1 to 8 monosaccharide units (preferably comprising a mono-, di- or trisaccharide),
**R⁵** is alkyl, alkenyl, cycloalkyl, aryl, heteroaryl, alkyl cycloalkyl, alkyl aryl, and alkyl heteroaryl (preferably C5 to C20 alkyl, C5 to C20 alkenyl, C3 to C6 cycloalkyl, or benzyl), wherein R⁵ is optionally substituted with C1-C5 alkyl, C1-C5 alkenyl or C3 to C6 cycloalkyl,
**R⁶** is H, alkyl, alkenyl, aryl or cycloalkyl (preferably C1 to C12 alkyl, C3 to C6 cycloalkyl),
**R⁷** is H, alkyl, alkenyl, aryl or cycloalkyl (preferably C1 to C12 alkyl, C3 to C6 cycloalkyl),
**X¹** is an amino acid with a side chain comprising an aromatic ring, wherein the ortho-, meta- and/or para-positions of the aromatic ring are optionally substituted with halogen (preferably CI, Br, F), C1-C5 alkyl (preferably C1-C3 alkyl), C1-C5 haloalkyl or hydroxyl,
**X²** is an amino acid with a side chain comprising an alkyl group, amine, alkyl amine, amide, alkyl amide, carbamate, carboxamide or a heterocyclic ring structure (preferably comprising nitrogen),
**X³** is an amino acid with a side chain comprising an aromatic ring, wherein the ortho-, meta- and/or para-positions of the aromatic ring are optionally substituted with halogen (preferably CI, Br, F), C1-C5 alkyl (preferably C1-C3 alkyl), C1-C5 haloalkyl or hydroxyl,
**X⁴** is an amino acid with a side chain comprising an alkyl group or a hydroxyl group -R-OH, whereby R is C1 to C5 alkyl,
**X⁵** is an amino acid with a side chain comprising a comprising an alkyl group, amine, alkyl amine, amide, alkyl amide, carbamate, carboxamide or a heterocyclic ring structure (preferably comprising nitrogen),
**X⁶** is an amino acid with a side chain comprising an aromatic ring, wherein the ortho-, meta- and/or para-positions of the aromatic ring are optionally substituted with halogen (preferably CI, Br, F), C1-C5 alkyl (preferably C1-C3 alkyl), C1-C5 haloalkyl or hydroxyl, or with a side chain comprising an alkyl group, amine, alkyl amine, amide, alkyl amide, carbamate, carboxamide or a heterocyclic ring structure (preferably comprising nitrogen).

9. The compound for use according to the preceding claim, according to **Formula I** 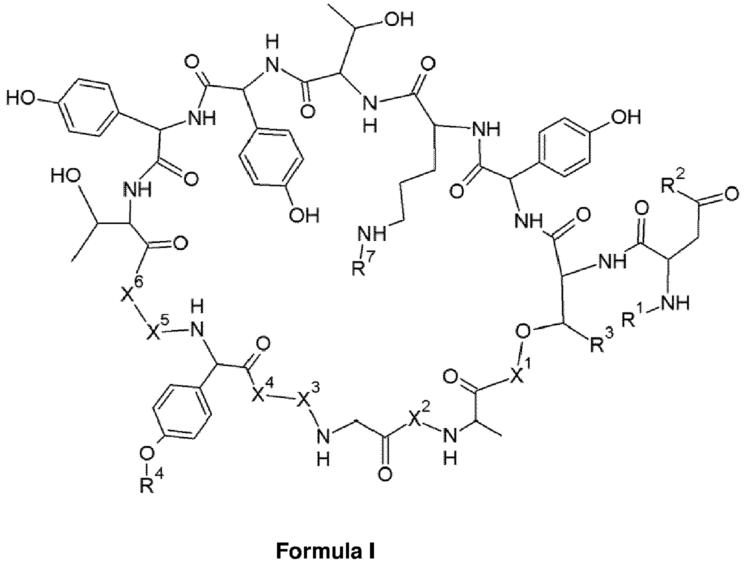 wherein:
**R¹** is 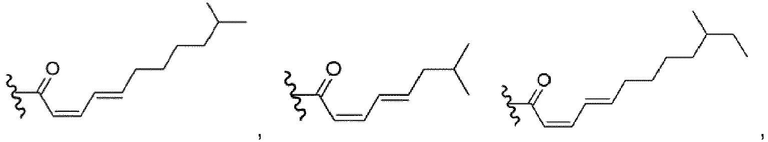 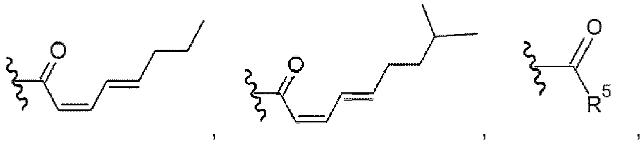 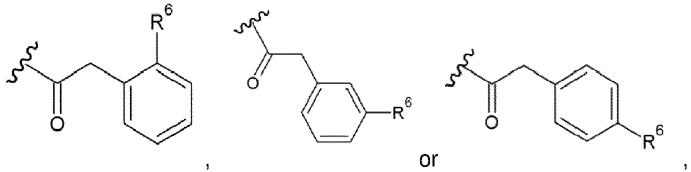
**R²** is -NH₂ or -OH,
**R³** is -CONH₂ or -CH₃,
**R⁴** is H or a mono-, di- or trisaccharide, preferably 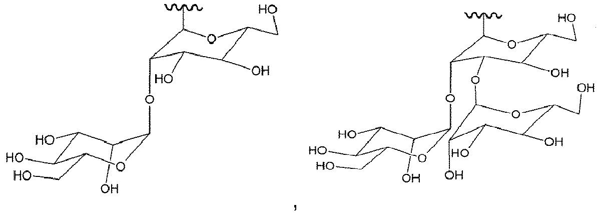 or 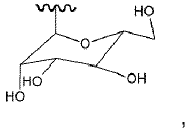
**R⁵** is C5 to C20 alkyl, C5 to C20 alkenyl, C3 to C6 cycloalkyl, or benzyl, wherein R⁵ is optionally substituted with C1-C5 alkyl, C1-C5 alkenyl or C3 to C6 cycloalkyl,
**R⁶** is H, alkyl, alkenyl, aryl or cycloalkyl (preferably C1 to C12 alkyl, C3 to C6 cycloalkyl),
**R⁷** is H, alkyl, alkenyl, aryl or cycloalkyl (preferably C1 to C12 alkyl, C3 to C6 cycloalkyl),
**X¹** is 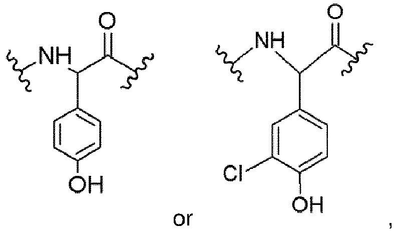
**X² is** 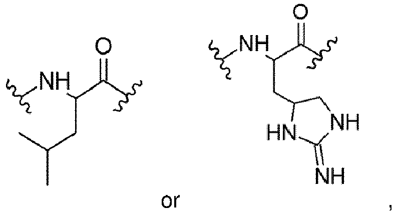 **X³ is** 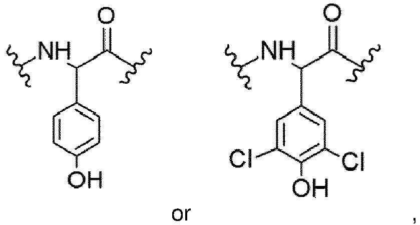
**X⁴** is 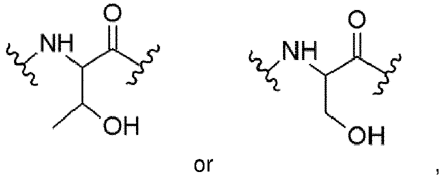 **X⁵** is 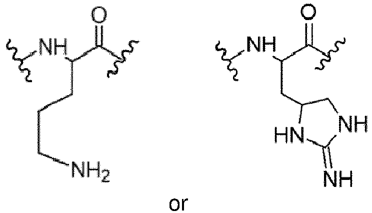
and **X⁶** is

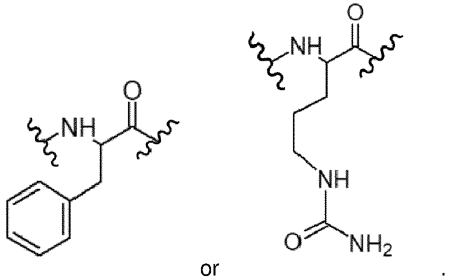

10. The compound for use according to any one of the preceding claims, wherein the compound is a ramoplanin, or derivative and/or salt thereof, such as ramoplanose, ramoplanin aglycon, ramoplanin A1-A3, enduracidin A, NAI-603 and/or enduracidin B, preferably ramoplanin A2 and/or NAI-603.

11. The compound for use according to claims 1 to 5, wherein:
a. the compound comprises 7 amino acids (numbered 1 to 7), preferably comprising 5 to 7 amino acids with an aromatic side chain and 5 non-proteinogenic amino acids,
b. the compound comprises two to four ring structures, preferably three or four ring structures, and
c. amino acids 1, 2 and/or 4 of the compound are substituted with a sugar moiety, which may be the same or different,
wherein one or more said sugar moieties are optionally substituted with a hydrophobic moiety comprising -NHC=OR, wherein R is selected from alkyl, aryl, heteroaryl, alkyl aryl, and alkyl heteroaryl (preferably C5 to C20 alkyl, C5 to C20 alkenyl, C3 to C6 cycloalkyl, or benzyl), wherein R is optionally substituted with C1-C5 alkyl, C1-C5 alkenyl or C3 to C6 cycloalkyl.

12. The compound for use according to the preceding claim, according to **Formula II** 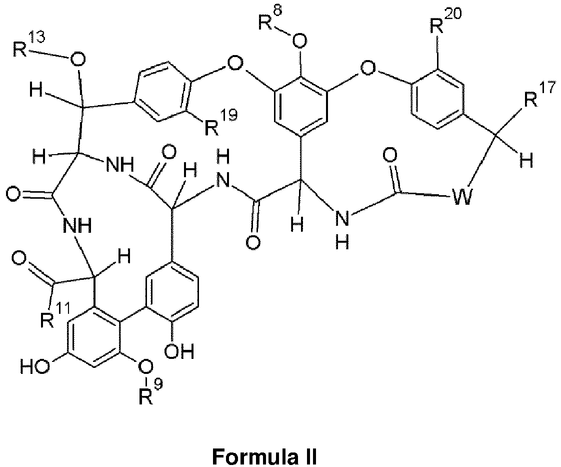 wherein
**R⁸, R⁹** and **R¹³** may be the same or different,
**R⁸, R⁹** and **R¹³** are H, or a sugar moiety comprising 1 to 8 monosaccharide units (preferably comprising a mono-, di-, tri- or tetra-saccharide), wherein at least **R⁸** is a sugar moiety,
wherein **R⁸** is optionally substituted with -NHR¹⁰,
**R¹⁰** is a carbonyl group C=OR, wherein R is alkyl, alkenyl, cycloalkyl, aryl, heteroaryl, alkyl cycloalkyl, alkyl aryl, and alkyl heteroaryl (preferably C5 to C20 alkyl, C5 to C20 alkenyl, C3 to C6 cycloalkyl, or benzyl), wherein R is optionally substituted with C1-C5 alkyl, C1-C5 alkenyl or C3 to C6 cycloalkyl,
wherein **R¹³** is optionally substituted with an amine (-NH₂) or amide (preferably -NH-C=OCH₃),
**R¹¹** is -H, -OH, an amine, or -O**R¹²,**
**R¹²** is alkyl, alkenyl, aryl or cycloalkyl (preferably C1 to C12 alkyl, C3 to C6 cycloalkyl),
**W** is a linker selected from 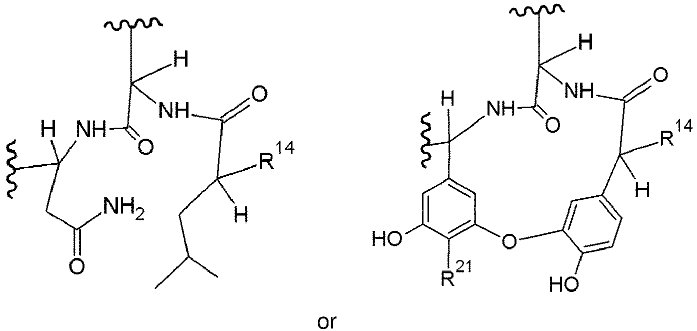
**R¹⁴** is alkyl, amine, alkyl amine, amide, alkyl amide, carbamate, carboxamide or a heterocyclic ring structure (preferably comprising nitrogen), optionally substituted with one or more halogens (preferably CI, Br, F), C1-C5 alkyl (preferably C1-C3 alkyl), C1-C5 haloalkyl, hydroxyl, alkoxy (preferably OCH₃, OCH₂CH₃), such as 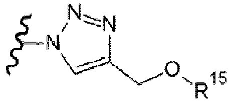
wherein **R¹⁴** is optionally substituted with **R¹⁵,**
**R¹⁵** is alkyl, aryl or cycloalkyl (preferably C1 to C12 alkyl, C3 to C6 cycloalkyl), optionally substituted with one or more halogens (preferably Cl, Br, F), C1-C5 alkyl (preferably C1-C3 alkyl), C1-C5 haloalkyl, hydroxyl, alkoxy (preferably OCH₃, OCH₂CH₃), such as 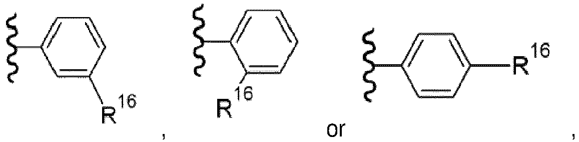 and
**R¹⁶** is alkyl, aryl or cycloalkyl (preferably C1 to C12 alkyl, C3 to C6 cycloalkyl)
**R¹⁷** is -H, -OH, or -O**R¹⁸**
**R¹⁸** is alkyl, alkenyl, aryl or cycloalkyl (preferably C1 to C12 alkyl, C3 to C6 cycloalkyl),
**R¹⁹** is -H or -a halogen (preferably CI, Br, F),
**R²⁰** is -H or -a halogen (preferably CI, Br, F),
**R²¹** is -H or alkyl, alkenyl, aryl or cycloalkyl (preferably C1 to C12 alkyl, C3 to C6 cycloalkyl).

13. The compound for use according to the preceding claim, according to **Formula II** 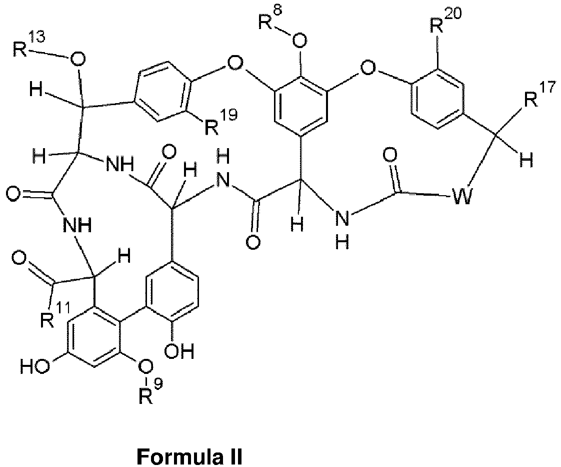 Wherein **R⁸** is 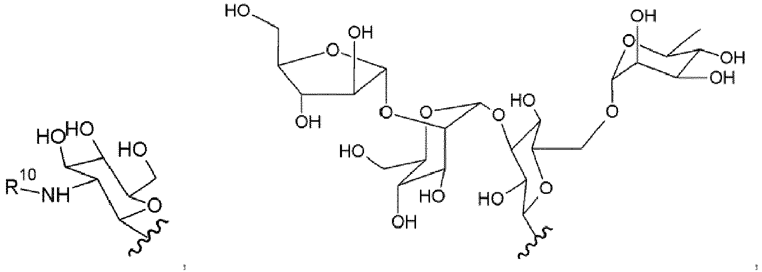 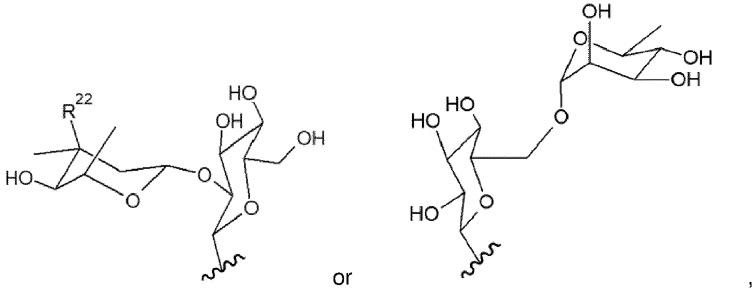
**R⁹ is** 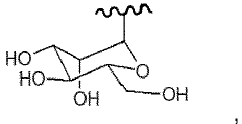
**R¹³** is 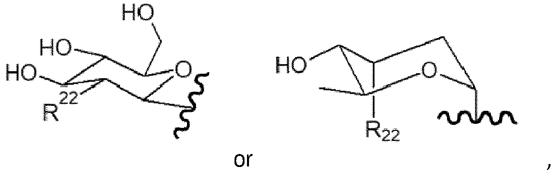
**R¹⁰** is a carbonyl group selected from: 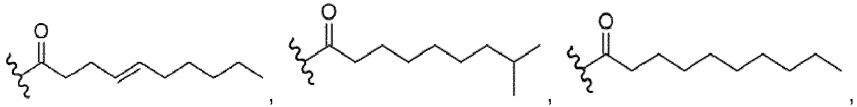 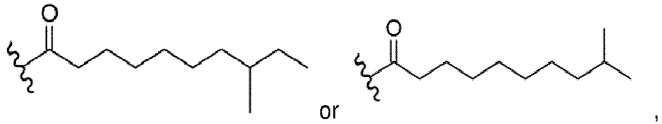
**R¹¹** is -H, -OH, or -O**R¹²,**
**R¹²** is alkyl, aryl or cycloalkyl (preferably C1 to C12 alkyl, C3 to C6 cycloalkyl),
**W** is a linker selected from 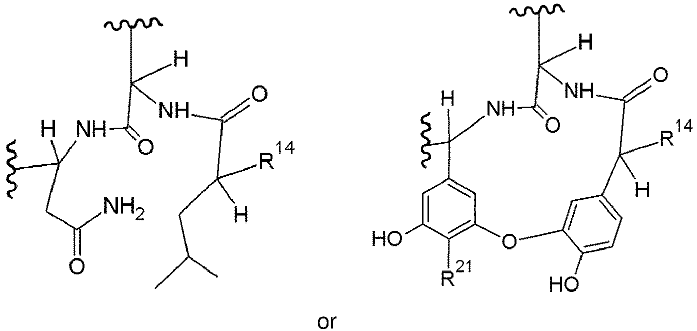
**R¹⁴** is amine (preferably NH₂), or 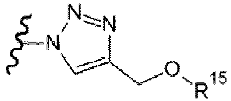
**R¹⁵** is H, alkyl, aryl or cycloalkyl (preferably C1 to C12 alkyl, C3 to C6 cycloalkyl), optionally substituted with **R¹⁶,** or is 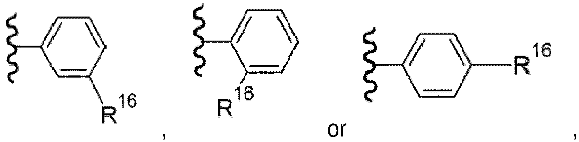 and
**R¹⁶** is alkyl, aryl or cycloalkyl (preferably C1 to C12 alkyl, C3 to C6 cycloalkyl),
**R¹⁷** is -H, -OH, or -O**R¹⁸,**
**R¹⁸** is alkyl, alkenyl, aryl or cycloalkyl (preferably C1 to C12 alkyl, C3 to C6 cycloalkyl),
**R¹⁹** is -H or -a halogen (preferably CI),
**R²⁰** is -H or -a halogen (preferably CI),
**R²¹** is -H or alkyl, alkenyl, aryl or cycloalkyl (preferably C1 to C12 alkyl, C3 to C6 cycloalkyl)
**R²² is** amine (preferably NH2), amide (preferably -NHC=OCH3) or 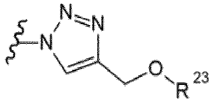
**R²³** is H, alkyl, aryl or cycloalkyl (preferably C1 to C12 alkyl, C3 to C6 cycloalkyl), or is 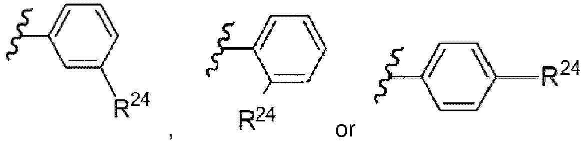 and
**R²⁴** is alkyl, aryl or cycloalkyl (preferably C1 to C12 alkyl, C3 to C6 cycloalkyl).

14. The compound for use according to claims 10 to 12, wherein the compound is a teicoplanin, or derivative and/or salt thereof, such as teicoplanin A2-1, teicoplanin A2-2, teicoplanin A2-3, teicoplanin A2-4, teicoplanin A2-5, ristocetin A, ristocetin B and/or vancomycin.

15. The compound for use according to any one of the preceding claims, wherein the medical condition comprises a protein aggregate of one or more of medin, tau protein, α-synuclein (Asyn), transactive response DNA binding protein 43 kDa (TPD-43), superoxide dismutase (SOD1), islet amyloid polypeptide (IAPP), kerato-epithelin, monoclonal immunoglobulin light chains (AL), transthyretin (TTR), serum amyloid A (AA), prion protein (PrP), p53 and/or gelsolin (Gel).

16. The compound for use according to any one of the preceding claims, wherein the medical condition is:
a. Alzheimer's disease, comprising amyloid protein aggregation, preferably comprising amyloid protein aggregation of a tau protein, preferably tau selfassemblies in β-sheet-rich amyloid structures,
b. Parkinson's disease, comprising amyloid protein aggregation, preferably comprising amyloid protein aggregation of a α-synuclein protein, more preferably the presence of Lewy bodies comprising aggregated α-synuclein (Asyn) protein accumulated in neurons of the subject,
c. amytrophic lateral sclerosis (ALS), comprising amyloid protein aggregation, preferably comprising amyloid protein aggregation of TDP-43 and/or SOD1,
d. an autoimmune disease, comprising amyloid protein aggregation, preferably diabetes mellitus, such as type 2 diabetes, preferably comprising amyloid protein aggregation of an islet amyloid polypeptide (IAPP) protein,
e. A cardiovascular, ocular and/or kidney disease, comprising amyloid protein aggregation, preferably comprising amyloid protein aggregation of monoclonal immunoglobulin light chains (AL) or transthyretin (TTR),
f. an age related vascular disfunction and/or vascular dementia, comprising amyloid protein aggregation, preferably comprising amyloid protein aggregation of a medin protein,
g. a cancer, comprising amyloid protein aggregation, preferably comprising p53 amyloid aggregation,
h. an ocular disease, comprising amyloid protein aggregation, such as ocular amyloidosis, preferably comprising amyloid protein aggregation of kerato-epithelin,
i. an encephalopathy, comprising amyloid protein aggregation, preferably comprising amyloid protein aggregation of a prion protein (PrP), such as Creutzfeld-Jakob disease (CJD), bovine spongiform encephalopathy, chronic wasting disease or scrapie,
j. is an AA-amyloidosis, preferably comprising an aggregate of a serum amyloid A protein, or
k. an AGel-amyloidosis, preferably comprising amyloid protein aggregation of gelsolin.
